(19) Europäisches Patentamt
European Patent Office
Office européen des brevets

(11) **EP 1 646 608 B1**

(12) **EUROPEAN PATENT SPECIFICATION**

(45) Date of publication and mention of the grant of the patent:
**02.03.2011 Bulletin 2011/09**

(21) Application number: **04773996.6**

(22) Date of filing: **23.06.2004**

(51) Int Cl.:
***C07C 317/30*** *(2006.01)*

(86) International application number:
**PCT/KR2004/001518**

(87) International publication number:
**WO 2004/113281 (29.12.2004 Gazette 2004/53)**

(54) **TRICYCLIC DERIVATIVES OR PHARMACEUTICALLY ACCEPTABLE SALTS THEREOF, THEIR PREPARATIONS AND PHARMACEUTICAL COMPOSITIONS CONTAINING THEM**

TRICYCLISCHE DERIVATE ODER PHARMAZEUTISCH UNNBEDENKLICHE SALZE DAVON, DEREN HERSTELLUNG UND PHARMAZEUTISCHE ZUSAMMENSETZUNGEN, DIE DIESE ENTHALTEN

DERIVES TRICYCLIQUES OU SELS PHARMACEUTIQUEMENT ACCEPTABLES DE CES DERIVES, LEURS PREPARATIONS ET COMPOSITIONS PHARMACEUTIQUES LES CONTENANT

(84) Designated Contracting States:
**AT BE BG CH CY CZ DE DK EE ES FI FR GB GR HU IE IT LI LU MC NL PL PT RO SE SI SK TR**

(30) Priority: **25.06.2003 KR 2003041547**

(43) Date of publication of application:
**19.04.2006 Bulletin 2006/16**

(73) Proprietor: **JE IL Pharmaceutical Co., Ltd.**
**Seocho-gu,**
**Seoul 137-810 (KR)**

(72) Inventors:
- **KIM, Myung-Hwa**
  **Yongin city,**
  **Kyunggi-do 449-942 (KR)**
- **CHUN, Kwangwoo**
  **Yongin city,**
  **Kyunggi-do 449-718 (KR)**
- **CHOI, Jae-Won**
  **Seoul 122-090 (KR)**
- **JOE, Bo-Young**
  **Yongin city,**
  **Kyunggi-do 449-812 (KR)**
- **PARK, Sang-Woo**
  **Jangan-gu,**
  **Suwon city,Kyunggi-do 440-827 (KR)**
- **KIM, Kwang Hee**
  **Yongin city,**
  **Kyunggi-do 449-735 (KR)**
- **OH, Byung-Kyu**
  **Yongin-si,**
  **Gyeonggi-do 449-724 (KR)**
- **CHOI, Jong-Hee**
  **Suwon city,**
  **Kyunggi-do 441-822 (KR)**

(74) Representative: **Grünecker, Kinkeldey,**
**Stockmair & Schwanhäusser**
**Anwaltssozietät**
**Leopoldstrasse 4**
**80802 München (DE)**

(56) References cited:
**WO-A1-97/01570 WO-A1-02/100824**

- **DATABASE CA [Online] CHEMICAL ABSTRACTS SERVICE, COLUMBUS, OHIO, US; RABOW, ALFRED A. ET AL RABOW, ALFRED A. ET AL: "Mining the National Cancer Institute's Tumor-Screening Database: Identification of Compounds with Similar Cellular Activities Mining the National Cancer Institute's Tumor-Screening Database: Identification of Compounds with Similar Cellular Activities" XP002402241 retrieved from STN Database accession no. 2002:59014 & JOURNAL OF MEDICINAL CHEMISTRY , 45(4), 818-840 CODEN: JMCMAR; ISSN: 0022-2623 JOURNAL OF MEDICINAL CHEMISTRY , 45(4), 818-840 CODEN: JMCMAR; ISSN: 0022-2623, 2002,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 646 608 B1

- DATABASE CA [Online] CHEMICAL ABSTRACTS SERVICE, COLUMBUS, OHIO, US; RABOW, ALFRED A. ET AL RABOW, ALFRED A. ET AL: "Mining the National Cancer Institute's Tumor-Screening Database: Identification of Compounds with Similar Cellular Activities Mining the National Cancer Institute's Tumor-Screening Database: Identification of Compounds with Similar Cellular Activities" XP002402242 retrieved from STN Database accession no. 2002:59014 & JOURNAL OF MEDICINAL CHEMISTRY , 45(4), 818-840 CODEN: JMCMAR; ISSN: 0022-2623 JOURNAL OF MEDICINAL CHEMISTRY , 45(4), 818-840 CODEN: JMCMAR; ISSN: 0022-2623, 2002,
- DATABASE CA [Online] CHEMICAL ABSTRACTS SERVICE, COLUMBUS, OHIO, US; STERZL, J. ET AL STERZL, J. ET AL: "Effect of colchicine derivatives on the antibody response induced in vitro Effect of colchicine derivatives on the antibody response induced in vitro" XP002402243 retrieved from STN Database accession no. 1983:27483 & FOLIA MICROBIOLOGICA (PRAGUE, CZECH REPUBLIC) , 27(4), 256-66 CODEN: FOMIAZ; ISSN: 0015-5632 FOLIA MICROBIOLOGICA (PRAGUE, CZECH REPUBLIC) , 27(4), 256-66 CODEN: FOMIAZ; ISSN: 0015-5632, 1982,
- DATABASE CA [Online] CHEMICAL ABSTRACTS SERVICE, COLUMBUS, OHIO, US; PAUL, BUDDHA D. ET AL PAUL, BUDDHA D. ET AL: "New agents for prostatic cancer activated specifically by prostatic acid phosphatase New agents for prostatic cancer activated specifically by prostatic acid phosphatase" XP002402244 retrieved from STN Database accession no. 1977: 495679 & CANCER TREATMENT REPORTS , 61 (2), 259-63 CODEN: CTRRDO; ISSN: 0361-5960 CANCER TREATMENT REPORTS , 61(2), 259-63 CODEN: CTRRDO; ISSN: 0361-5960, 1977,
- GUAN JAIN ET AL.: 'Antitumor agents. 185. Synthesis and biological evaluation of tridemethylthiocolchicine analogs as novel topoisomerase II inhibitors' JOURNAL OF MEDICINAL CHEMISTRY vol. 41, no. 11, 1998, pages 1956 - 1961, XP008074348
- SUN LI ET AL.: 'Antitumor agents. 141. Synthesis and biological evaluation of novel thiocolchicine analogs : N-acyl, N-aroyl, and N-(substituted benzyl)deacetylthiocolchicines as potent cytotoxic and antimitotic compounds' JOURNAL OF MEDICINAL CHEMISTRY vol. 36, no. 10, 1993, pages 1474 - 1479, XP008074350
- PLOURDE R. ET AL.: 'A hepatocyte-targeted conjugate capable of delivering biologically active colchicine in vitro' BIOCONJUGATE CHEMISTRY vol. 7, no. 2, 1996, page 281, XP008074334

## Description

FIELD OF THE INVENTION

The present invention relates to tricyclic derivatives represented by following <Formula 1>, or pharmaceutically acceptable salts thereof, their preparations and pharmaceutical compositions containing them.

<Formula 1>

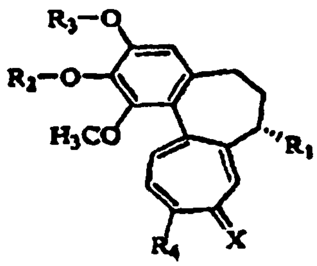

(Wherein, $R_1$, $R_2$, $R_3$, $R_4$ and X are as defined in the description.)

BACKGROUND

One of pseudo-alkaloid compounds, colchicine has an anti-inflammation action, making it a therapeutic agent for rheumatoid arthritis [Internal Medicine, 86, No.2, 342-345, 2000]. Colchicine and thiocolchicine derivatives have functions of muscle relaxation and anti-inflammation (USP 5 973 204, EP 0870761 A1). Thiocolchicoside has been used for the treatment of contracture and inflammation in skeletal muscles. Also, colchicine inhibits infiltration of monocytes and T-cells in a transplanted organ in animal experiment and at the same time restrains the production of TNF-$\alpha$, IL-1 and IL-6, inflammatory cytokines, suggesting an inhibiting effect on immune response [J. Am. Soc. Nephrol., 4(6), 1294-1299, 1993; Transplantation Proceedings, 32, 2091-2092, 2002]. Thus, colchicine is very attractive candidate for the development of an immune response inhibitor (WO 02/100824). Sun et al., J. Med. Chem 1993, 36, 1474-1479 describe the synthesis and biological evaluation of thiocolchicine analogs.

Colchicine inhibits a microtubule assembly by the interaction with tubulin, resulting in the suppression of cell division [The Alkaloids, 1991, 41, 125-176; USP 4 533 675]. Such colchicine has been used for the treatment of gout and other inflammatory diseases related to gout. However, the use of colchicine is limited to an acute inflammatory disease because of the limitation in therapeutic index and toxicity to gastrointestinal tract [Pharmacotherapy, 11, 3, 196-211, 1991].

All the endeavors to develop colchicine derivatives as an anticancer drug have not been successful so far [USP 3 222 253; USP 00/6080739; WO 97/01570], and only demecolcine has been used for the treatment of leukemia. However, toxicity to gastrointestinal tract and limitation in therapeutic index are still problems of demecolcine.

The present inventors have completed this invention by developing colchicine derivatives having excellent activities of anticancer, anti-proliferation and angiogenesis inhibition that have now stable therapeutic index resulted from decreased toxicity.

SUMMARY OF THE INVENTION

It is an object of the present invention to provide tricyclic derivatives or pharmaceutically acceptable salts thereof having excellent activities of anti-cancer, anti-proliferation and angiogenesis inhibition with stable therapeutic index by reduced toxicity.

It is also an object of this invention to provide a preparation method for tricyclic derivatives or pharmaceutically acceptable salts thereof.

It is a further object of this invention to provide a pharmaceutical composition containing tricyclic derivatives or pharmaceutically acceptable salts thereof as an effective ingredient.

The present invention is solved on the basis of claims 1 to 2.

BRIEF DESCRIPTION OF THE DRAWINGS

FIG. 1 is a graph showing the changes of the volume of a tumor in a BALB/c nude mouse transplanted with human lung cancer cell line NCI-H460 after the administration of tricyclic derivatives of the present invention (Example 8),

FIG. 2 is a graph showing the changes of the body weight of a BALB/c nude mouse transplanted with human lung cancer cell line NCI-H460 after the administration of tricyclic derivatives of the present invention (Example 8),

FIG. 3 is a graph showing the changes of the volume of a tumor in a BALB/c nude mouse transplanted with human lung cancer cell line NCI-H460 after the administration of tricyclic derivatives of the present invention (Example 12) by different concentrations (1, 3, 10 mg/kg),

FIG. 4 is a graph showing the changes of the body weight of a BALB/c nude mouse transplanted with human lung cancer cell line NCI-H460 after the administration of tricyclic derivatives of the present invention (Example 12) by different concentrations (1, 3, 10 mg/kg),

FIG. 5 is a set of photographs showing the volume of a tumor growing in a BALB/c nude mouse transplanted with human lung cancer cell line NCI-H460, which was separated on the 14th day after the administration of tricyclic derivatives of the present invention,

FIG. 6 is a set of photographs showing the activity of tricyclic derivatives of the present invention to inhibit angiogenesis in HUVEC cells.

DETAILED DESCRIPTION OF PREFERRED EMBODIMENTS

The present invention relates to tricyclic derivatives represented by following <Formula 1>, or pharmaceutically acceptable salts thereof.

<Formula 1>

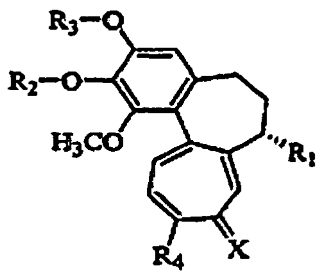

(Wherein,

(1) $R_1$ is $-T_1-B_1$;
in which $T_1$ is $-X_1-$, $-X_1-C(X_2)-$, $-N(R_5-$, $-N(R_5)C(X_2)-$, $-N(R_5)S(O)n_1-$, $-N(R_5)C(O)-X_1-$ or $-N(R_5)C(X_1)NH-$, in that $X_1$ and $X_2$ are each O or *S*, $R_5$ is each H or $C_1$~$C_5$ alkyl group, $n_1$ is an integer of 1~2; and $B_1$ is selected from a group consisting of following (a)~(j),

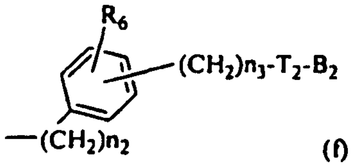

(f)

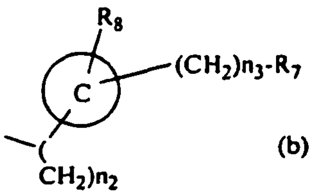

(b)

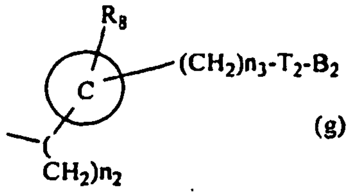

(g)

$-Z_1-T_2-B_2$ (h)

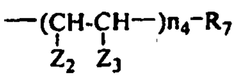

(d)

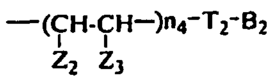

(i)

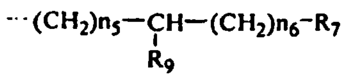

(e)

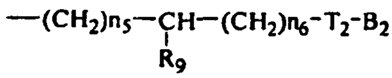

(j)

wherein, $R_6$ and $R_8$ are each H, halogen, hydroxy, $C_1$~$C_3$ alkoxy, amino, nitro, cyano or $C_1$~$C_3$ lower alkyl group; $R_7$ and $R_9$ are each independently halogen, hydroxy, mercapto, -ONO, - $ONO_2$ or SNO, in which $R_7$ and $R_9$ are same or different;

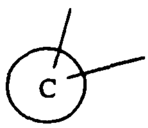

is $C_5$~$C_6$ membered saturated or unsaturated heterocyclic ring containing 1~2 of hetero atom, in which the hetero atom is selected from a group consisting of 0, S and N, preferably,

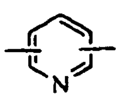
C1

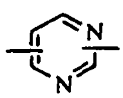
C2

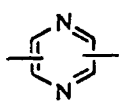
C3

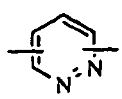
C4

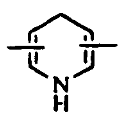
C5

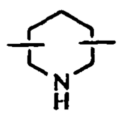
C6

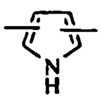
C7

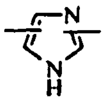
C8

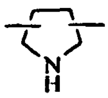
C9

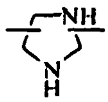
C10

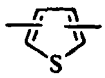
C11

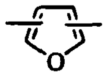
C12 ,

more preferably, C1 (pyridyl group) substituted at position 2 and 6 or position 2 and 5, C7 (pyrrolyl group) substituted at position 2 and 5 or position 2 and 4, C11 (thiophenyl group) or C12 (furanyl group) ; $Z_1$ is $C_1$~$C_{10}$ straight-chain or branched-chain alkyl group, preferably $C_2$~$C_5$ straight-chain or branched-chain alkyl group or cycloalkyl group having substituent; $Z_2$ and $Z_3$ are each independently H or methyl group, in which $Z_3$ is H when $Z_2$ is methyl group, $Z_2$ is H when $Z_3$ is methyl group; $T_2$ is $-X_1-$ or $-X_1$-C $X_2$) -, in that $X_1$ and $X_2$ are each independently O or S; $B_2$ is selected from a group consisting of said

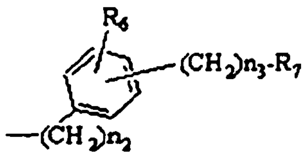
,

(b) , $-Z_1-R_7$, (d) or (e) ; $n_2$ is an integer of 0~3, $n_3$ is an integer of 0~5, $n_4$ is an integer of 1~5, $n_5$ and $n_6$ are each independently an integer of 1~6;

(2) $R_2$ and $R_3$ are each independently H, $-PO_3H_2$, phosphonate, sulfate, $C_3$~$C_7$ cycloalkyl, $C_2$~$C_7$ alkenyl, $C_2$~$C_7$ alkynyl, $C_1$~$C_7$ alkanoyl, $C_1$~$C_7$ straight-chain or branched-chain alkyl or sugar, in which sugar is a monosaccharide such as glucuronyl, glucosyl or galactosyl;

(3) $R_4$ is $OCH_3$, $SCH_3$ or $NR_{10}R_{11}$, in which $R_{10}$ and $R_{11}$ are each independently H or $C_{1-5}$ alkyl;

(4) X is O or S.

The present invention further relates to tricyclic derivatives represented by the following <Formula 1>, or pharmaceutically acceptable salts thereof.

<Formula 1>

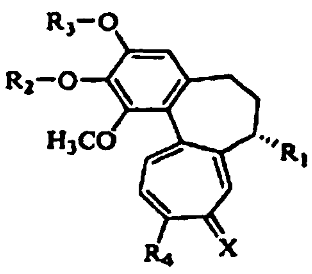

Wherein,

(1) $R_1$ is $-T_1-B_1$;

in which $T_1$ is $-X_1-$, $-X_1-C(X_2)-$, $-N(R_5)-$, $-N(R_5)C(X_2)-$, $-N(R_5)S(O)n_1-$, $-N(R_5)C(O)-X_1-$ or $-N(R_5)C(X_1)NH-$, in that $X_1$ and $X_2$ are each O or S, $R_5$ is each H or $C_1$~$C_5$ alkyl group, $n_1$ is an integer of 1~2; and $B_1$ is selected from a group consisting of

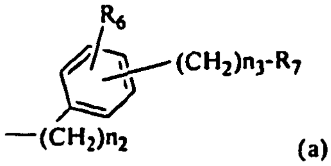

(a)

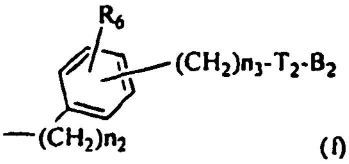

(f)

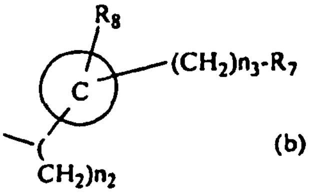

(b)

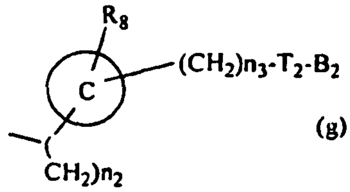

(g)

$-Z_1-T_2-B_2$ (h)

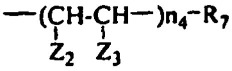

(d)

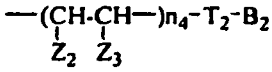

(i)

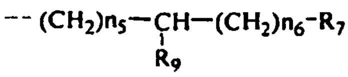

(e)

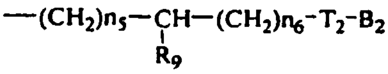

(j)

wherein, $R_6$ is halogen, hydroxy, $C_1$~$C_3$ alkoxy, amino, nitro, cyano or $C_1$~$C_2$ lower alkyl group; $R_8$ is H, halogen, hydroxy, $C_1$~$C_3$ alkoxy, amino, nitro, cyano or $C_1$~$C_3$ lower alkyl group; $R_7$ and $R_9$ are each independently halogen, hydroxy, mercapto, -ONO, $-ONO_2$ or SNO, in which $R_7$ and $R_9$ are same or different;

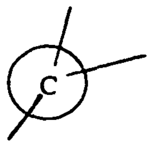

is $C_5$~$C_6$ membered saturated or unsaturated heterocyclic ring containing 1~2 of hetero atom, in which the hetero atom is selected from a group consisting of O, S and N, preferably,

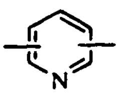

C1

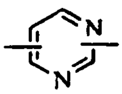

C2

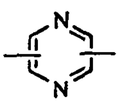

C3

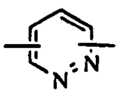

C4

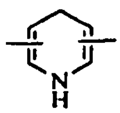

C5

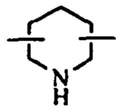

C6

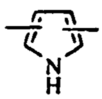
C7
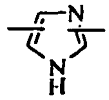
C8
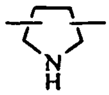
C9
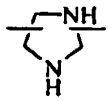
C10
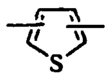
C11
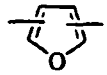
C12
,

more preferably, C1 (pyridyl group) substituted at position 2 and 6 or position 2 and 5, C7 (pyrrolyl group) substituted at position 2 and 5 or position 2 and 4, C11 (thiophenyl group) or C12 (furanyl group); $Z_1$ is $C_1$~$C_{10}$ straight-chain or branched-chain alkyl group, preferably $C_2$~$C_5$ straight-chain or branched-chain alkyl group or cycloalkyl group having substituent; $Z_2$ and $Z_3$ are each independently H or methyl group, in which $Z_3$ is H when $Z_2$ is methyl group, $Z_2$ is H when $Z_3$ is methyl group; $T_2$ is $-X_1-$ or -X1-C($X_2$)-, in that $X_1$ and $X_2$ are each independently O or S; $B_2$ is selected from a group consisting of said (a), (b), $-Z_1-R_7$, (d) or (e); $n_2$ is an integer of 0~3, $n_3$ is an integer of 0~5, $n_4$ is an integer of 1~5, $n_5$ and $n_6$ are each independently an integer of 1~6;

(2) $R_2$ and $R_3$ are each independently H, $-PO_3H_2$, phosphonate, sulfate, $C_3$~$C_7$ cycloalkyl, $C_2$~$C_7$ alkenyl, $C_2$~$C_7$ alkynyl, $C_1$~$C_7$ alkanoyl, $C_1$~$C_7$ straight-chain or branched-chain alkyl or sugar, in which sugar is a monosaccharide such as glucuronyl, glucosyl or galactosyl;

(3) $R_4$ is $OCH_3$, $SCH_3$ or $NR_{10}R_{11}$, in which $R_{10}$ and $R_{11}$ are each independently H or $C_{1\sim5}$ alkyl;

(4) X is O or S.

Preferably in the compound of <Formula 1>,

(1) $R_1$ is $-T_1-B_1$:
in which $T_1$ is $-N(R_5)C(X_2)-$, $-N(R_5)C(O)-X_1-$ or - $N(R_5)C(X_1)NH-$, in that $X_1$ and $X_2$ are each O, $R_5$ is each H or $C_1$~$C_5$ alkyl group; and $B_1$ is selected from a group consisting of

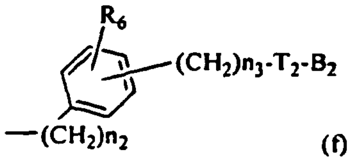
(f)

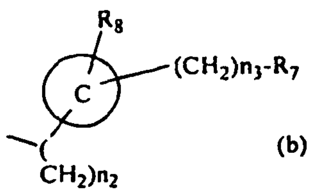
(b)

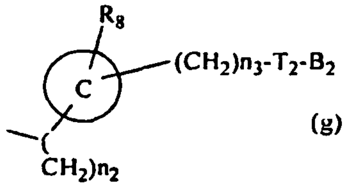
(g)

$-Z_1-T_2-B_2$ (h)

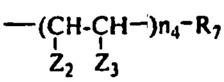
(d)

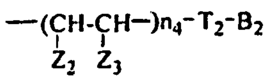
(i)

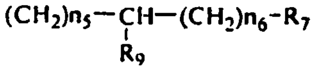 (e) 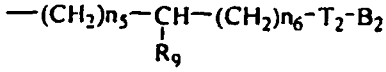 (j)

wherein, $R_6$ and $R_8$ are each H, halogen, hydroxyl, $C_1$~$C_3$ alkoxy, amino, nitro, cyano or $C_1$~$C_3$ lower alkyl group; $R_7$ and $R_9$ are each independently halogen, hydroxyl, mercapto(thiol), - ONO, $-ONO_2$ or SNO, in which $R_7$ and $R_9$ are same or different;

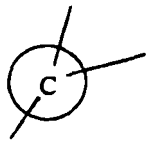

is $C_5$~$C_6$ membered saturated or unsaturated heterocyclic ring containing 1~2 of hetero atom, in which the hetero atom is selected from a group consisting of 0, S and N, preferably,

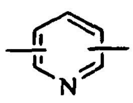 C1 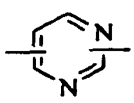 C2 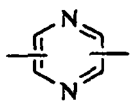 C3 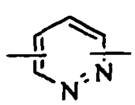 C4 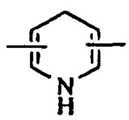 C5 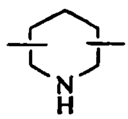 C6

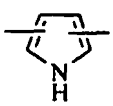 C7 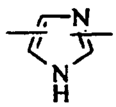 C8 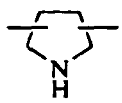 C9 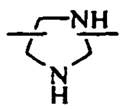 C10 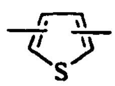 C11 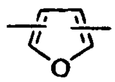 C12 ,

more preferably, C1 (pyridyl group) substituted at position 2 and 6 or position 2 and 5, C7 (pyrrolyl group) substituted at position 2 and 5 or position 2 and 4, C11 (thiophenyl group) or C12 (furanyl group), a bond of substituents may be at symmetrical or asymmetrical position; $Z_1$ is $C_1$~$C_{10}$ straight-chain or branched-chain alkyl group, preferably $C_2$~$C_5$ straight-chain or branched-chain alkyl group or cycloalkyl group
having substituent; $Z_2$ and $Z_3$ are each independently H or methyl group, in which $Z_3$ is H when $Z_2$ is methyl group, $Z_2$ is H when $Z_3$ is methyl group; $T_2$ is $-X_1-$ or $-X_1-C(X_2)$ -, in that $X_1$ and $X_2$ are each O or S; $B_2$ is selected from a group consisting of said

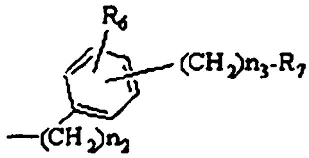 ,

(b) , $-Z_1-R_7$, (d) or (e) ; $n_2$ is an integer of 0~3, $n_3$ is an integer of 0~5, $n_4$ is an integer of 1~3, $n_5$ and $n_6$ are each independently an integer of 1~3;

(2) $R_2$ and $R_3$ are each independently $C_3$~$C_7$ cycloalkyl or $C_1$~$C_7$ alkyl;

(3) $R_4$ is $SCH_3$ or $OCH_3$;

(4) X is O or S.

In another preferred embodiment, in the compound of <Formula 1>,

(1) $R_1$ is $-T_1-B_1$;
in which $T_1$ is $-N(R_5)C(X_2)-$, $-N(R_5)C(O)-X_1-$ or $-N(R_5)C(X_1)NH-$, in that $X_1$ and $X_2$ are each O $R_5$ is each H or $C_1$~$C_5$ alkyl group; and $B_1$ is selected from a group consisting of

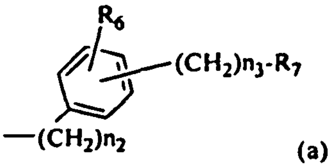 (a)

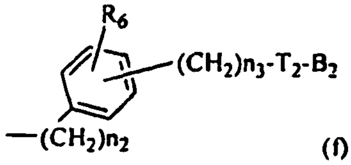 (f)

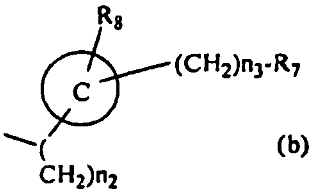 (b)

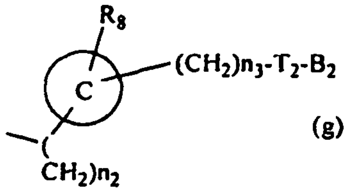 (g)

$-Z_1-T_2-B_2$ (h)

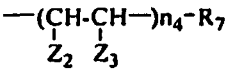 (d)

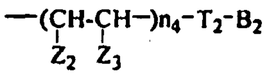 (i)

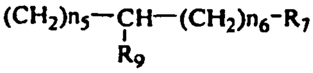 (e)

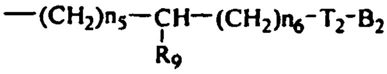 (j)

wherein, $R_6$ is halogen, hydroxy, $C_1$~$C_3$ alkoxy, amino, nitro, cyano or $C_1$~$C_3$ lower alkyl group; $R_8$ is H, halogen, hydroxy, $C_1$~$C_3$ alkoxy, amino, nitro, cyano or $C_1$~$C_3$ lower alkyl group; $R_7$ and $R_9$ are each independently halogen, hydroxy, mercapto, -ONO, $-ONO_2$ or SNO, in which $R_7$ and $R_9$ are same or different;

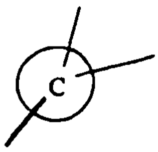

is $C_5$~$C_6$ membered saturated or unsaturated heterocyclic ring containing 1~2 of hetero atom, in which the hetero atom is selected from a group consisting of O, S and N, preferably,

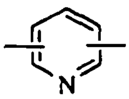 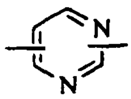 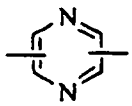 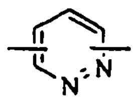 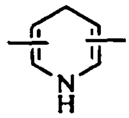 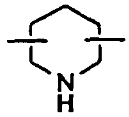

C1 C2 C3 C4 C5 C6

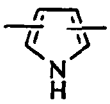 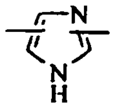 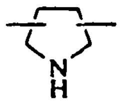 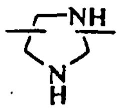 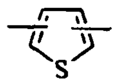 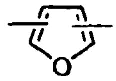

C7 C8 C9 C10 C11 C12 ,

more preferably, C1 (pyridyl group) substituted at position 2 and 6 or position 2 and 5, C7 (pyrrolyl group) substituted at position 2 and 5 or position 2 and 4, C11 (thiophenyl group) or C12 (furanyl group), a bond of substituents may be at symmetrical or asymmetrical position; $Z_1$ is $C_1$~$C_{10}$ straight-chain or branched-chain alkyl group, preferably $C_2$~$C_5$ straight-chain or branched-chain alkyl group or cycloalkyl group having substituent; $Z_2$ and $Z_3$ are each independently H or methyl group, in which $Z_3$ is H when $Z_2$ is methyl group, $Z_2$ is H when $Z_3$ is methyl group; $T_2$ is $-X_1-$ or $-X_1-C(X_2)-$, in that $X_1$ and $X_2$ are each O or S; $B_2$ is selected from a group consisting of said (a), (b), $-Z_1-R_7$, (d) or (e); $n_2$ is an integer of 0~3, $n_3$ is an integer of 0~5, $n_4$ is an integer of 1~5, $n_5$ and $n_6$ are each independently an integer of 1~6;

(2) $R_2$ and $R_3$ are each independently $C_3$~$C_7$ cycloalkyl or $C_1$~$C_7$ alkyl ;

(3) $R_4$ is $SCH_3$ or $OCH_3$;

(4) X is O or S.

Preferably, the compounds of <Formula 1> comprise:

1) 6-nitrooxymethyl-N-[(7S)-1,2,3-trimethoxy-10-methylsulfanyl-9-oxo-5,6,7,9-tetrahydro-benzo[a]heptalen -7-yll-nicotineamide;

2) 5-nitrooxymethyl-furan-2-carboxylic acid-[(7S)-1,2,3-trimethoxy-10-methylsulfanyl-9-oxo-5,6,7,9-tetrahydro-benzo[a]heptalen-7-yl]-amide;

5) 6-nitrooxymethyl-pyridine-2-carboxylic acid-[(7S)-1,2,3-trimethoxy-10-methylsulfanyl-9-oxo-5,6,7,9-tetrahydro-benzo[a]heptalen-7-yl]-amide;

6) 5-nitrooxymethyl-thiophene-2-carboxylic acid-[(7S)-1,2,3-trimethoxy-10-methylsulfanyl-9-oxo-5,6,7,9-tetrahydro-benzo[a]heptalen-7-yl]-amide;

8) N-[(7S)-3-ethoxy-1,2-dimethoxy-10-methylsulFanyl-9-oxo-5,6,7,9-tetrahydro-benzo[a]heptalen-7-yl]-2-fluoro-3-nitrooxymethyl-benzamide;

9) 2-fluoro-N-[(7S)-3-isopropoxy-1,2-dimethoxy-10-methylsulfanyl-9-oxo-5,6,7,9-tetrahydro-benzo[a]heptalen-7-yl]-3-nitrooxymethyl-benzamide;

10) 2-fluoro-3-nitrooxymethyl-N-((7S)-1,2,3-trimethoxy-10-methylsulfanyl-9-oxo-5,6,7,9-tetrahydro-benzo[alheptalen-7-yl]-benzamide;

11) N-[(7S)-3-cyclopentyloxy-1,2-dimethoxy-10-methylsulfanyl-9-oxo-5,6,7,9-tetrahydro-benzo[a]heptalen-7-yl]-2-fluoro-3-nitrooxymethyl-benzamide;

12) 3-fluoro-5-nitrooxymethyl-N-[(7S)-1,2,3-trimethoxy-10-methylsulfanyl-9-oxo-5,6,7,9-tetrahydro-benzo[a]heptalen-7-yl]-benzamide;

13) N-[(7S)-3-ethoxy-1,2-dimethoxy-10-methylsulfanyl-9-oxo-5,6,7,9-tetrahydro-benzo[a]heptalen-7-yl]-3-fluoro-5-nitrooxymethyl-benzamide;

14) 3-fluoro-N-[(7S)-3-isopropoxy-1,2-dimethoxy-10-methylsulfanyl-9-oxo-5,6,7,9-tetrahydro-benzo[a]heptalen-7-yl]-5-nitrooxymethyl-benzamide;

15) N-[(7S)-3-cyclopentyloxy-1,2-dimethoxy-10-methylsulfanyl-9-oxo-5,6,7,9-tetrahydro-benzo(alheptalen-7-yll-3-flucro-5-nitrooxymethyl-benzamide;

16) 4-fluoro-3-nitrooxymethyl-N-[(7S)-1,2,3-trimethoxy-10-methylsulfanyl-9-oxo-5,6,7,9-tetrahydro-benzo[a]heptalen-7-yl]-benzamide;

17) 2-fluoro-5-nitrooxymethyl-N-[(7S)-1,2,3-trimethoxy-10-methylsulfanyl-9-oxo-5,6,7,9-tetrahydro-benzo[alheptalen-7-yl]-benzamide;
18) 3-hydroxy-5-nitrooxymethyl-N-[(7S)-1,2,3-trimethoxy-10-methylsulfanyl-9-oxo-5,6,7,9-tetrahydro-benzo[a]heptalen-7-yl]-benzamide;
20) 2-hydroxy-4-nitrooxymethyl-N-[(7S)-1,2,3-trimethoxy-10-methylsulfanyl-9-oxo-5,6,7,9-tetrahydro-benzo[a]heptalen-7-yl]-benzamide;
21) 4-nitrooxymethyl-thiophene-2-carboxylic acid [(7S)-1,2,3-trimethoxy-10-methylsulfanyl-9-oxo-5,6,7,9-tetrahydro-benzo[a]heptalen-7-yl]-amide;
22) 3-nitrooxymethyl-thiophene-2-carboxylic acid [(7S)-1,2,3-trimethoxy-10-methylsulfanyl-9-oxo-5,6,7,9-tetrahydro-benzo[a]heptalen-7-yl]-amide;
25) 3-nitrooxybenzoic acid-5-[(7S)-1,2,3-trimethoxy-10-methylsulfanyl-9-oxo-5,6,7,9-tetrahydro-benzo[a]heptalen-7-yl-carbamoyl]-pyridine-2-yl-methylester;
26) 4-nitrooxybutyric acid-5-((7S)-1,2,3-trimethoxy-10-methylsulfanyl-9-oxo-5,6,7,9-tetrahydro-benzo[a]heptalen-7-yl-carbamoylj-pyridine-2-yl-methyleater;
27) 3-nitrooxymethyl-benzoic acid-6-[(7S)-1,2,3-trimethoxy-10-methylsulfanyl-9-oxo-5,6,7,9-tetrahydro-benzo[a]heptalen-7-yl-carbamoyl]-pyridine-2-yl-methylester;
28) 4-nitrooxybutyric acid-6-[(7S)-1,2,3-trimethoxy-10-methylaulfanyl-9-oxo-5,6,7,9-tetrahydro-benzo[a]heptalen-7-yl-carbamoyl]-pyridine-2-yl-methylester;
29) 3-nitrooxymethyl-benzoic acid-2-[(75)-1,2,3-trimethoxy-10-methylsulfanyl-9-oxo-5,6,7,9-tetrahydro-benzo[a]heptalen-7-yl-carbamoyl]-phenyleater;
30) 4-nitrooxybutyric acid-2-[(7S)-1,2,3-trimethoxy-10-methylsulfanyl-9-oxo-5,6,7,9-tetrahydro-benzo[a]heptalen-7-yl-carbamoyl]-phenylester;
31) 3-nitrooxymethyl-benzoic acid-3-[(7S)-1,2,3-trimethoxy-10-methylsulfanyl-9-oxo-5,6,7,9-tetrahydro-benzo[a]heptalen-7-yl-carbamoyl]-phenyleater;
32) 4-nitrooxybutyric acid-3-[(7S)-1,2,3-trimethoxy-10-methylsulfanyl-9-oxo-5,6,7,9-tetrahydro-benzo[a]heptalen-7-yl-carbamoyl]-phenyleater;
33) 3-nitrooxymethyl-benzoic acid-3-[(7S)-1,2,3-trimethoxy-10-methylsulfanyl-9-oxo-5,6,7,9-tetrahydro-benzo[a]heptalen-7-yl-carbamoyl]-benzylester;
34) 4-nitrooxybutyric acid-3-[(7S)-1,2,3-trimethoxy-10-methylsulfanyl-9=oxo-5,6,7,9-tetrahydro-benzo[a]heptalen-7-yl-carbamoyl]-benzylester;
37) 3-fluoro-S-nitrosooxymethyl-N-[(7S)-1,2,3-trimethoxy-10-methylsulfanyl-9-oxo-5,6,7,9-tetrahydro-benzo[a]heptalen-7-yl]-benzamide;
39) 3-fluoro-5-nitrosothiomethyl-N-[(7S)-1,2,3-trimethoxy-10-methylsulfanyl-9-oxo-5,6,7,9-tetrahydro-benzo[a]heptalen-7-yl]-benzamide;
40) 3-fluoro-5-nitrooxymethyl-N-[(7S)-1,2,3,10-tetramethoxy-9-oxo-5,6,7,9-tetrahydro-benzo[a]heptalen-7-yl]-benzamide;
42) 3-fluoro-N-methyl-5-nitrooxymethyl-N-[(7S)-1,2,3-trimethoxy-10-methylsulfanyl-9-oxo-5,6,7,9-tetrahydro-benzo[a]heptalen-7-yl]-benzamide;
43) 2-(3-fluoro-5-nitrooxymethyl-phenyl)-N-[(7S)-1,2,3-trimethoxy-10-methylsulfanyl-9-oxo-5,6,7,9-tetrahydro-benzo[a]heptalen-7-yl]-acetamide; or
44) 2-(2-fluoro-5-nitrooxymethyl-phenyl)-N-[(7S)-1,2,3-trimethoxy-10-methylsulfanyl-9-oxo-5,6,7,9-tetrahydro-benzo[a]heptalen-7-yl]-acetamide.

The present invention also provides pharmaceutically acceptable salts of the compound represented by <Formula 1>. Pharmaceutically acceptable salts of the present invention can include acid addition salt of a compound according to the invention when the compound is fully basic. Such acid addition salt includes salts holding inorganic acid providing pharmaceutically acceptable anion such as hydrogen halide, or organic acid, or salts holding sulfuric acid or phosphoric acid, or salts holding trifluoroacetic acid, citric acid or maleic acid. And, it include for example hydrochlorides, hydrobromides, phosphonates, sulfates, alkylsulfonates, arylsulfonates, acetates, benzoates, citrates, maleates, fumarates, succinates, lactates and tartarates by suitable salts. When a compound of the present invention is fully acidic, pharmaceutically acceptable salts can include inorganic salts or organic salts providing pharmaceutically acceptable cation. Said inorganic salts include sodium salts, potassium salts, calcium salts or magnesium salts, etc., said organic salts include methylamine salts, dimethylamine salts, trimethylamine salts, piperidine salts or morpholine salts, etc.
The present invention also provides a preparation method for tricyclic derivatives represented by the <Formula 1>. The preparation method for tricyclic derivatives of the present invention is described in the below Scheme 1 ~ Scheme 8. Precisely, in the <Formula 1>, when $R_1$ is $-T_1-B_1$ and $B_1$ is one of said (a), (b), (c), (d) and (e), the derivatives are prepared according to the method of Scheme 1 ~ Scheme 6. In the meantime, in the <Formula 1>, when $R_1$ is $-T_1-B_1$ and $B_1$ is one of said (f), (g), (h), (i) and (j), the derivatives are prepared by the method of Scheme 7 and Scheme 8.

And a concrete compound of the <Formula 1> is represented by general formulas (IIa), (IIb), (IIc), (IId), (IIe), (IIf), (IIg), (IIh), (IIi), (IIj), (IIk), (IIl), (IIm), (IIn), (IIo) and (IIp) in Scheme 1 ~ Scheme 8.

<Scheme 1>

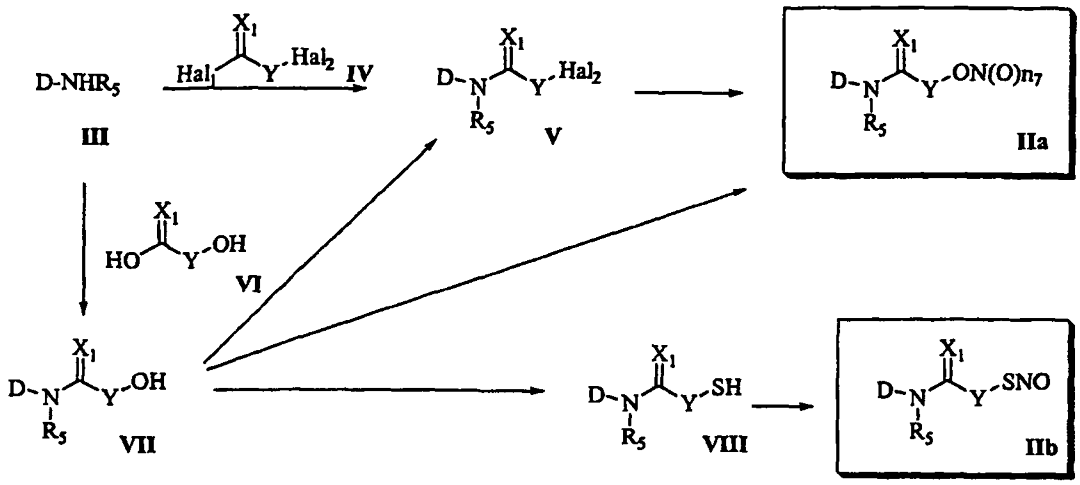

<Scheme 2>

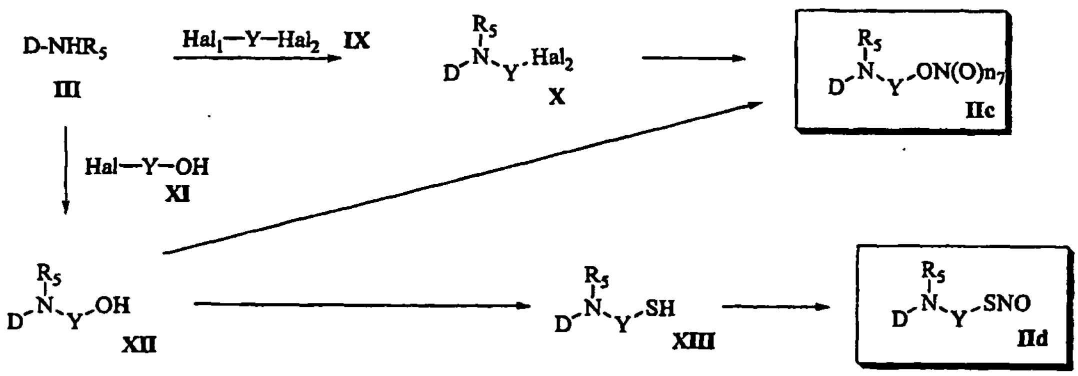

<Scheme 3>

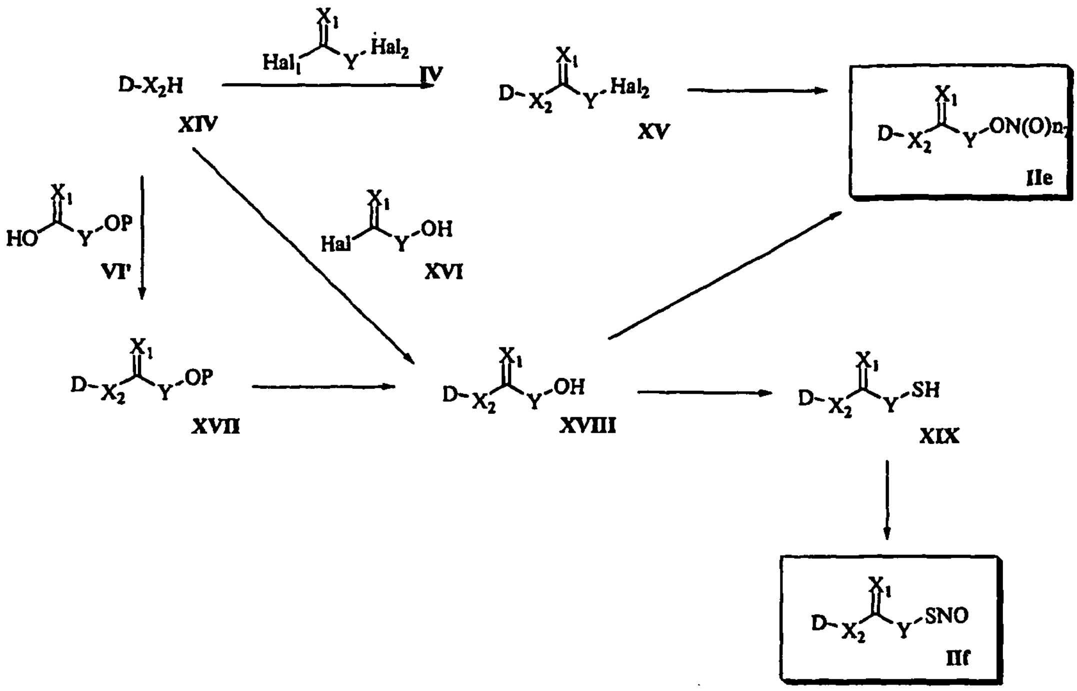

<Scheme 4>

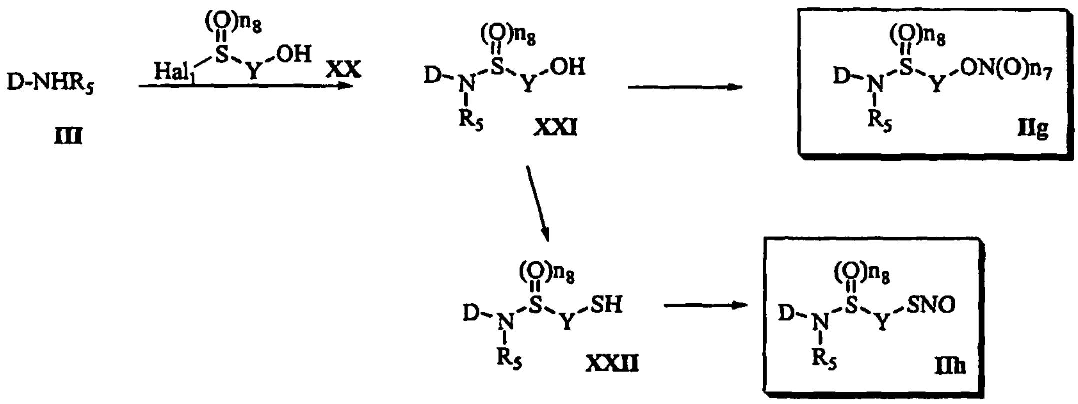

<Scheme 5>

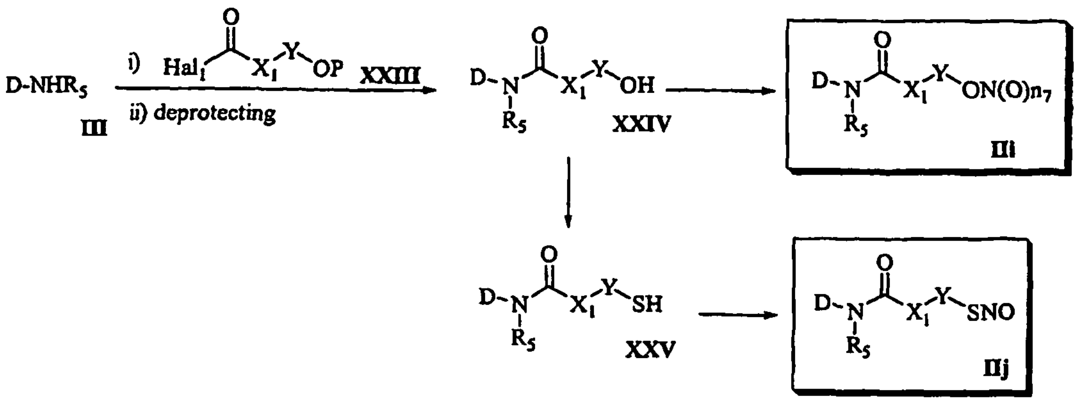


<Scheme 6>

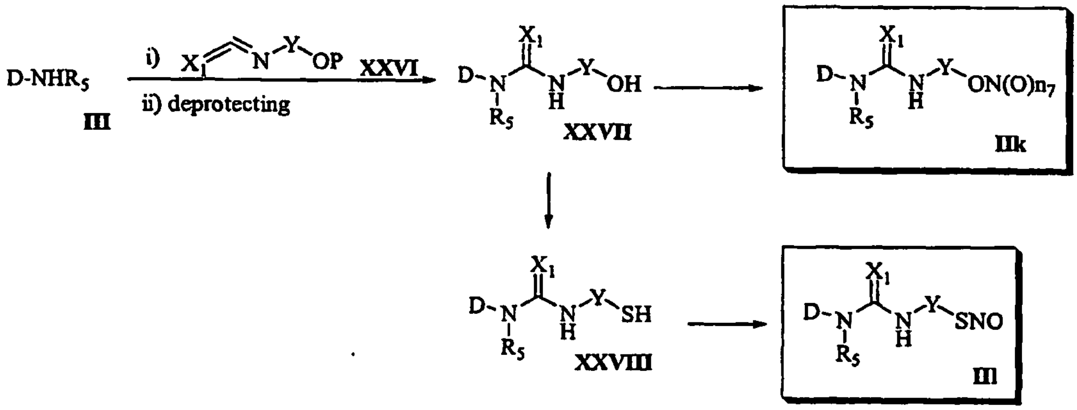


<Scheme 7>

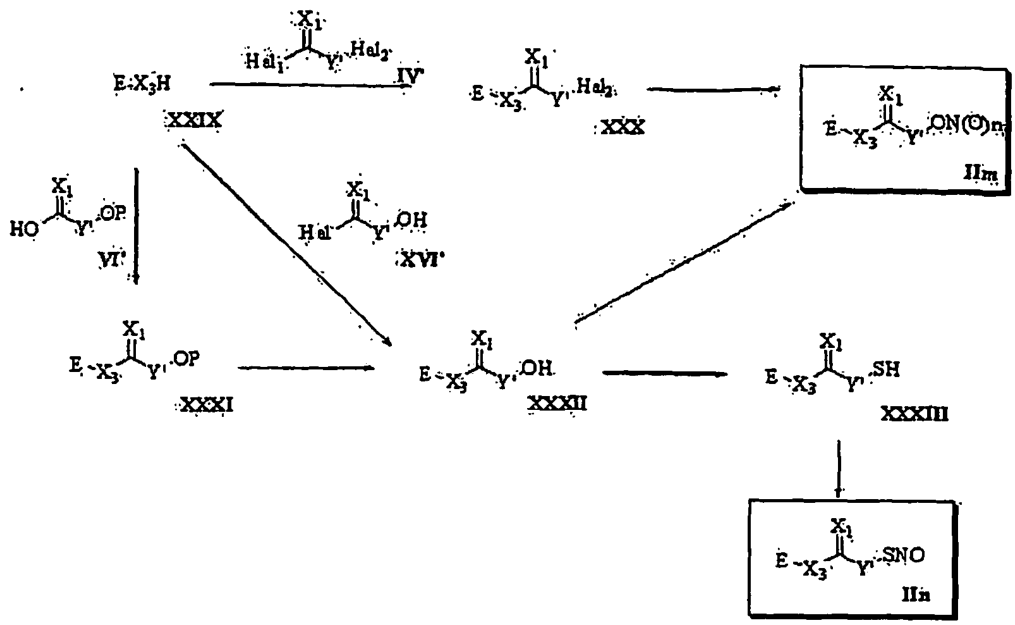

<Scheme 8>

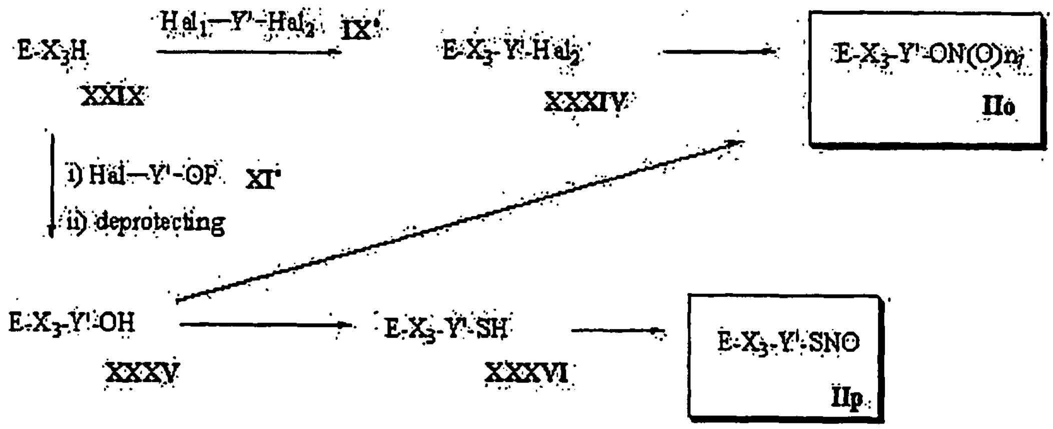


In the above Scheme, E is following E1 ~ E6 respectively;

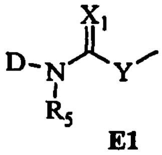
E1

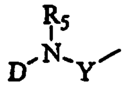
E2

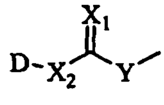
E3

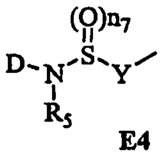
E4

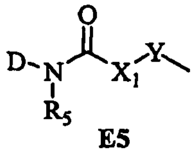
E5

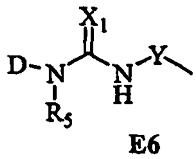
E6

Wherein, $X_1$, $X_2$ and $X_3$ are each O or S.

In the above Scheme, D is

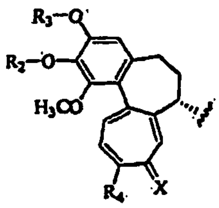
,

and $R_2$, $R_3$, $R_4$ and X are same as defined in the <Formula 1>;

$R_5$ is H or lower alkyl; $X_1$, $X_2$ and $X_3$ are each independently O or S; $Hal_1$ and $Hal_2$ are halogens; $Hal_1$ and $Hal_2$ of general formula (IV) and (IX) are each same or different halogens, for example F, C1, Br or I; P is conventional protecting group of hydroxy such as methoxymethyl, t-butyldimethylsilyl or benzyl; Y and Y' are same or different, and indicate following general formula (a'), (b'), (c'), (d') and (e') respectively,

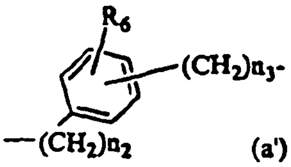
(a')

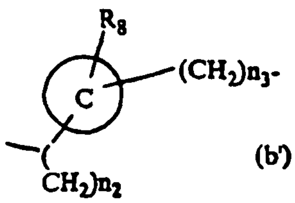
(b')

$-Z_1-$
(c')

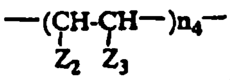
(d')

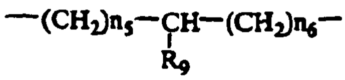
(e')

Wherein,

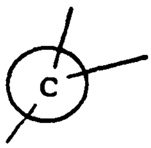
,

$R_6$, $R_8$, $R_9$, $Z_1$, $Z_2$, $Z_3$, $n_2$, $n_3$, $n_4$, $n_5$ and $n_6$ are same as defined in the <Formula 1>, $n_7$ and $n_6$ are integers of 1~2.

The preparation method for tricyclic derivatives of the present invention is illustrated more precisely hereinafter.

<Method 1>

According to method 1 of the present invention for the preparation of compounds of formula (lla) and (11b), the compound represented by formula (V) is prepared by amidation reaction making amine compound of formula (III) be reacted with halogen compound of formula (IV), which is step 1. In step 1, a base might be excluded, but the reaction is generally performed with a solvent such as dichloromethane, chloroform, tetrahydrofuran, diethylether, toluene or dimethylformamide etc., which have no influence on amidation reaction, in the presence of pyridine, triethylamine, diethylisopropylamine or N-methylmorpholine etc., a base that can be acceptable for amidation reaction in general. Reaction temperature is not limited in particular, but generally reaction can performed under cold temperature or elevated temperature, is performed preferably at room temperature.

In step 2, conversion of the compound of formula (V) prepared in the above step 1 to nitrooxy compound ($n_7$ - 2) of formula (IIa) and to nitrosooxy compound ($n_7$ = 1) of formula (IIa) was accomplished by nitration reaction and nitrosation reaction, respectively. Nitration reaction needs a compound that is able to convert halogen into nitrate, and is performed using silver nitrate ($AgNO_3$), t-butylammonium nitrate ($Bu_4NNO_3$), etc., in the presence of chloroform, acetonitrile, a mixture of acetonitrile and aqueous solution, or dichloromethane, which are all solvents not affecting the reaction. Nitrosation reaction might use a compound that is able to convert halogen into nitrosate, too, and is performed preferably using silver nitrite ($AgNO_2$) or sodium nitrite ($NaNO_2$) in the presence of chloroform, acetonitrile, a mixture of acetonitrile and aqueous solution, aqueous solution, or dichloromethane, which are also solvents not affecting the reaction. Reaction temperature is not limited in particular, but generally reaction can performed under cold temperature or elevated temperature, is performed preferably at room temperature.

Another way to give the compound of formula (IIa) is as follows; reaction of the compound of formula (III) with the compound of formula (VI) is performed to give the compound of formula (VII), and then, conversion of the compound of formula (VII) to the compound of formula (IIa) is accomplished. The reaction of the compound of formula (III) and the compound of formula (VI) is performed in the presence of a coupling agent such as 1-(3-dimethylaminopropyl)-3-ethylcarbodiimide(EDCI), 1-hydroxybenzotriazole hydrate(HOBT) or 1,3-dicyclohexyl carbodiimide(DCC). This reaction might be performed without a base, but generally with a base such as 4-dimethylaminopyridine, pyridine, triethylamine, diethylisopropylamine, N-methylmorpholine or dimethylphenylamine etc., which can be used in amidation reaction, in a solvent having no negative effect on the reaction, for example acetonitrile, dimethylformamide, dichloromethane, etc. Reaction temperature is not limited in particular, but generally reaction can performed under cold temperature or elevated temperature, is performed preferably at room temperature. Direct conversion of the compound of formula (VII) to the compound of formula (IIa) is accomplished by the reaction of alcohol with triphenylphosphin ($PPh_3$), N-bromosuccineimide (NBS) and silver nitrate, or silver nitrite. The reaction is performed in a solvent having no effect on the reaction such as chloroform, acetonitrile, dichloromethane, a mixture of acetonitrile and dichloromethane, etc. Reaction temperature is not limited in particular, but generally reaction is performed under cold temperature or at room temperature. Another way for conversion of the compound of formula (VII) to the compound of formula (IIa) is as follows; conversion of the compound of formula (VII) to halogen compound of formula (V) is accomplished first, and then conversion thereof to the compound of formula (IIa) is accomplished again. At this time, the conversion into halogen compound is performed by using a reagent that generally converts hydroxy group to halogen, for example tribromophosphin, tetrabromomethane etc., in the presence of chloroform, acetonitrile, dichloromethane etc., which are solvents having no negative effect on the reaction. Reaction temperature is not limited in particular, but generally reaction is performed under cold temperature or at room temperature.

Processes for preparing the compound of formula (IIb) of the method 1 of the present invention are as follows; conversion of hydrogen included in alcohol of formula (VII) to a leaving group such as mesylate, tosylate or triplate is accomplished, followed by reaction with potassium thioacetate, to give thioacetate ester compound. Hydrolysis of the compound in the presence of a base is accomplished to give the compound of formula (VIII). At this time, a base is selected among general bases that are able to hydrolyze an ester compound, for example sodium hydroxide, potassium hydroxide or sodium thiomethoxide. And an alcohol solution such as methanol or ethanol is preferred as a solvent for the reaction. Reaction temperature is not limited in particular, but generally reaction can performed under cold temperature or elevated temperature, is performed preferably at room temperature. Reaction of the compound of formula (VIII) with sodium nitrite under an acidic condition,'leads to the conversion of the compound to nitrosothio compound of formula (IIb). A solvent for the reaction is selected from a group consisting of methanol, ethanol, acetonitrile, a mixture of acetonitrile and aqueous solution, or dichloromethane etc., which is not to affect the reaction. Reaction temperature is not limited in particular, but generally reaction can performed under cold temperature or elevated temperature, is performed preferably at room temperature.

<Method 2>

According to method 2 of the present invention, compounds of formula (IIc) and (IId) are prepared. Particularly,

in step 1, reaction of the compound of formula (III) with the compound of formula (IX) is accomplished to give the compound of formula (X). This reaction is performed in analogy to the procedure described in method 1 in which conversion of the compound of formula (III) to the compound of formula (V) was accomplished by amidation reaction.

In step 2, conversion of the compound of formula (X) prepared in step 1 to the compound of formula (IIc) is accomplished by nitration reaction along with nitrosation reaction. This reaction is performed in analogy to the procedure described in method 1 in which conversion of the compound of formula (V) to the compound of formula (IIa) was accomplished.

Another way to give the compound of formula (IIc) is as follows; reaction of the compound of formula (III) with the compound of formula (XI) is performed to give the compound of formula (XII), and then, conversion of the compound of formula (XII) to the compound of formula (IIc) is accomplished. Reaction of the compound of formula (XI) with the compound of formula (III) is performed in analogy to the procedure described in method 1 in which conversion of the compound of formula (III) to the compound of formula (V) was accomplished by amidation reaction. Conversion of the compound of formula (XII) to the compound of formula (IIc) is accomplished under the same condition as provided for the conversion of the compound of formula (VII) to the compound of formula (IIa) in method 1.

Conversion of the compound of formula (XII) to the compound of formula (IId) is accomplished under the same condition as provided for the conversion of the compound of formula (VII) to the compound of formula (IIb) in method 1.

<Method 3>

According to method 3 of the present invention, compounds of formula (IIe) and (IIf) are prepared. Particularly, in step 1, reaction of the compound of formula (XIV) with the compound of formula (IV) is accomplished to give the compound of formula (XV). The reaction in this method is esterification reaction of alcohol ($X_2$=O) or thioalcohol ($X_2$=S) with acyl or thioacyl halide, which is performed in the presence of a base that is generally acceptable for esterification reaction. Preferable bases are pyridine, 4-dimethylaminopyridine, triethylamine, diethylisopropylamine, 2,6-lutidine, sodium hydride (NaH), cesium carbonate, or sodium hydroxide and can be used along with a phase transfer catalyst such as benzyltriethylammoniumchloride. Also, above reaction is preferably performed in a solvent having no negative effect on the reaction, for example dichloromethane, chloroform, tetrahydrofuran, diethylether, toluene, dimethylformamide, acetonitrile or aqueous solution. Reaction temperature is not limited in particular, but generally reaction can performed under cold temperature or elevated temperature, is performed preferably at room temperature.

In step 2, conversion of the compound of formula (XV) prepared in step 1 to the compound of formula (IIe) is accomplished by nitration reaction along with nitrosation reaction. This reaction is performed in analogy to the procedure described in method 1 in which conversion of the compound of formula (V) to the compound of formula (IIa) was accomplished.

Another way to give the compound of formula (IIe) is as follows; reaction of the compound of formula (XIV) with the compound of formula (VI') having a protective group in alcohol group is performed to give the compound of formula (XVII), followed by deprotection reaction to give the compound of formula (XVIII). Conversion of the resultant compound to the compound of formula (IIe) is accomplished. The reaction of the compound of formula (XIV) with the compound of formula (VI') is processed by esterification reaction of alcohol ($X_2$=O) or thioalcohol ($X_2$=S) and carboxylic acid or thiocarboxylic acid. The reaction is performed either in an aqueous solution supplemented with an acid such as hydrochloric acid, sulfuric acid, dodecylbenzene sulfonic acid or p-toluenesulfonic acid, at room temperature or under elevated temperature, or under the same condition provided for conversion of the compound of formula (III) to the compound of formula (VII) in method 1. Another esterification reaction is performed by Misunobu reaction using triphenylphosphine and diethyl azodicarboxylate in a solvent not affecting the reaction. And the solvent is preferably selected from a group consisting of dichloromethane, chloroform, tetrahydrofuran, diethylether, toluene or acetonitrile. Reaction temperature is not limited in particular, but generally reaction is performed under cold temperature or at room temperature. Protecting and deprotecting reaction of alcohol group is performed by known method in general organic synthesis.

Reaction of the compound of formula (XIV) with the compound of formula (XVI) is performed to give the compound of formula (XVIII) in analogy to the procedure described in method 3 in which conversion of the compound of formula (XIV) to the compound of formula (XV) was accomplished.

Conversion of the compound of formula (XVIII) to the compound of formula (IIe) is performed under the same condition provided for conversion of the compound of formula (VII) to the compound of formula (IIa) in method 1.

Conversion of the compound of formula (XVIII) to the compound of formula (IIf) is performed under the same condition provided for conversion of the compound of formula (VII) to the compound of formula (IIb) in method 1.

<Method 4>

According to method 4 of the present invention, compounds of formula (IIg) and (IIh) are prepared. Particularly, in step 1, reaction of the compound of formula (III) with the compound of formula (XX) is accomplished to give the

compound of formula (XXI).

When the compound of formula (IIg) is sulfinylamide ($n_6$=1), the reaction of the compound of formula (III) with sulfinylhalide of formula (XX) is performed without a base or with a base that is applicable to amidation reaction, for example pyridine, triethylamine, diethylisopropylamine, N-methylmorpholine or dimethylphenylamine, in a solvent having no negative effect on the reaction such as dichloromethane, chloroform, tetrahydrofuran, diethylether, toluene or dimethylformamide. Reaction temperature is not limited in particular, but generally reaction can performed under cold temperature or elevated temperature, is performed preferably at room temperature.

When the compound of formula (IIg) is sulfonylamide ($n_8$=2), the reaction of the compound of formula (III) with sulfonylhalide of formula (XX) is performed either without a base or with a base that is applicable to amidation reaction in general, for example pyridine, triethylamine, diethylisopropylamine, N-methylmorpholine, sodium hydroxide, sodium carbonate or potassium carbonate, in a solvent having no negative effect on the reaction such as dichloromethane, chloroform, tetrahydrofuran, diethylether, toluene or dimethylformamide. Reaction temperature is not limited in particular, but generally reaction can performed under cold temperature or elevated temperature, is performed preferably at room temperature.

In step 2, conversion of the compound of formula (XXI) prepared in step 1 to the compound of formula (IIg) is performed under the same condition provided for conversion of the compound of formula (VII) to the compound of formula (IIa) in method 1.

Conversion of the compound of formula (XXI) to the compound of formula (IIh) is performed under the same condition provided for conversion of the compound of formula (VII) to the compound of formula (IIb) in method 1.

<Method 5>

According to method 5 of the present invention, compounds of formula (IIi) and formula (IIj) are prepared as follows. In step 1, reaction of the compound of formula (III) with the compound of formula (XXIII) having a protecting group to alcohol group is performed, followed by deprotection reaction. Conversion of the compound of formula (XXIV) resulted from the above reaction to the compound of formula (IIi) is accomplished. At this time, the reaction of the compound of formula (III) with the compound of formula (XXIII) is performed by using a coupling reagent such as carbonyl dichloride, triphosgen, di-t-butyl dicarbonate or 1,1'-carbonyl diimidazole etc. The reaction can be performed either without a base or with a base that is generally acceptable for amidation reaction, for example pyridine, triethylamine, diethylisopropylamine, N-methylmorpholine or dimethylphenylamine, in a solvent having no negative effect on the reaction such as dichloromethane, chloroform, tetrahydrofuran, diethylether, ethanol or dimethylformamide. Reaction temperature, is not limited in particular, but generally reaction is performed under cold temperature or at room temperature. And deprotection reaction is performed by known method in general organic synthesis.

In step 2, conversion of the compound of formula (XXIV) prepared in step 1 to the compound of formula (IIi) is performed under the same condition provided for conversion of the compound of formula (VII) to the compound of formula (IIa) in method 1.

Conversion of the compound of formula (XXIV) to the compound of formula (IIj) is performed under the same condition provided for conversion of the compound of formula (VII) to the compound of formula (IIb) in method 1.

<Method 6>

According to method 6 of the present invention, compounds of formula (IIk) and formula (III) are prepared as follows. In step 1, reaction of the compound of formula (III) with the compound of formula (XXVI) having a protecting group to alcohol group is performed, followed by deprotection reaction. Then, conversion of the resultant compound of formula (XXVII) to the compound of formula (IIk) is accomplished. At this time, the reaction of the compound of formula (III) with the compound of formula (XXVI) is performed either without a base or with a base that is acceptable for amidation reaction, for example pyridine, triethylamine, diethylisopropylamine or N-methylmorpholine etc, in a solvent having no negative effect on the reaction such as dichloromethane, chloroform, tetrahydrofuran, diethylether, benzene, acetonitrile, etc. Reaction temperature is not limited in particular, but generally reaction is performed under cold temperature or at room temperature. Protecting and deprotecting reaction of alcohol group is performed by known method in general organic synthesis.

In step 2, conversion of the compound of formula (XXVII) prepared in step 1 to the compound of formula (IIk) is performed under the same condition provided for conversion of the compound of formula (VII) to the compound of formula (IIa) in method 1.

Conversion of the compound of formula (XXVII) to the compound of formula (III) is performed under the same condition provided for conversion of the compound of formula (VII) to the compound of formula (IIb) in method 1.

<Method 7>

According to method 7 of the present invention, compounds of formula (IIm) and formula (IIn) are prepared from the compound of formula (XXIX) in analogy to the procedure described in method 3.

<Method 8>

According to method 8 of the present invention, compounds of formula (IIo) and formula (IIp) are prepared as follows. In step 1, reaction of the compound of formula (XXIX) with the compound of formula (IX') is performed to give a compound of formula (XXXIV). This reaction is performed by etherification reaction of alcohol ($X_2$=O) or thioalcohol ($X_2$=S) with alkylhalide in the presence of a base acceptable for etherification reaction. As a base suitable for that purpose, sodium hydride (NaH), t-potassium butoxide (t-BuOK), n-BuLi, sodium hydroxide, potassium hydroxide and a phase transfer catalyst such as benzyltriethylammoniumchloride etc, or crown ether is preferred. The reaction is performed preferably in a solvent having no negative effect on the reaction, for example dichloromethane, chloroform, tetrahydrofuran, diethylether, toluene, dimethylformamide, aqueous solution, dimethylsufoxide or benzene, etc. Reaction temperature is not limited in particular, but generally reaction can performed under cold temperature or elevated temperature, is performed under cold temperature or at room temperature.
In step 2, conversion of the compound of formula (XXXIV), prepared in the above step 1, to the compound of formula (IIo) is accomplished by nitration reaction or nitrosation reaction. This reaction is performed in analogy to the procedure described in method 1 in which conversion of the compound of formula (V) to the compound of formula (IIa) was accomplished.
Another way to prepare the compound of formula (IIo) is as follows. Reaction of the compound of formula (XXIX) with the compound of formula (XI') having a protecting group to alcohol group is performed, followed by deprotection reaction to give the compound of formula (XXXV). Conversion of the compound of formula (XXXV) to the compound of formula (IIo) is accomplished. Reaction of the compound of formula (XXIX) with the compound of formula (XI') is performed under the same condition given for conversion of the compound of formula (XXIX) to the compound of formula (XXXIV) accomplished by etherification reaction in method 8.
Conversion of the compound of formula (XXXV) to the compound of formula (IIo) is performed under the same condition provided for conversion.of the compound of formula (VII) to the compound of formula (IIa) in method 1.
Conversion of the compound of formula (XXXV) to the compound of formula (IIp) is performed under the same condition provided for conversion of the compound of formula (VII) to the compound of formula (IIb) in method 1.
The target compounds given by the above reactions can be separated and purified by general methods such as column chromatography, recrystallisation, etc.
The present invention provides also a pharmaceutical composition containing tricyclic derivatives represented by the <Formula 1> or pharmaceutically acceptable salts thereof as an effective ingredient.
Tricyclic derivatives according to the present invention or pharmaceutically acceptable salts thereof show very strong cytotoxicity to cancer cell lines but have much less toxicity to test animals than colchicine or taxol injection has.
When tricyclic derivatives of the present invention were administered to a BALB/c nude mouse transplanted with human lung cancer cell line NCI-H460, the size and the weight of a tumor were remarkably decreased in proportion to the dosage.
Tricyclic derivatives of the present invention also have a strong activity of antiangiogenesis in HUVEC cells.
Therefore, tricyclic derivatives of the present invention or pharmaceutically acceptable salts thereof can be effectively used as an anticancer agent, an anti-proliferation agent and an angiogenesis inhibitor.
The composition of the present invention might additionally include, in addition to tricyclic derivatives or pharmaceutically acceptable salts thereof, at least one of active ingredients having the same or similar function to the mentioned tricyclic derivatives or pharmaceutically acceptable salts thereof.
The said tricyclic derivatives or pharmaceutically acceptable salts thereof can be administered orally or parenterally and be prepared in general forms of pharmaceutical formulation. The tricyclic derivatives of the present invention or pharmaceutically acceptable salts thereof can be prepared for oral or parenteral administration by mixing with generally used fillers, extenders, binders, wetting agents, disintegrant, diluents such as surfactants, or excipients. Solid formulations for oral administration are tablets, pills, powders, granules and capsules. These solid formulations are prepared by mixing one or more suitable excipients such as starch, calcium carbonate, sucrose, lactose and gelatin, etc. Except for the simple excipients, lubricants, for example magnesium stearate, talc, etc, can be used. Liquid formulations for oral administrations are suspensions, solutions, emulsions and syrups, and the above mentioned formulations can contain various excipients such as wetting agents, sweeteners, aromatics and preservatives in addition to generally used simple diluents such as water and liquid paraffin. Formulations for parenteral administration are sterilized aqueous solutions, water-insoluble excipients, suspensions, emulsions, lyophilized agent and suppositories. Water insoluble excipients and suspensions can contain, in addition to the active compound or compounds, propylene glycol, polyethylene glycol,

vegetable oil like olive oil, injectable ester like ethylolate, etc. Suppositories can contain witepsol, macrogol, tween 61, cacao butter, laurin butter, glycerol and gelatin.

The composition of the present invention can be prepared for either oral or parenteral administration (for example, intravenous, subcutaneous, intraperitoneal or local injection), and dosage is determined by weight, age, gender, condition of health and diet of a patient and administration method, excretion rate and severity of a disease. The preferable effective dosage of the tricyclic derivatives of the present invention is 3~300 mg/kg (body weight), and administration times are once or several times per day.

EXAMPLES

Practical and presently preferred embodiments of the present invention are illustrative as shown in the following Examples.

However, the present invention is not limited by following examples.

7-Deacetylcolchicine used in the below examples was prepared by the method described in [EP 0493064; Synthetic Communications 1997, 27(2), 293-296].

7-Amino-1,2,3-trimethoxy-10-methylsulfanyl-6,7-dihydro-5*H*-benzo[a]-heptalen-9-one was prepared by the method described in (WO 9421598; Bioorganic & Medicinal Chemistry, Vol 5, No. 12, pp 2277-2282, 1997).

Thiodemecolcine was prepared by the method described in (J. Med. Chem, 1985, 28, 1204-1208).

(7S)-7-Amino-3-cyclopentyloxy-1,2-dimethoxy-10-methylsulfanyl-6,7-dihydro-5H-benzo[a]heptalen-9-one, (7S)-7-amino-3-isopropoxy-1,2-dimethoxy-10-methylsulfanyl-6,7-dihydro-5H-benzo[a]heptalen-9-one, (7S)-7-amino-3-ethoxy-1,2-dimethoxy-10-methylsulfanyl-6,7-dihydro-5H-benzo[a]heptalen-9-one were prepared by the method described in (WO 9611184).

Example 1: Preparation of 6-nitrooxymethyl-*N*-[(7S)-1,2,3-trimethoxy-10-methylsulfanyl-9-oxo-5,6,7,9-tetrahydro-benzo[a]heptalen-7-yl]-nicotineamide

<Step 1> Preparation of 6-hydroxymethyl-*N*-[(7S)-1,2,3-trimethoxy-10-methylsulfanyl-9-oxo-5,6,7,9-tetrahydro-benzo[a]heptalen-7-yl]-nicotineamide

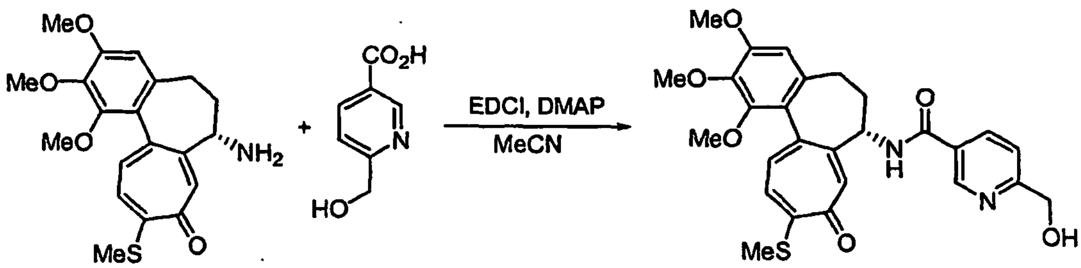


6-Hydroxymethyl-nicotinic acid was synthesized by the method described in (Bioorg. Med. Chem. Lett, 1996, 6, 3025-3028).

To a solution of 7-amino-1,2,3-trimethoxy-10-methylsulfanyl-6,7-dihydro-5*H*-benzo[a]-heptalen-9-one (300 mg, 0.80 mmol), 6-hydroxymethylnicotinic acid (135 mg, 0.88 mmol) and DMAP (60 mg, 0.48 mmol) in 10 mℓ of acetonitrile was added EDCI (308 mg, 1.60 mmol) at 0°C. The reaction mixture was stirred at room temperature for 2 hours. Water was added to quench the reaction, and aqueous layer was extracted with ethyl acetate. Combined organic layer was dried over anhydrous sodium sulfate, filtered and concentrated under reduced pressure. The residue was purified by column chromatography (ethyl acetate:methanol = 8:1), to give 244 mg (yield: 60%, solid having yellow color) of the target compound.

$^1$H NMR (400MHz, $CDCl_3$) : δ2.07-2.15(m, 1H), 2.31-2.44(m, 2H), 2.45(s, 3H), 2.56-2.59(m, 1H), 3.75(s, 3H), 3.91(s, 3H), 3.97(s, 3H), 4.66(q, J=10.2Hz, 2H), 4.90-4.93(m, 1H), 6.56(s, 1H), 7.13(t, J=9.1Hz, 2H), 7.40(d, J=10.2Hz, 1H), 7.52(s, 1H), 8.15(dd, J=2.2, 5.8Hz, 1H), 8.80(d, J=6.9Hz, 1H), 8.96(s, 1H)

<Step 2> Preparation of 6-nitrooxymethyl-*N*-[(7*S*)-1,2,3-trimethoxy-10-methylsulfanyl-9-oxo-5,6,7,9-tetrahydro-benzo [a]heptalen-7-yl]-nicotineamide

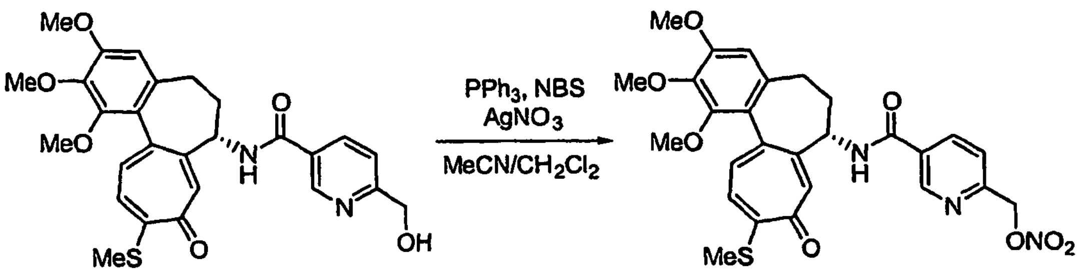


The compound (100 mg, 0.19 mmol) prepared in the step 1 of Example 1 and triphenylphosphine (57 mg, 0.21 mmol) were dissolved in acetonitrile/dichloromethane (1.25 ml/0.5 ml), therein NBS (42 mg, 0.23 mmol) was added at -35°C. The reaction mixture was stirred for 20 minutes. Thereafter, therein silver nitrate (40 mg, 0.23 mmol) was slowly added dropwise at room temperature, the reaction mixture was stirred at room temperature for 18 hours. Water was added to quench the reaction, and aqueous layer was extracted with chloroform. Combined organic layer was dried over anhydrous sodium sulfate, filtered and concentrated under reduced pressure. The residue was purified by column chromatography (chloroform:methanol = 10:1), to give 18 mg (yield: 35%, solid having yellow color) of the target compound.

$^1$H NMR (400MHz, $CDCl_3$): δ2.09-2.13(m, 1H), 2.31-2.43(m, 2H), 2.46(s, 3H), 2.55-2.64(m, 1H), 3.75(s, 3H), 3.91(s, 3H), 3.97(s, 3H), 4.93-4.98(m, 1H), 5.50(s, 2H), 6.56(s, 1H), 7.16(t, J=10.9Hz, 1H), 7.26(d, J=8.8Hz, 1H), 7.40 (d, J=10.6Hz, 1H), 7.59(s, 1H), 8.27(dd, J=2.2, 5.8Hz, 1H), 8.77(d, J=7.3Hz, 1H), 9.08(s, 1H)

Example 2 , Reference Examples 3 and 4

Compounds of Example 2 and Reference Examples 3 and 4 were synthesized in analogy to the procedure as described in Example 1, and intermediates were prepared by the method described as follows.

<Intermediate 1> Preparation of 5-hydroxymethyl-furan-2-carboxylic acid

5-Hydroxymethyl-furan-2-carboxylic acid was synthesized by the method described in (Helv. Chim. Acta, 1926, 9, 1068).

<Intermediate 2> Preparation of 3-hydroxymethylbenzoic acid

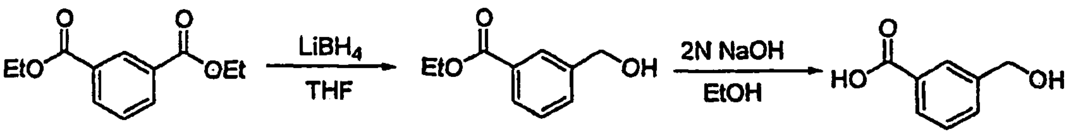


Isophthalic acid diethylester (9.100 g, 40.95 mmol) was dissolved in tetrahydrofuran (20ml). Thereafter, therein lithiumborohydride (11.26 ml, 22.52 mmol, 2M tetrahydrofuran solution) was slowly added dropwise, and the reaction mixture was refluxed for 3 hours. Water was added to quench the reaction, and aqueous layer was extracted with ethyl acetate. Combined organic layer was dried over anhydrous magnesium sulfate, filtered and concentrated under reduced pressure. The residue was purified by column chromatography (hexane:ethyl acetate = 2:1), to give 5.76 g (yield: 77.1%, colorless liquid) of 3-hydroxymethylbenzoic acid ethylester.

Ester compound (1.317 g, 7.311 mmol) prepared above was dissolved in ethanol (6 ml). Thereafter, therein 2N NaOH aqueous solution (11.0 ml, 21.93 mmol) was slowly added dropwise, and the reaction mixture was stirred at room temperature for 1 hour. The reaction mixture was neutralized with 1% HCl aqueous solution, extracted with ethyl

acetate, and washed with saturated NaCl solution. Combined organic layer was dried over anhydrous magnesium sulfate, filtered and concentrated under reduced pressure. The residue was purified by column chromatography (ethyl acetate: methyl alcohol = 5:1), to give 1.03 g (yield: 99.2%, white solid) of the target compound.

$^1$H NMR (400MHz, $CD_3OD$) : δ4.66(s, 2H), 7.44 (t, *J*=7.7Hz, 1H), 7.58(d, *J*=7.7Hz, 1H), 7.92(d, *J*=7.7Hz, 1H), 8.04(s, 1H)

Example 2: 5-nitrooxymethyl-furan-2-carboxylic acid-[(7S)-1,2,3-trimethoxy-10-methylsulfanyl-9-oxo-5,6,7,9-tetrahydro-benzo[a]heptalen-7-yl]-amide

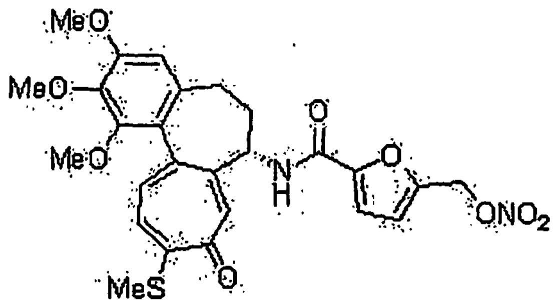

$^1$H NMR (400MHz, $CDCl_3$) : δ2.37-2.51(m, 3H), 2.46(s, 3H), 2.61-2.92(m, 1H) , 3.74(s, 3H), 3.93(s, 3H), 3.98 (s, 3H), 4.82-4.85(m, 1H), 4.85(d, J=13.2Hz, 1H), 4.90(d, J=13.2Hz, 1H), 6.39(s, 1H), 6.58(s, 1H), 6.64(s, 1H), 7.16 (d. J=10.6Hz, 1H), 7.42(d, J=10.2Hz, 1H), 7.72(s, 1H), 8.99(*s*, 1H)

Reference Example 3: N- [(7S)-3-isopropoxy-1,2-dimethoxy-10-methylsulfanyl-9-oxo-5,6,7,9-tetrahydro-benzo[a]heptalen-7-yl]-3-nitrooxymethyl-benzamide

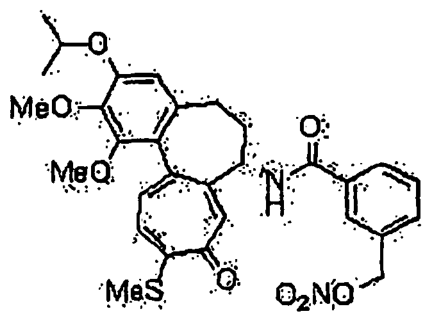

$^1$H NMR (400MHz, $CDCl_3$): δ1.42(t, J=6.6Hz, 6H), 2.22-2.27(m, 1H), 2.34-2.49(s, 2H) , 2.54-2.57(m, 1H), 3.75 (s, 3H), 3.96(s, 3H), 4.59-4.63 (m, 1H), 4.93-4.97(m, 1H), 5.23(q, J=.13.0Hz, 2H) , 6.56(s, 1H), 7.15 (d, J=10.6Hz, 1H), 7.18-7.25(m, 1H), 7.33(d, J=8.0Hz, 2H), 7.46(d, J=10.2Hz, 1H), 7.64(s, 1H),7.71(d, J=8.0Hz, 1H), 7.88(s, 1H), 8.40(s, 1H)

Reference Example - 4: N-[(7S)-3-ethoxy-1,2-dimethoxy-10-methylsulfanyl-9-oxo-5,6,7,9-tetrahydro-benzo[a]heptalen-7-yl]-3-nitrooxymethyl-benzamide

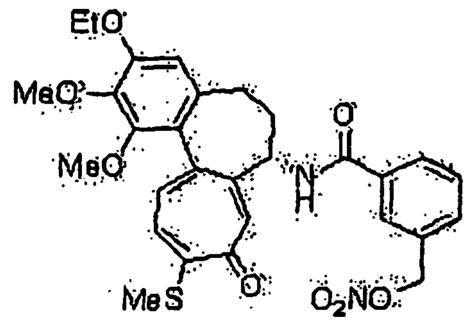

$^1H$ NMR (400MHz, $CDCl_3$): δ1.49(t, J=6.9Hz, 3H), 2.17-2.24(m, 1H), 2.34-2.47(m, 2H), 2.45(s, 3H), 2.49-2.58 (m, 1H), 3.75(s, 3H), 3.97(s, 3H), 4.12-4.15(m, 2H), 4.89-4.96(m, 1H), 5.24(q, J=12.0Hz, 2H), 6.56(s, 1H), 7.15(d, J=10.2Hz, 1H), 7.21-7.25(m, 1H), 7.31-7.35 (m, 1H), 7.41(d, J=10.6Hz, 1H), 7.60(s, 1H), 7.71(d, J=7.3Hz, 1H), 7.78(s, 1H), 8.23(s, 1H)

Example 5: Preparation of 6-nitrooxymethyl-pyridine-2-carboxylic acid-[(7S)-1,2,3-trimethoxy-10-methylsulfanyl-9-oxo-5,6,7,9-tetrahydro-benzo[a]heptalen-7-yl]-amide

<Step 1> Preparation of 6-hydroxymethyl-pyridine-2-carboxylic acid-[(7*S*)-1,2,3-trimethoxy-10-methylsulfanyl-9-oxo-5,6,7,9-tetrahydro-benzo[a]heptalen -7-yl]-amide

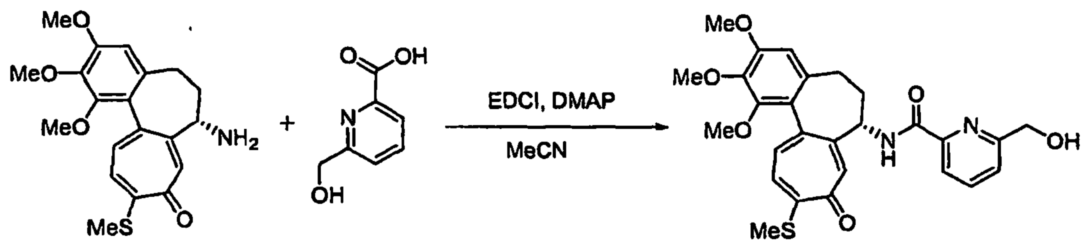


According to the similar procedure in the step 1 of Example 1, by using 6-hydroxymethyl pyridine-2-carboxylic acid (23 mg, 0.17 mmol), 35 mg (yield: 53%, yellow solid) of the target compound was obtained.

$^1H$ NMR (400MHz, $CDCl_3$): δ2.45(s, 3H), 2.33-2.50(m, 3H) 2.52-2.73(m, 1H), 3.75(s, 3H), 3.92(s, 3H), 3.97(S, 3H), 4.28(d, *J*=14Hz, 1H), 4.44(d, *J*=14Hz, 1H) 4.86-4.92(m, 1H), 6.57(s, 1H), 7.13(d, *J*=10.4Hz, 1H) , 7.37-7.48(m, 4H), 7.73(s, 1H), 9.80(d, *J*=8Hz, 1H)

<Step 2> Preparation of 6-bromomethyl-pyridine-2-carboxylic acid-[(7S)-1,2,3-trimethoxy-10-methylsulfanyl-9-oxo-5,6,7,9-tetrahydro0benzo[a]heptalen -7-yl]-amide

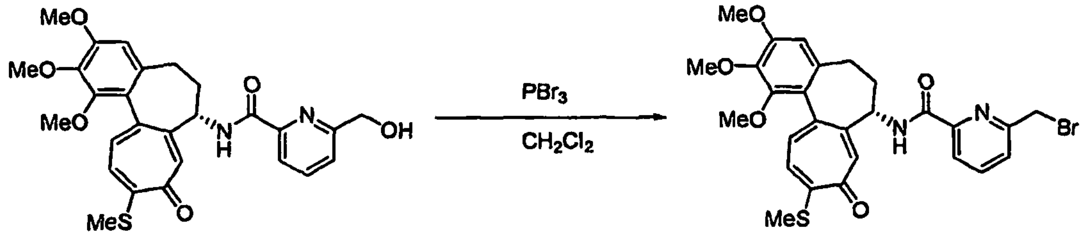

A compound (50 mg, 0.098 mmol) prepared in the step 1 was dissolved in dichloromethane (6 ml). Thereafter, therein tribromophosphine ($PBr_3$, 0.005 ml, 0.05 mmol) was slowly added dropwise at 0°C, and the reaction mixture was stirred at room temperature for 3 hours. Methanol was added to quench the reaction. Combined organic layer was dried over anhydrous magnesium sulfate, filtered and and concentrated under reduced pressure. The residue was purified by column chromatography (chloroform:methanol = 99:1), to give 40 mg (yield: 71%, yellow solid) of the target compound.

$^1$H NMR (400MHz, $CDCl_3$) : δ2.06-2.14(m, 1H), 2.33-2.41(m, 1H), 2.41(s, 3H), 2.46-2.54(m, 1H) 2.58-2.63(m, 1H), 3.72(s, 3H), 3.90(s, 3H), 3.95(S, 3H), 4.55(d, *J*=2.8Hz, 2H), 4.77-4.83(m, 1H), 6.57(s, 1H), 7.04(d, *J*=10.4Hz, 1H), 7.29(s, 1H), 7.31(d, *J*=10.4Hz, 1H), 7.58(d, *J*=7.6Hz, 1H), 7.78(t, *J*=7.6Hz, 1H), 7.90(d, *J*=8.0Hz, 1H), 8.53(d, *J*=7.6Hz, 1H)

<Step 3> Preparation of 6-nitrooxymethyl-pyridine-2-carboxylic acid-[(7S)-1,2,3-trimethoxy-10-methylsulfanyl-9-oxo-5,6,7,9-tetrahydro-benzo [a] heptalen -7-yl] -amide

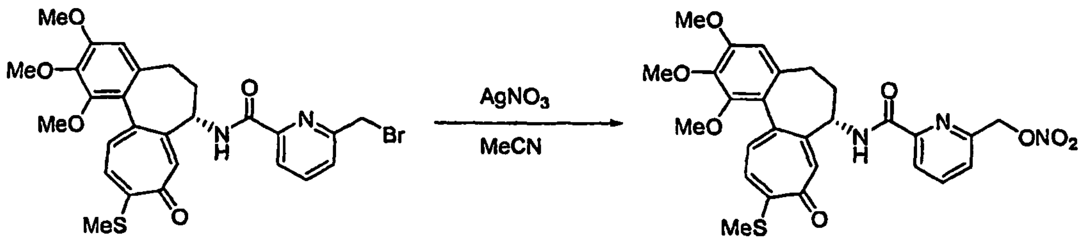


A compound (40 mg, 0.070 mmol) prepared in the step 2 was dissolved in acetonitrile (3ml). Thereafter, therein silver nitrate (23 mg, 0.14 mmol) was slowly added dropwise, and the reaction mixture was stirred at room temperature for 12 hours. The reaction mixture was washed with water and concentrated under reduced pressure. The residue was purified by column chromatography (chloroform:methanol = 99:1), to give 17 mg (yield: 44.7%, yellow solid) of the target compound.

$^1$H NMR (400MHz, $CDCl_3$) : δ2.03-2.10(m, 1H), 2.33-2.41(m, 1H), 2.43(s, 3H), 2.46-2.54(m, 1H) 2.58-2.65(m, 1H), 3.73(s, 3H), 3.92(s, 3H), 3.95(S, 3H), 4.77-4.83(m, 1H), 5.63(d, *J*=3.2Hz, 2H), 6.58(s, 1H), 7.06(d, *J*=10.4Hz, 1H), 7.27(s, 1H), 7.31(d, *J*=10.4Hz, 1H), 7.53(d, *J*=7.6Hz, 1H) , 7.88(t, *J*=8.0Hz, 1H), 8.02(d, *J*=7.2Hz, 1H), 8.39(d, *J*=7.2Hz, 1H)

Example 6~24

Compounds of Example 6 - Example 24 were synthesized in analogy to the procedure as described in Example 5, and intermediates were prepared by the method described as follows.

<Intermediate 3> Preparation of 6-hydroxymethyl-pyridine-2-carboxylic acid

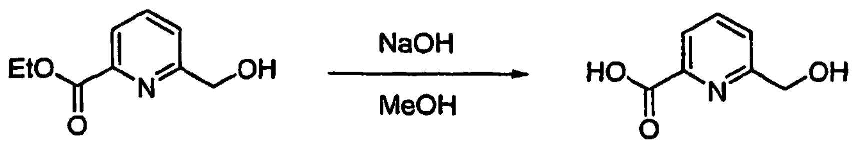


6-Hydroxymethyl-pyridine-2-carboxylic acid ethylester (200 mg, 1.1 mmol) (J. Amer. Chem. Soc, 1982, 104, 2251-2257) was dissolved in methanol (1 ml). Thereafter, therein 2N NaOH aqueous solution (1 ml) was slowly added dropwise, and the reaction mixture was stirred at room temperature for 1 hour. The reaction mixture was acidified (pH=3) with 2N HCl. Solvent was concentrated under reduced pressure, then, dissolved in methanol and filtered. The filtrate was concentrated under reduced pressure to give 150 mg (yield: 89%, white solid) of the target compound.

$^1$H NMR (400MHz, $CD_3OD$) : δ5.05(s, 2H), 8.34 (d, *J*=8.0Hz, 1H), 8.47(d, *J*=8.0Hz, 1H) 8.73(d, *J*=8.0Hz, 1H)

<Intermediate 4> Preparation of 5-hydroxymethyl-thiophene-2-carboxylic acid

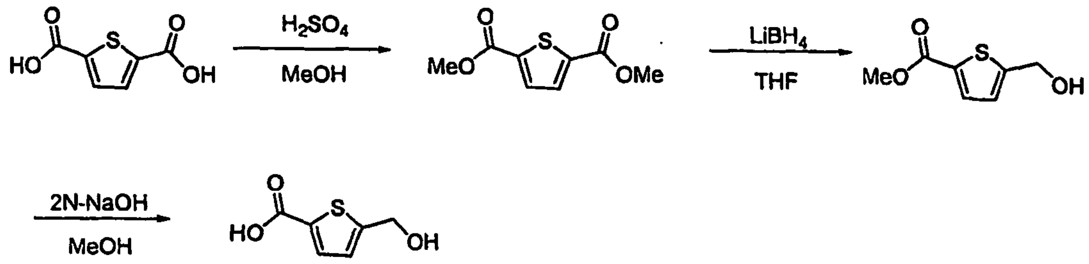


Thiophene-2,5-dicarboxylic acid (4 g, 23.3 mmol) was dissolved in methanol (300ml). Catalytic amount of sulfuric acid was slowly added therein. The reaction mixture was refluxed to give 3.8 g (yield: 81.7%, white solid) of thiophene-2,5-dicarboxylic acid dimethylester. The thiophene-2,5-dicarboxylic acid dimethylester (3.7 g, 18.84 mmol) was dissolved in anhydrous tetrahydrofuran (50 ml) at room temperature under a nitrogen atmosphere. 2.0M Lithiumborohydride tetrahydrofuran solution (5.5 ml, 11 mmol) was slowly added therein at 0°C. The reaction mixture was refluxed for 3 hours to give 2.1 g (yield: 64.7%, white solid) of 5-hydroxymethyl-thiophene-2-carboxylic acid methylester. 5-Hydroxymethyl-thiophene-2-carboxylic acid methyl ester (2.1 g, 12.2 mmol) was dissolved in methanol (20 ml). 2N NaOH aqueous solution (15ml) was slowly added therein. The reaction mixture was stirred at room temperature for 1 hour to give 1.75 g (yield: 89%, white solid) of the target compound.

$^1$H NMR (400MHz, $CDCl_3$) : δ3.90(Br, 1H), 4.79(d, *J*=0.8Hz, 2H), 6.97(d, *J*=4Hz, 1H), 7.66(d, *J*=4Hz, 1H)

<Intermediate 5> Preparation of 2-fluoro-3-hydroxymethyl-benzoic acid

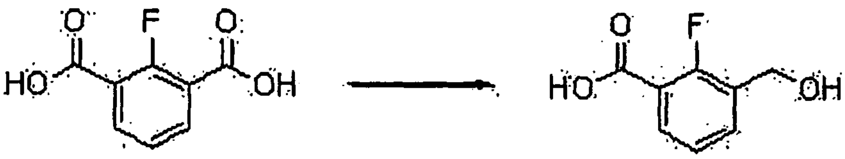

2-Fluoroisophthalic acid (3 g, 16.3 mmol) (J. Amer. Chem. Soc., 1943, 65, 2308) was dissolved in methanol (150 ml). Catalytic amount of sulfuric acid was slowly added therein. The reaction mixture was refluxed. The resultant was concentrated under reduced pressure to remove solvent, and dissolved in ethyl acetate. Combined organic layer was washed with saturated sodium carbonate, dried over anhydrous sodium sulfate and concentrated under reduced pressure. The residue was purified by column chromatography (ethyl acetate:hexane = 1:2), to give 3.1 g (yield: 88%, white solid) of 2-fluoro-isophthalic acid dimethylester. Thiophene-2,5-dicarboxylic acid dimethylester (3.1 g, 14.6 mmol) was dissolved in anhydrous tetrahydrofuran (50 ml) at room temperature under a nitrogen atmosphere. 2.0M Lithiumborohydride tetrahydrofuran solution (4.4 ml, 8.7 mmol) was slowly added therein at 0°C. The reaction mixture was refluxed for 3 hours. The reaction mixture was acidified with 1N HCl aqueous solution, concentrated under reduced pressure to remove solvent, and extracted with chloroform. Combined organic layer was dried over anhydrous sodium sulfate, filtered, and solvent was concentrated under reduced pressure. The residue was purified by column chromatography (dichloromethane:methanol = 99:1), to give 1.5 g (yield: 58%, white solid) of 2-fluoro-3-hydroxymethyl-benzoic acid methylester. The 2-fluoro-3-hydroxymethyl-benzoic acid methylester (1.3 g, 7.6 mmol) was dissolved in methanol (20 ml). 2N NaOH aqueous solution (14 ml) was slowly added therein. The reaction mixture was stirred at room temperature for 1 hour. The reaction mixture was acidified (pH=3) with 2N HCl. Solvent was concentrated under reduced pressure, then, dissolved in methanol and filtered. The filtrate was concentrated under reduced pressure to give 1.15 g (yield: 88%, white solid) of the target compound.

$^1$H NMR (400MHz, DMSO-$d_6$) : δ4.58 (d, *J*=5.2Hz, 2H),5.37(t, *J*=5.2Hz, 1H) 7.28(t, *J*=8Hz, 1H), 7.68(1, *J*=8Hz,

1H), 7.74(t, J=8Hz, 1H)

<Intermediate 6> Preparation of 3-fluoro-5-hxdroxymethxl-benzoic acid

<Step 1> Preparation of 3-fluoro-5-hydroxymethyl-benzoic acid methylester

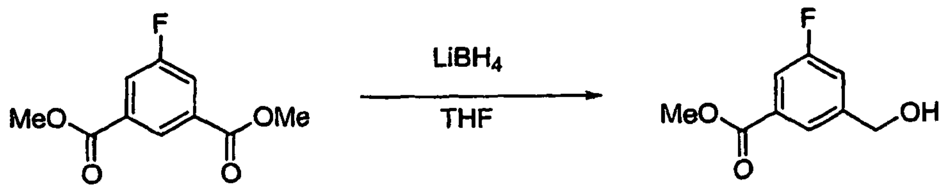


5-Fluoroisophthalic acid dimethylester (1.6 g, 7.54 mmol) (J. Org. Chem; 1969, 34, 1960-1961) was dissolved in tetrahydrofuran (15 ml). Thereafter, therein 2.0 M lithiumborohydride tetrahydrofuran solution (2.6 ml, 5.27 mmol) was slowly added dropwise at 0°C, and the reaction mixture was refluxed for 3 hours. The reaction mixture was acidified with 1N HCl aqueous solution, concentrated under reduced pressure to remove solvent, and extracted with chloroform. Combined organic layer was dried over anhydrous sodium sulfate, filtered, and concentrated under reduced pressure. The residue was purified by column chromatography (dichloromethane:methanol = 99:1), to give 800 mg (yield: 57%, white solid) of the target compound.

$^1H$ NMR (400MHz, $CDCl_3$)1 δ3.92(d, J=1.6Hz, 3H), 4.75(d, J=4Hz, 2H), 7.29-7.32(m, 1H), 7.61(dd, J=9.2, 1.6Hz, 1H), 7.8(d, J=0.8Hz, 1H)

<Step 2> Preparation of 3-fluoro-5-hydroxymethyl-benzoic acid

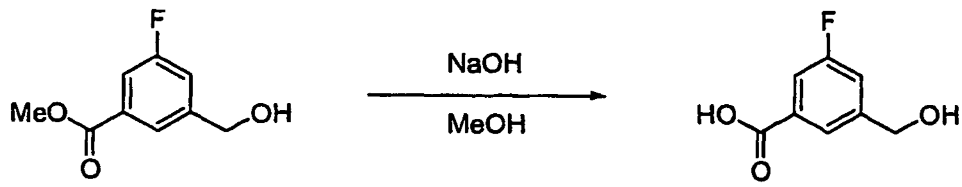


According to the similar procedure in the method of intermediate 3, by using a compound prepared in the step 1, 1.6 g (yield: 94%, white solid) of the target compound was obtained.

$^1H$ NMR (400MHz, $CD_3OD$) : δ4.66(d, J=0.8Hz, 2H), 7.33-7.36(m, 1H), 7.56-7.59(m, 1H), 7.83-7.84(m, 1H)

<Intermediate 7> Preparation of 4-fluoro-3-hydroxymethyl-benzoic acid

<Step 1> Preparation of 4-fluoro-isophthalic acid

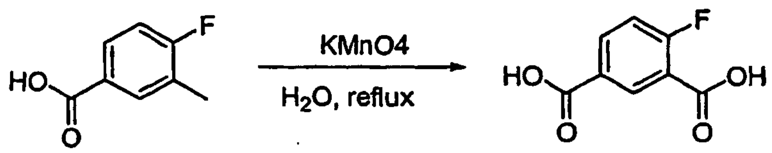


4-Fluoro-3-methyl-benzoic acid (2.52 g, 16.346 mmol) and potassium permanganate (10.33 g, 65.382 mmol) were dissolved in aqueous solution (300 ml), and the mixture was refluxed for 1 day. The reaction mixture was filtered

and the resultant solution was cooled down at room temperature, then conc. HCl solution was added thereto. The produced solid was heated until it was completely melted. The temperature was lowered again into room temperature and then the solid was filtered, to give 2.08 g (yield: 69.1%, white solid) of the target compound.

$^1$H NMR (400MHz, $CD_3OD$) : δ7.32(dd, *J*=10.4, 8.6Hz, 1H), 8.21-8.25(m, 1H), 8.59(dd, *J*=7.0, 2.4Hz, 1H)

<Step 2> Preparation of 4-fluoro-isophthalic acid dimethylester

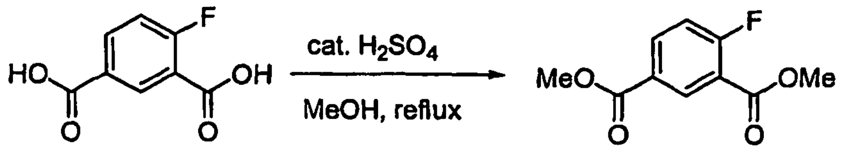


A compound prepared in the step 1 (2.08 g, 11.29 mmol) was dissolved in methanol (30 ml). Then, 10 drops of conc. sulfuric acid were added therein. The reaction mixture was refluxed for 1 day, neutralized with saturated sodium hydrogen carbonate aqueous solution, and extracted with chloroform. Combined organic layer was dried over anhydrous sodium sulfate, filtered, and concentrated under reduced pressure, to give 2.21 g (yield: 92.2%, white solid) of the target compound.

$^1$H NMR (400MHz, $CDCl_3$): δ3.96(s, 3H), 3.97(s, 3H), 7.22(dd, *J*=10.3, 8.8Hz, 1H), 8.20-8.23(m, 1H), 8.64(dd, *J*=7.0, 2.2Hz, 1H)

<Step 3> Preparation of 4-fluoro-3-hydroxymethyl-benzoic acid methylester and 2-fluoro-5-hydroxymethyl-benzoic acid methylester

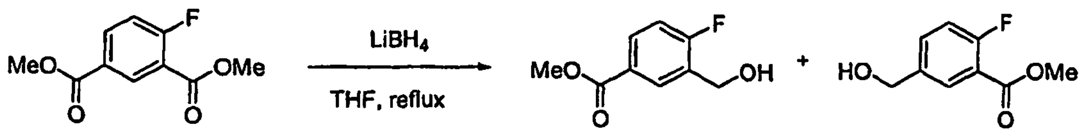


A compound prepared in the step 2 (104.5 mg, 0.493 mmol) was dissolved in tetrahydrofuran solution (4 ml). Then, 2M lithiumborohydride tetrahydrofuran solution (0.123 ml, 0.246 mmol) was slowly added therein. The reaction mixture was refluxed for 1 day. The reaction was quenched by water. Then, pH was adjusted to 5 with 1M HCl solution at 0°C. Extraction with ethyl acetate was performed. Combined organic layer was dried over anhydrous sodium sulfate, filtered, and concentrated under reduced pressure. The residue was purified by column chromatography (hexane:ethylacetate = 2:1), to give 4-fluoro-3-hydroxymethyl-benzoic acid methylester (45.4 mg, yield: 50.1%, colorless liquid) and 2-fluoro-5-hydroxymethyl-benzoic acid methylester (15.4 mg, yield: 17.0%, colorless liquid).

4-fluoro-3-hydroxymethyl-benzoic acid methylester:

$^1$H NMR (400MHz, $CDCl_3$) : δ2.73(t, *J*=5.1Hz, 1H), 3.90(s, 3H), 4.78(d, *J*=5.1Hz, 2H), 7.07(dd, *J*=9.2, 9.2Hz, 1H), 7.93-7.97(m, 1H), 8.14(dd, *J*=7.1, 2.2Hz, 1H)

2-fluoro-5-hydroxymethyl-benzoic acid methylester:

$^1$H NMR (400MHz, $CDCl_3$) : δ1.96(t, *J*=4.4Hz, 1H), 3.94(s, 3H), 4.70(d, *J*=4.4Hz, 2H), 7.13(dd, *J*=10.6, 8.4Hz, 1H), 7.52-7.56(m, 1H), 7.92(dd, *J*=7.0, 2.2Hz, 1H)

<Step 4> Preparation of 4-fluoro-3-hydroxymethyl-benzoic acid

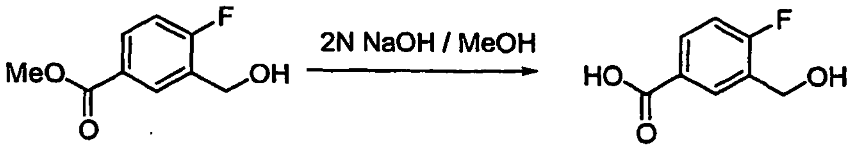


According to the similar procedure in the method of intermediate 3, by using a compound prepared in the step 3 (1.074 g, 5.380 mmol), 0.906 g (yield: 91.3%, white solid) of the target compound was obtained.

$^1H$ NMR (400MHz, $CD_3OD$) : δ4.69(s, 2H), 7.13(dd, *J*=9.9, 8.8Hz, 1H), 7.90-7.97(m, 1H), 8.16(dd, *J*=7.3, 2.2Hz, 1H)

<Intermediate 8> Preparation of 2-fluoro-5-hydroxymethyl-benzoic acid

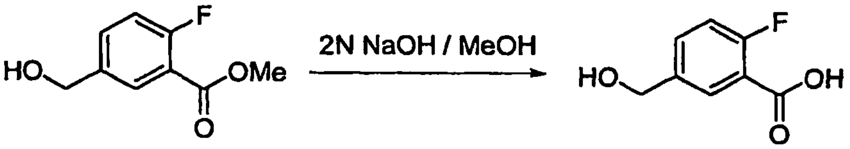


According to the similar procedure in the method of intermediate 3, by using 2-fluoro-5-hydroxymethyl-benzoic acid methylester prepared in the step 3 of intermediate 7, a target compound was obtained.

$^1H$ NMR (400 MHz, $CD_3OD$): δ4.61(s, 2H), 7.17(dd, *J*=11.0, 8.4Hz, 1H), 7.55-7.59(m, 1H), 7.93(dd, *J*=7.1, 2.4Hz, 1H)

<Intermediate 9> Preparation of 3-hydroxy-5-hydroxymethyl-benzoic acid

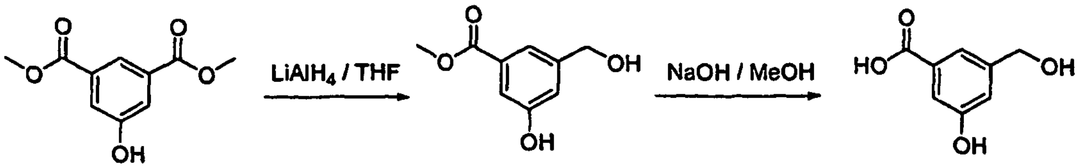


5-Hydroxy-isophthalic acid methylester (300 mg, 1.42 mmol) was dissolved in tetrahydrofuran (20 ml) at 0°C under a nitrogen atmosphere. Lithium aluminium hydride (30 mg, 0.7 mmol) was added therein. The reaction mixture was stirred at room temperature for 3 hours. Water was added to quench the reaction, and aqueous layer was extracted with ethyl acetate. Combined organic layer was dried over anhydrous sodium sulfate, filtered and concentrated under reduced pressure. The residue was purified by column chromatography (dichloromethane:methanol = 20:1). Above-obtained 3-hydroxy-5-hydroxymethyl-benzoic acid methylester (170 mg, 0.93 mmol) was dissolved in methanol (1 ml). 1N NaOH aqueous solution (1 ml) was added therein. The reaction mixture was stirred at room temperature for 1 hour. The reaction mixture was acidified (pH=3) with 2N HCl. Solvent was concentrated under reduced pressure, and then, dissolved in methanol and filtered. The filtrate was concentrated under reduced pressure to give 150 mg (yield: 96%, white solid) of the target compound.

$^1H$ NMR (400MHz, $CD_3OD$) : δ3.87(s, 3H), 4.57(s, 2H), 7.02(s, 1H), 7.31(s, 1H), 7.48 (s, 1H)

<Intermediate 10> Preparation of 3,5-*bis*-hydroxymethyl-benzoic acid

<Step 1> Preparation of 3,5-*bis*-hydroxymethyl-benzoic acid methylester

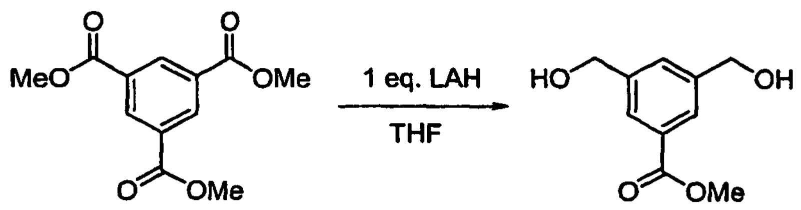


Benzene-1,3,5-tricarboxylic acid trimethylester (1.010 g, 4.003 mmol) was dissolved in tetrahydrofuran (15ml). Thereafter, the temperature was lowered into 0°C, and therein lithium aluminium hydride (0.160 g, 4.003 mmol) was slowly added, and the reaction mixture was stirred at room temperature for 3 hours. Water (0.15 ml) and 15% NaOH aqueous solution (0.15 ml) were slowly added to quench the reaction. Aqueous solution (0.45 ml) was added again. Solvent was concentrated under reduced pressure. The residue was purified by column chromatography (hexane:ethyl acetate = 1:2), to give 0.34 g (yield: 43.1%, colorless liquid) of the target compound.

$^1$H NMR (400MHz, $CD_3OD$) : δ3.91(s, 3H), 4.66(s, 4H), 7.59(s, 1H), 7.93(s, 2H)

<Step 2> Preparation of 3,5-*bis*-hydroxymethyl-benzoic acid

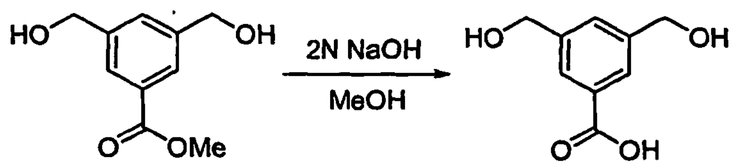


According to the similar procedure in the method of intermediate 3, by using a compound prepared in the step 1 (1.50 g, 7.65 mmol), 0.617 g (yield: 44.3%, white solid) of the target compound was obtained.

$^1$H NMR (400MHz, $CD_3OD$) : δ5.21(s, 4H), 7.55(s, 1H), 7.92(s, 2H)

<Intermediate 11> Preparation of 2-hydroxy-4-hydroxymethyl-benzoic acid

<Step 1> Preparation of 2-hydroxy-4-hydroxymethyl-benzoic acid methylester

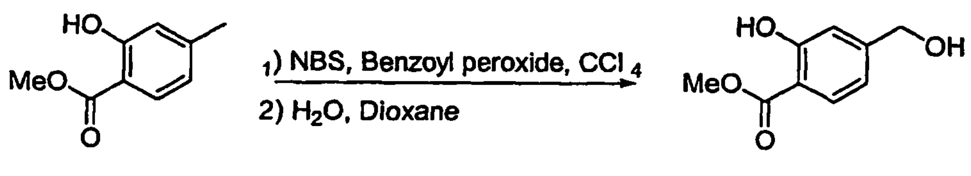


2-Hydroxy-4-methyl-benzoic acid methylester (166 mg, 1 mmol) was dissolved in $CCl_4$ (1.5ml). NBS (177 mg, 1 mmol) and benzoyl peroxide (5 mg, 0.02 mmol) were added therein. The reaction mixture was stirred at 70°C for 12 hours. Then, the reaction mixture was washed with water, dried over anhydrous sodium sulfate, and filtered. Solvent was concentrated under reduced pressure. The residue was purified by column chromatography (dichloromethane: hexane = 1:4), to give 4-bromomethyl-2-hydroxy-benzoic acid methylester (130 mg, yield: 53%, white solid). The resultant

compound (130 mg, 0.53 mmol) was dissolved in aqueous solution (1.5 ml) and 1,4-dioxane (1.5 ml), and the mixture was stirred at 90°C for 12 hours. The reaction mixture was extracted with chloroform. Combined organic layer was dried over anhydrous sodium sulfate, filtered and concentrated under reduced pressure. The residue was purified by column chromatography (ethyl acetate:hexane = 1:4), to give 55 mg (yield: 57%, white solid) of the target compound.

$^1H$ NMR (400MHz, $CDCl_3$) : δ3.95(s, 3H), 4.71(d, *J*=6Hz, 2H), 6.88(d, *J*=8Hz, 1H), 6.99(s, 1H), 7.82(d, *J*=8Hz, 1H), 10.79(s, 1H)

<Step 2> Preparation of 2-hydroxy-4-hydroxymethyl-benzoic acid

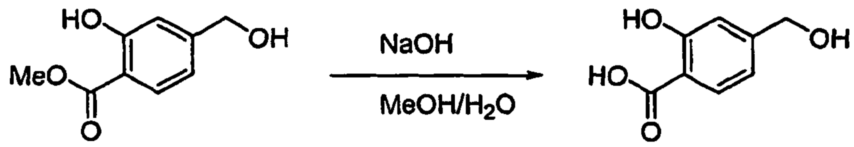


According to the similar procedure in the method of intermediate 3, by using a compound prepared in the step 1, 45 mg (yield: 90%, white solid) of the target compound was obtained.

<Intermediate 12> Preparation of 4-hydroxymethyl-thiophene-2-carboxylic acid

<Step 1> A: 4-methyl-thiophene-2-carboxylic acid,

B: Preparation of 3-methyl-thiophene-2-carboxylic acid

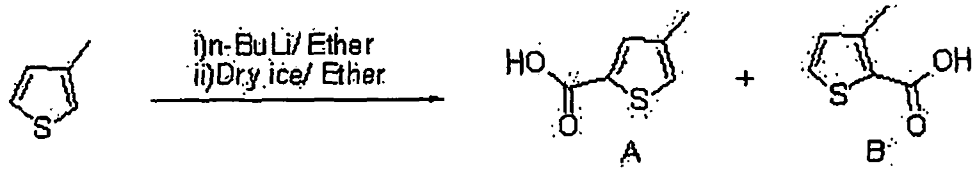


2.5M n-Butyllithium (12.2 ml, 30.55 mmol) was dissolved in diethyl ether (1 ml) at room temperature under a nitrogen atmosphere. 3-Methyl-thiophene (3 g, 30.55 mmol) dissolved in diethyl ether was slowly added therein. The mixture was refluxed for 2 hours. Reaction chamber was cooled to 0°C and dry-ice was slowly added therein. Reaction was quenched with 45 ml of water. Diethyl ether layer was extracted and removed. Water layer was acidified with 1N HCl solution and extracted with ethylacetate. Combined organic layer was dried over anhydrous sodium sulfate, filtered and concentrated under reduced pressure. The residue was purified by column chromatography (hexane:ethyl acetate = 9:1), to give the target compound A (900 mg, white solid) and B (650 mg, white solid).

<Step 2> Preparation of 4-bromomethyl-thiophene-2-carboxylic acid methyl ester

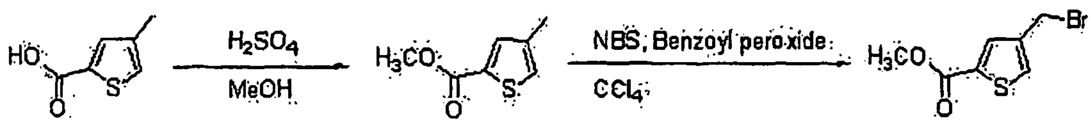


4-Methyl-thiophene-2-carboxylic acid (900 mg, 6.33 mmol) prepared in the step 1 was dissolved in methanol

(15 ml). Catalytic amount of sulfuric acid was slowly added therein. The reaction mixture was refluxed to give 4-methyl-thiophene-2-carboxylic acid methylester (890 mg, yield: 90%, white solid). 4-methyl-thiophene-2-carboxylic acid methyl ester (200 mg, 1.28 mmol) and NBS (215 mg, 1.216 mmol), and benzoyl peroxide of catalytic amount were dissolved in tetrachloromethane solution (5 ml). The reaction mixture was refluxed (70°C) for 3 hours to give 165 mg (yield: 55%, white solid) of the target compound.

$^1$H NMR (400MHz, $CDCl_3$) ; δ3.89(s, 3H), 4.46(s, 2H), 7.49(s, 1H), 7.80(s, 1H)

<step 3> Preparation of 4-hydroxymethyl-thiophene-2-carboxylic acid

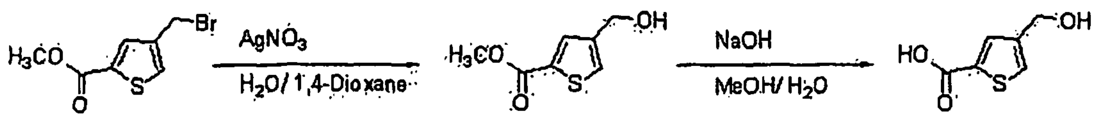


A compound (150 mg, 0.638 mmol) prepared in the step 2 was dissolved in 1,4-dioxane (1.5 ml) and water (1.5 ml). Silver nitrate (130 mg, 0.765 mmol) was slowly added therein. The reaction mixture was stirred at room temperature for 12 hours to give 4-hydroxymethyl-thiophene-2-carboxylic acid methyl ester (60 mg, yield: 55%, white solid). This compound (60 mg, 0.348 mmol) was dissolved in methanol (1 ml) at room temperature. 1N NaOH aqueous solution (1 ml) was slowly added therein, and the reaction mixture was stirred at room temperature for 1 hour, to give 50 mg (yield: 95%, white solid) of the target compound.

<Intermediate 13> Preparation of 3-hydroxymethyl-thiophene-2-carboxylic acid

<Step 1> Preparation of 3-bromomethyl-thiophene-2-carboxylic acid methyl ester

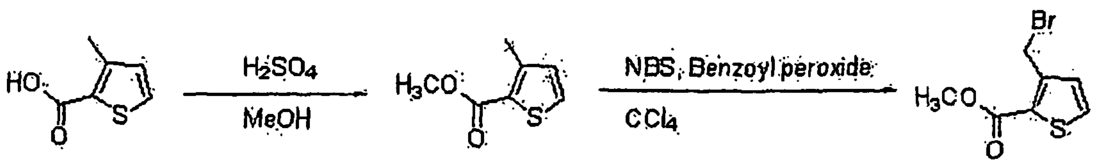


According to the similar procedure in the method of step 2 of intermediate 12, by using 3-methyl-thiophene-2-carboxylic acid (650 mg, 4.57 mmol) prepared in the step 1 of intermediate 12, 750 mg (yield: 79%, white solid) of the target compound was obtained.

$^1$H NMR (400MHz, $CDCl_3$) ; 63.90(s, 3H), 4.91(s, 2H), 7.18(d, J=5.2Hz, 1H), 7.46(d, J=5.2Hz, 1H)

<Step 2> Preparation of 3-hydroxymethyl-thiophene-2-carboxylic acid

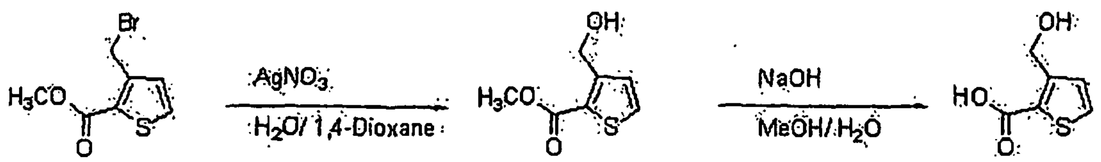


According to the similar procedure in the method of step 3 of intermediate 12, by using a compound prepared

in the step 1 (750 mg, 3.19 mmol), 210 mg (yield: 92%, white solid) of the target compound was obtained.

<Intermediate 14> Preparation of (3-hydroxymethylphenyl)-acetic acid

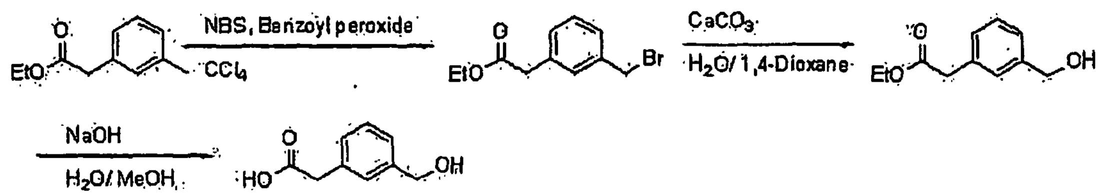


m-Tolyl acetic acid ethyl ester (1 g, 5.6 mmol), NBS (948 mg, 5.33 mmol) and benzoyl peroxide of catalytic amount were dissolved in tetrachloromethane (15 ml). The reaction mixture was refluxed (70°C) for 3 hours to give (3-bromomethyl-phenyl)-acetic acid ethyl ester (600 mg, yield: 42%, white solid). And (3-bromomethyl-phenyl)-acetic acid ethyl ester (130 mg, 0.50 mmol) and calcium carbonate (300 mg, 3 mmol) were dissolved in water (2 ml) and 1,4-dioxane (2 ml). The reaction mixture was refluxed to give (3-hydroxymethylphenyl)-acetic acid ethyl ester (85 mg, yield: 87%, white solid). (3-Hydroxymethyl-phenyl)-acetic acid ethyl ester (85 mg, 0.43 mmol) was dissolved in methanol (1 ml) at room temperature. 1N NaOH aqueous solution (1 ml) was added therein. The reaction mixture was stirred at room temperature for 1 hour, to give 65 mg (yield: 91%, white solid) of the target compound.

$^1H$ NMR(400MHz, $CDCl_3$) : δ3.66(s, 2H), 4.57 (s, 2H), 7.21-7.32(m, 4H)

<Intermediate 15> Preparation of 3-(2-hydroxy-ethyl)-benzoic acid

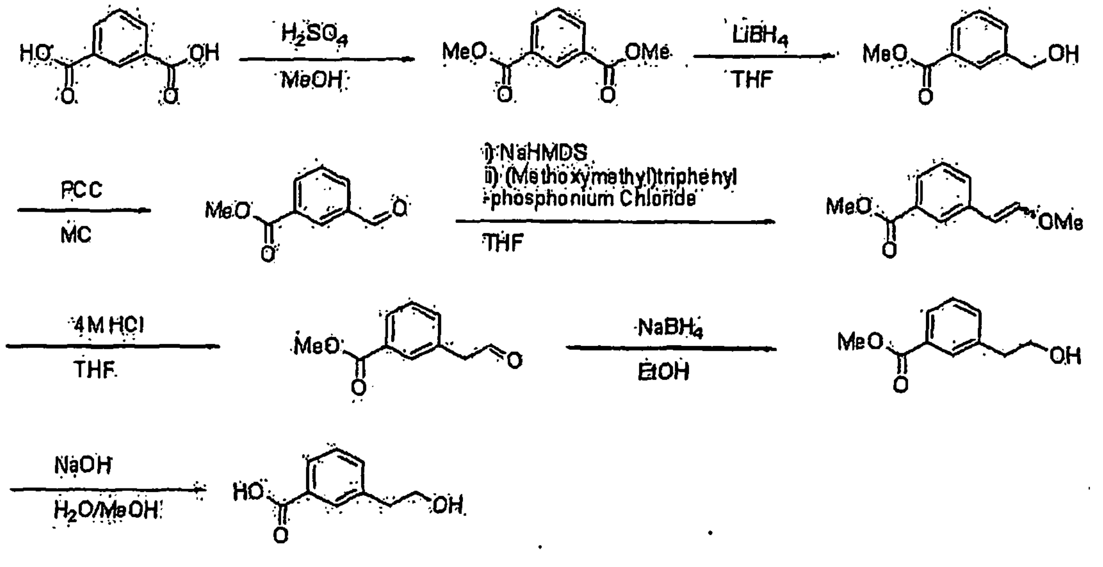


Isophthalic acid (5 g, 30 mmol) was dissolved in methanol (50 ml). Catalytic amount of sulfuric acid was added therein. The reaction mixture was stirred at reflux for 12 hours to give isophthalic acid dimethyl ester (5.2 g, yield: 90%, white solid). Isophthalic acid dimethyl ester (5.2 g, 26.7mmol) was dissolved in tetrahydrofuran (30 ml). 2M Lithiumborohydride tetrahydrofuran (13 ml, 26.7 mmol) was added therein. The reaction mixture was refluxed to give 3-hydroxymethyl-benzoic acid methyl ester (2.7 g, yield: 63%, colorless liquid), and 3-hydroxymethyl-benzoic acid methyl ester (200 mg, 1.20 mmol) was dissolved in dichloromethane (5 ml). PCC (388 mg, 1.8 mmol) was added therein. The reaction mixture was stirred at room temperature for 3 hours to give 3-formyl-benzoic acid methyl ester (140 mg, yield: 72%, white solid). (Methoxymethyl)triphenylphosphonium chloride (770 mg, 2.24 mmol) was dissolved in tetrahydrofuran (5ml) under a nitrogen atmosphere. 1M NaHMDS tetrahydrofuran (2 ml, 2.04 mmol) was slowly added therein at -78°C, and the reaction

mixture was stirred for 1 hour. 3-Formyl-benzoic acid methyl ester (160 mg, 0.975 mmol) dissolved in tetrahydrofuran (2 ml) was slowly added therein. The reaction mixture was stirred at room temperature for 24 hours to give 3-(2-methoxyvinyl)-benzoic acid methyl ester (121 mg, yield: 65%, white solid). 3-(2-Methoxyvinyl)-benzoic acid methyl ester (120 mg, 0.63 mmol) was dissolved in tetrahydrofuran (3 ml). 4M HCl (2 ml) was added therein, and the mixture was stirred at room temperature for 24 hours, to give 3-(2-oxo-ethyl)-benzoic acid methyl ester (60 mg, yield: 54%, white solid). 3-(2-Oxo-ethyl)-benzoic acid methyl ester (60 mg, 0.036 mmol) was dissolved in ethanol (2 ml). $NaBH_4$ (25 mg, 0.67 mmol) was added therein at 0°C, and the mixture was stirred at 1 hour, to give 3-(2-hydroxyethyl)-benzoic acid methyl ester (50 mg, yield: 84%, white solid). 3-(2-Hydroxyethyl)-benzoic acid methyl ester (50 mg, 0.28 mmol) was dissolved in methanol (1 ml). 1N NaOH aqueous solution (1 ml) was added therein. The reaction mixture was stirred at room temperature for 1 hour to give 43 mg (yield: 95%, white solid) of the target compound.

$^1H$ NMR (400MHz, $CDCl_3$) ; δ2.32(br, 1H), 2.89(t, *J*=6.4Hz, 2H), 3.85(t, *J*=6.4Hz, 2H), 7.36(t, *J*=7.6Hz, 1H), 7.42(d, *J*=5.6Hz, 1H), 7.88(d, *J*=8.8Hz, 1H), 7.89(s, 1H)

Example 6: 5-nitrooxymethyl-thiophene-2-carboxylic acid-[(7S)-1,2,3-trimethoxy-10-methylsulfanyl-9-oxo-5,6,7,9-tetrahydro-benzo[a]heptalen-7-yl]-amide

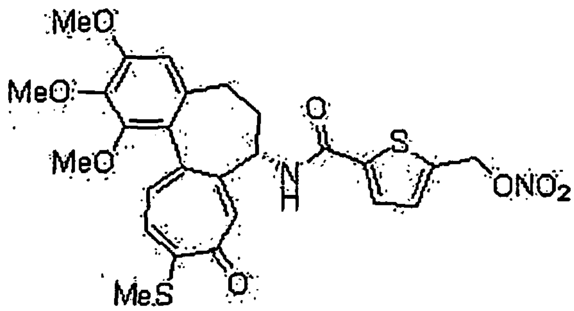

$^1H$ NMR(400MHz, $CDCl_3$); δ2.07-2.11(m, 1H), 2.32-2.45(m, 2H), 2.45(s, 3H), 2.53-2.56(m, 1H), 3.71(s, 3H), 3.91(s, 3H), 3.96(S, 3H), 4.84-4.91(m, 1H), 5.46(s, 2H), 6.56(s, 1H), 6.95(d, *J*=3.6Hz, 1H), 7.13(d, *J*=10.8Hz, 1H), 7.37 (d, *J*=10.8Hz, 1H), 7.58(d, *J*=4.0Hz, 1H), 7.61(s, 1H), 8.50(d, *J*=6.8Hz, 1H)

Reference Example 7: *N*-[(7*S*)-3-cyclopentyloxy-1,2-dimethoxy-10-methylsulfanyl-9-oxo-5,6,7,9-tetrahydro-benzo[a]heptalen-7-yl]-3-nitrooxymethyl-benzamide

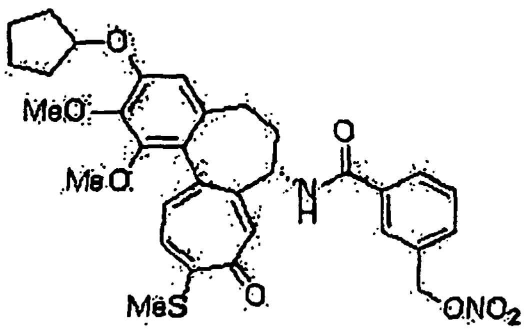

$^1H$ NMR(400MHz, $CDCl_3$); δ1.61-1.73(m, 2H), 1.85-2.00(m, 6H), 2.21-2.65(m, 4H), 2.44(s, 3H), 3.75(s, 3H), 3.93(s, 3H), 4.82-4.88(m, 1H), 4.92-5.00(m, 1H), 5.16(dd, *J*=12, 31.2Hz, 2H), 6.55(s, 1H), 7.13-7.17(m, 2H), 7.28(d, *J*=6.4Hz, 1H), 7.41(d, *J*=10.8Hz, 1H), 7.68-7.70(m, 1H), 7.72(s, 1H), 7.78(s, 1H), 8.78(d, *J*=7.2Hz, 1H)

Example 8: *N*-C(7S)-3-ethoa-1,2-dimethoxy-10-methylsulfanyl-9-oxo-5,6,7,9-tetrahydro-benzo[a]heptalen-7-yl]-2-fluoro-3-nitrooxymethyl-benzamide

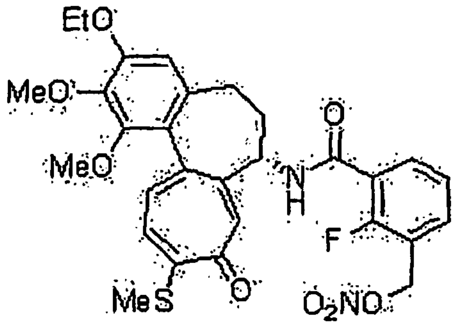

$^1$H NMR(400MHz, $CDCl_3$) ; δ1.49(t, *J*=6.9Hz, 3H), 1.93-1.98(m, 1H), 2.31-2.39(m, 1H), 2.43(s, 3H), 2.46-2.49 (m, 1H), 2.51-2.59 (m, 1H), 3.73(s, 3H), 3.96(s, 3H), 4.11-4.14(m, 2H), 4.83-4.86(m, 1H), 5.53(d, *J*=12.8Hz, 1H), 5.59 (d, *J*=12.4Hz, 1H), 6.56(s, 1H), 7.07(d, *J*=10.2Hz, 1H), 7.10-7.15(m, 1H), 7.20-7.28(m, 1H), 7.31(d, *J*=10.2Hz, 1H), 7.57 (t, *J*=6.4Hz, 1H), 7.97(t, *J*=6.9Hz, 1H)

Example 9: 2-fluoro-*N*-[(7S)-3-isopropoxy-1,2-dimethoxy-10-methylsulfanyl-9-oxo-5,6,7,9-tetrahydro-benzo[a]heptalen-7-yl]-3-nitrooxymethyl-benzamide

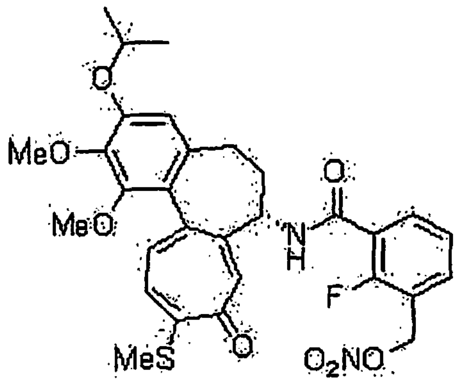

$^1$H NMR (400MHz, $CDCl_3$) ; δ1.41(q, *J*=6.0Hz, 6H), 1.92-1.99(m, 1H), 2.31-2.40(m, 1H), 2.43(s, 3H), 2.46-2.52 (m, 1H), 2.55-2.60(m, 1H), 3.72(s, 3H), 3.94(s, 3H), 4.57-4.63(m, 1H), 4.83-4.89(m, 1H), 5.53(d, *J*=12.8Hz, 1H), 5.59 (d, *J*=12.4Hz, 1H), 6.57(s, 1H), 7.07(d, *J*=10.6Hz, 1H), 7.15-7.19(m, 1H), 7.23-7.27(m, 1H), 7.34(d, *J*=10.2Hz, 1H), 7.56 (t, *J*=7.1Hz, 1H), 7.7(t, *J*=7.5Hz, 1H)

Example 10: 2-fluoro-3-nitrooxymethyl-*N*-[(7S)-1,2,3-trimethoxy-10-methylsulfanyl-9-oxo-5,6,7,9-tetrahydro-benzo[a]heptalen-7-yl]-benzamide

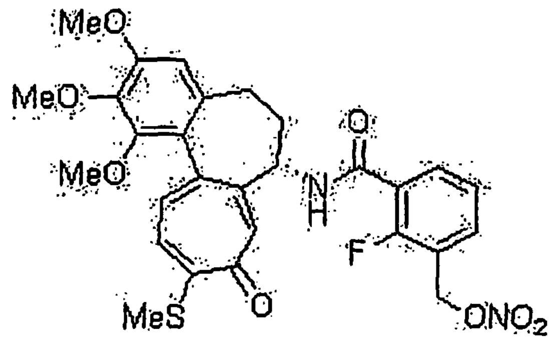

$^1H$ NMR (400MHz, $CDCl_3$) ; δ1.95-2.02 (m, 1H), 2.31-2.54(m, 2H), 2.43(s, 3H), 2.59-2.63(m, 1H), 3.73(s, 3H), 3.87(s, 3H), 3.92(s, 3H), 4.82-4.88(m, 1H), 5.51(d, *J*=12.8Hz, 1H), 5.60(d, *J*=12.8Hz, 1H), 6.58(s, 1H), 7.06(d, *J*=10.6Hz, 1H), 7.22-7.27(m, 2H), 7.28(s, 1H), 7.32(d, *J*=10.6Hz, 1H), 7.54-7.58 (m, 1H), 7.93-7.98(m, 1H)

Example 11: N-[(7S)-3-cyclopentyloxy-1,2-dimethoxy-10-methylsulfanyl-9-oxo-5,6,7,9-t-etrahydro-benzo[a]heptalen-7-yl]-2-fluoro-3-nitrooxymethyl-benzamide

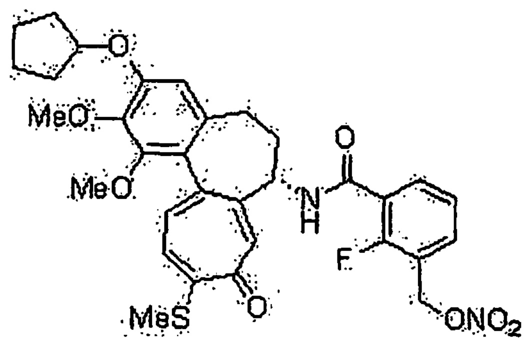

$^1H$ NMR (400MHz, $CDCl_3$) : δ1.61-1.73 (m, 2H), 1.85-2.00(m, 6H), 2.35-2.52(m, 4H), 2.42(s, 3H), 3.72(s, 3H), 3.93(s, 3H), 4.82-4.88(m, 2H), 5.57(dd, *J*=12.8, 37.6Hz, 2H), 6.55(s, 1H), 7.05(d, *J*=10.4Hz, 1H), 7.09-7.13(m, 1H), 7.26 (d, *J*=7.2Hz, 2H), 7.32(d, *J*=10.4Hz, 1H), 7.55-7.59(m, 1H), 7.96-8.00(m, 1H)

Example 12: 3-fluoro-5-nitrooxymethyl-*N*-[(7S)-1,2,3-trimethoxy-10-methylsulfanyl-9-oxo-5,6,7,9-tetrahydro-benzo[a]heptalen-7-yl]-benzamide

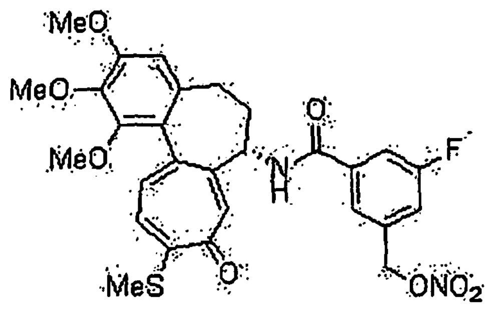

$^1H$ NMR(400MHz, $CDCl_3$): δ2.31-2.45(m, 3H), 2.46(s, 3H), 2.59-2.63(m, 1H), 3.75(s, 3H), 3.93(s, 3H), 3.98(S, 3H), 4.90-4.95(m, 1H), 5.11(dd, *J*=12.8, 45.6Hz, 2H), 6.57(s, 1H), 7.03(d, *J*=7.6Hz,1H), 7.19(d, *J*=8.8Hz, 1H), 7.30-7.33 (m, 1H), 7.42(d, *J*=10.8Hz, 1H), 7.55(s, 1H), 7.68(s, 1H), 8.76(d, *J*=7.2Hz, 1H)

Example 13: *N*-[(7S)-3-ethoxy-1,2-dimethoxy-10-methylsulfanyl-9-oxo-5,6,7,9-tetrahydro-benzo[a]heptalen-7-yl)-3-fluoro-5-nitrooxymethyl-benzamide

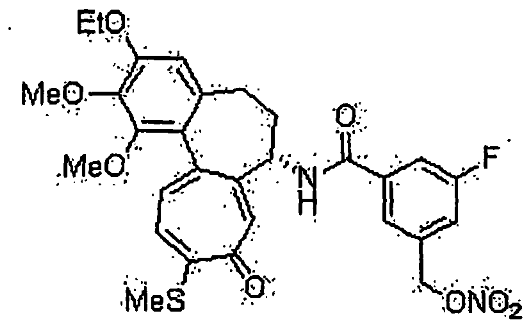

$^1H$ NMR (400MHz, $CDCl_3$) ; δ1.50(t, J=6.9, 3H), 2.33-2.42(m, 3H), 2.46(s, 3H), 2.51~2.59(m, 1H), 3.75(s, 3H), 3.98(s, 3H), 4.11-4.16(m, 2H), 4.90-4.93(m, 1H), 5.05(d, J=12.4, 1H), 5.17(d, J=12.4, 1H), 6.56(s, 1H), 7.03(d, J=7.3, 1H), 7.20(d, J=10.6, 1H), 7.31(d, J=9.1, 1H), 7.45(d, J=10.6, 1H), 7.55(s, 1H), 7.69(s, 1H), 8.88(d, J=7.3, 1H)

Example 14: 3-fluoro-N-[(7S)-3-isopropoxy-1,2-dimethoxy-10-methylsulfanyl-9-oxo-5,6,7,9-tetrahydro-benzo[a]heptalen-7-yl]-5-nitrooxymethyl-benzamide

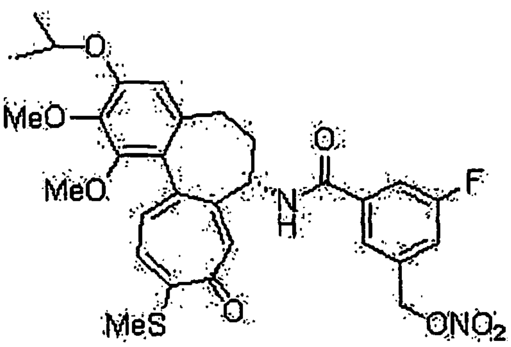

$^1H$ NMR(400MHz, $CDCl_3$) ; δ1-37-1.44 (m, 6H) , 2.34-2.41(m, 3H), 2.46(s, 3H), 2.51~2.58(m, 1H), 3.75(s, 3H), 3.96(s, 3H), 4.57-4.62(m, 1H), 4.91-4.95(m, 1H), 5.05(d, J=12.4, 1H), 5.17(d, J=12.4, 1H), 6.56(s, 1H), 7.03(d, J=8.4, 1H), 7.20(d, J=10.6, 1H), 7.31(d, J=7.3, 1H), 7.45(d, J=10.6, 1H), 7.55(s, 1H), 7.70(s, 1H), 8.91(d, J=7.3, 1H)

Example 15: *N*-[(7S)- 3-cyclopentyloxy- 1,2-dimethoxy- 10-methylsulfanyl- 9-oxo- 5,6,7,9-tetrahydro-benzo[a]heptalen-7-yl]-3-fluoro-5-nitrooxymethyl-benzamide

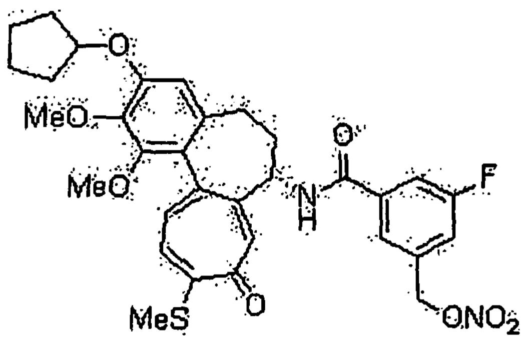

$^1H$ NMR (400MHz, $CDCl_3$) ; δ1.64~1.69(m, 2H) , 1.83~1.98(m, 6H), 2 .35~2.43(m, 3H), 2.46(s, 3H), 2.51~2.58 (m, 1H), 3.74(s, 3H), 3.94(s, 3H), 4.82~4.84(m, 1H), 4.91~4.94(m, 1H), 5.04(d, J=12.4, 1H), 5.16(d, J=12.4, 1H), 6.55 (s, 1H), 7.02(d, J=8.4, 1H), 7.20(d, J=10.6, 1H), 7.31(d, J=7.3, 1H), 7.45(d, J=10.2, 1H), 7.55(s, 1H), 7.70(s, 1H), 8.93 (d, J=6.9, 1H)

Example 16: 4-fluoro-3-nitrooxymethyl-*N*-[(7S)-1,2,3-trimethoxy-10-methylsulfanyl-9-oxo-5,6,7,9-tetrahydro-benzo[a]heptalen-7-yl]-benzamide

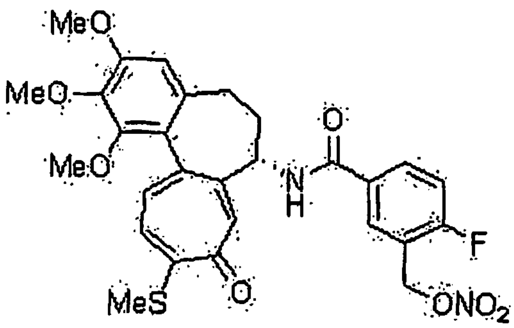

$^1H$ NMR(400MHz, $CDCl_3$); δ2.22-2.51(m, 3H) , 2.46(s, 3H), 2.55-2.63(m, 1H), 3.76(s, 3H), 3.92(s, 3H), 3.98 (s, 3H), 4.90-4.97(m, 1H), 5.10(d, *J*=12.1Hz, 1H), 5.35(d, *J*=12.1Hz, 1H), 6.57(s, 1H), 6.86(dd, *J*=9.2, 8.8Hz, 1H), 7.18 (d, *J*=10.3Hz, 1H), 7.43(d, *J*=10.3Hz, 1H), 7.72(s, 1H), 7.76-7.80(m, 1H), 7.91(dd, *J*=6.80, 2.2Hz, 1H), 8.93(d, *J*=7.2Hz, 1H)

Example 17: 2-fluoro-5-nitrooxymethyl-*N*-[(7S)-1,2,3-trimethoxy-10-methylsulfanyl-9-oxo-5,6,7,9-tetrahydro-benzo[a]heptalen-7-yl]-benzamide

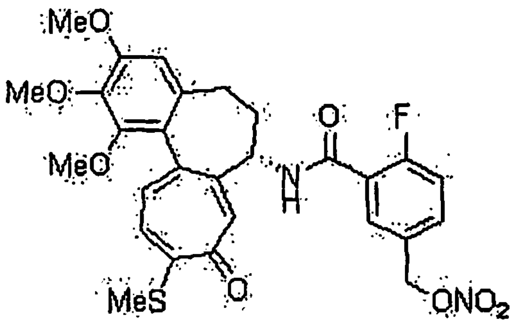

$^1$H NMR(400MHz, $CDCl_3$); δ1.94-2.01(m, 1H), 2.31-2.54(m, 2H), 2.43(s, 3H), 2.58-2.63(m, 1H), 3.73(s, 3H), 3.92(s, 3H), 3.97(s, 3H), 4.81-4.87(m, 1H), 5.36(s, 2H), 6.57(s, 1H), 7.06(d, *J*=10.3Hz, 1H), 7.16-7.28(m, 2H), 7.26(s, 1H), 7.33(d, *J*=10.3Hz, 1H), 7.51-7.55(m, 1H), 7.99(dd, J=7.3, 2.6Hz, 1H)

Example 18: 3-hydroxy-5-nitrooxymethyl-*N*-[(7S)-1,2,3-trimethoxy-10-methylsulfanyl-9-oxo-5,6,7,9-tetrahydro-benzo[a]heptalen-7-yl]-benzamide

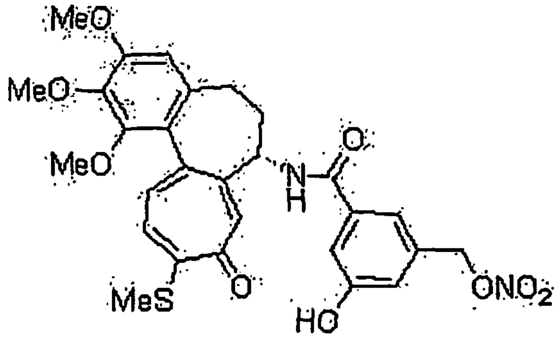

$^1$H NMR(400MHz, CDCl3); δ2.30-2.43(m, 6H), 2.56-2.57(m, 1H), 3.61(s, 3H), 3.90(s, 3H), 3.92(s, 3H), 4.82-4.85 (m, 1H), 5.00(dd, *J*=30.8Hz, 12.8Hz, 2H), 6.56(s, 1H), 6.62(s, 1H), 7.11(s, 2H), 7.16-7.19(m, 2H), 7.41(d, *J*=10.8Hz, 1H), 7.66(s, 1H), 8.57(brs, 1H, NH), 8.78(brs, 1H, OH)

Reference Example 19: 3,5-*bis*-nitrooxymethyl-*N*-[(75)-1,2,3-trimethoxy-10-methylsulfanyl-9-oxo-5,6,7,9-tetrahydro-benzo[a]heptalen-7-yl]-benzamide

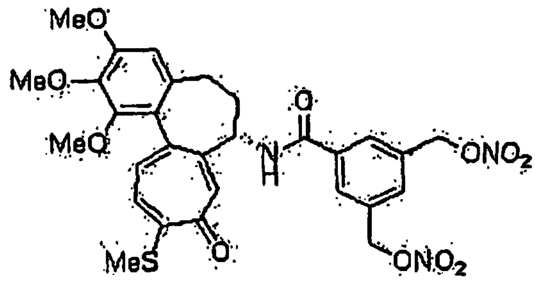

$^1$H NMR(400MHz, $CDCl_3$); δ2.29-2.51(m, 3H), 2.46(s, 3H), 2.60-2.62(m, 1H), 3.77(s, 3H), 3.93(s, 3H), 3.99(s, 3H), 4.94-4.98(m, 1H), 5.18(s, 4H), 6.58(s, 1H), 7.20(d, *J*=10.6Hz, 1H), 7.26(s, 1H), 7.45 (d, *J*=10.6Hz, 1H), 7.77(s, 2H), 7.81(s, 1H), 9.12(d, *J*=7.0Hz, 1H)

Example 20: 2-hydroxy-4-nitrooxymethyl-*N*-[(7S)-1,2,3-trimethoxy-10-methylsulfanyl-9-oxo-5,6,7,9-tetrahydro-benzo[a]heptalen-7-yl]-benzamide

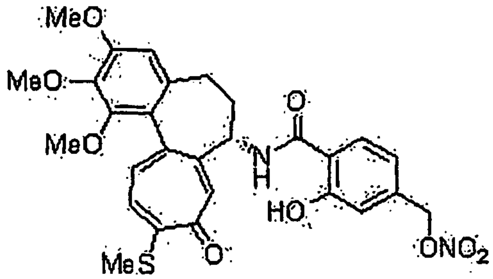

$^1H$ NMR(400MHz, $CDCl_3$); δ2.25-2.63(m, 4H), 2.47(s, 3H), 3.74(s, 3H), 3.91(s, 3H), 3.97(S, 3H), 4.85-4.92(m, 1H), 5.27(d, *J*=4.8Hz, 2H), 6.53(d, *J*=8.4Hz, 1H), 6.57(s, 1H), 6.61(s, 1H), 7.20(d, *J*=10.4Hz, 1H), 7.42(d, *J*=10Hz, 1H), 7.63(s, 1H), 7.72(d, *J*=8Hz, 1H), 9.13(d, *J*=6.8Hz, 1H), 12.29(s, 1H)

Example 21: 4-nitrooxymethyl-thiophene-2-carboxylic acid [(7S)-1,2,3-trimethoxy-10-methylsulfanyl-9-oxo-5,6,7,9-tetrahydro-benzo[a]heptalen-7-yl]-amide

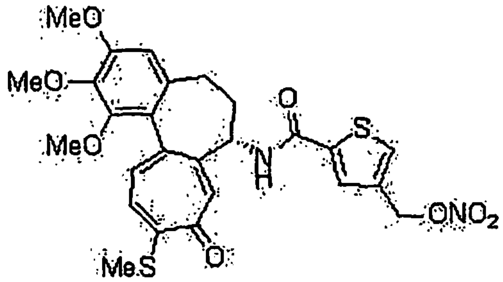

$^1H$ NMR(400MHz, $CDCl_3$): δ2.21-2.29(m, 1H), 2.34-2.46(m, 5H), 2.51-2.58(m, 1H), 3.71(s, 3H), 3.91(s, 3H), 3.96(S, 3H), 4.88-4.95(m, 1H), 5.18(dd, *J*=12.8, 20Hz, 2H), 6.56(s, 1H), 7.17(d, *J*=10.4Hz, 1H), 7.30(s, 1H), 7.41(d, *J*=10.4Hz, 1H), 7.61(s, 1H), 7.74(s, 1H), 8.81(d, *J*=6.4Hz, 1H)

Example 22: 3-nitrooxymethyl-thiophene-2-carboxylic acid [(7S)-1,2,3-trimethoxy-10-methylsulfanyl-9-oxo-5,6,7,9-tetrahydro-benzo[a]heptalen-7-yl]-amide

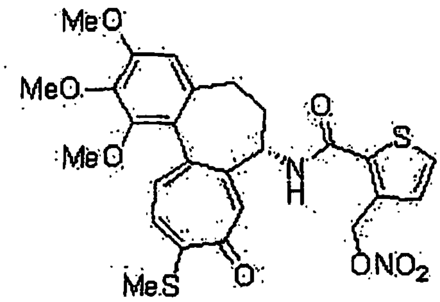

$^1H$ NMR(400MHz, $CDCl_3$); δ2.04-2.17(m, 1H), 2.30-2.45(m, 5H), 2.57-2.64(m, 1H), 3.73(s, 3H), 3.91(s, 3H), 3.96(S, 3H), 4.67-4.88(m, 1H), 5.66(d, *J*=13.6Hz, 1H), 5.80(d, *J*=13.6Hz, 1H), 6.56(s, 1H), 7.02d, *J*=4.4Hz, 1H), 7.08 (d, *J*=10.4Hz, 1H), 7.26(d, *J*=4.4Hz, 1H), 7.29(d, *J*=10.4Hz, 1H), 7.43 (s, 1H), 7. 45 (d, *J*=7.2Hz, 1H)

Reference Example 23 : 2-(3-nitrooxymethyl-phenyl)-N-[(7S)-1,2,3-trimethoxy-10-methylsulfanyl-9-oxo-5,6,7,9-tetrahydro-benzo[a]heptalen-7-yl)-acetamide

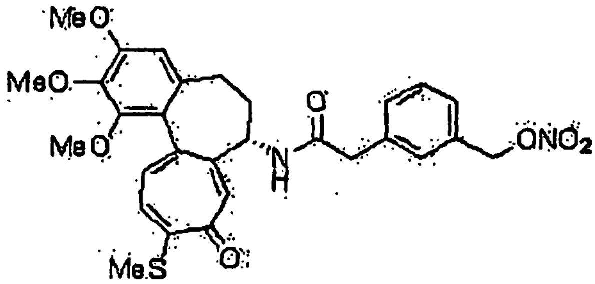

$^1H$ NMR(400MHz, $CDCl_3$); δ1.82-1.95(m, 1H), 2.21-2.28(m, 1H), 2.31-2.42(m, 1H), 2.45(s, 3H), 2.47-2.53(m, 1H), 3.51(d, *J*=14.0Hz, 1H), 3.64(s, 3H), 3.66(d, *J*=14.0Hz, 1H), 3.88(s, 3H), 3.93(s, 3H), 4.66-4.72(m, 1H), 5.40(s, 2H), 6.51(s, 1H), 7.11(d, *J*=10.4Hz, 1H), 7.24-7.38(m, 5H), 7.48(s, 1H), 7.90(d, *J*=7.2Hz, 1H)

Reference Example 24: 3-(2-nitrooxy-ethyl)-N-[(7S)-1,2,3-trimethoxy-10-methylsulfanyl-9-oxo-5,6,7,9-tetrahydro-benzo[a]heptalen-7-yl]-benzamide

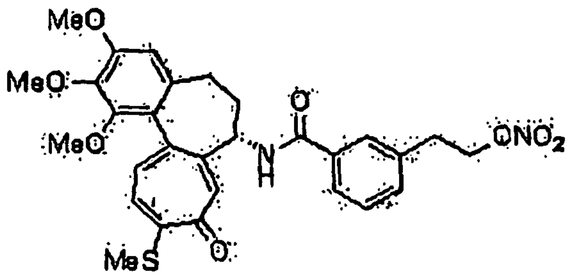

$^1H$ NMR(400MHz, $CDCl_3$) δ2.05-2.11(m, 1H), 2.17-2.56(m, 2H), 2.46(s, 3H), 2.56-2.60(m, 1H), 2.81-2.91(m, 2H), 3.69(s, 3H), 3.91(s, 3H), 3.97(s, 3H), 4.45(t, *J*=6.8,Hz, 2H), 4.89-4.95(m, 1H), 6.56(s, 1H), 7.12-7.24(m, 3H), 7.39 (d, *J*=10.4Hz, 1H), 7.61-7.64(m, 2H), 7.69(s, 1H), 8.22(d, *J*=6.8Hz, 1H)

Example 25: Preparation of 3-nitrooxy-benzoic acid-5-[(7S)-1,2,3-trimethoxy-10-methylsulfanyl-9-oxo-5,6,7,9-tetrahydro-benzo[a]heptalen-7-yl-carbamoyl]-pyridine-2-yl-methylester

<Step 1> Preparation of 3-chloromethyl-benzoic acid-{5-[(7S)-1,2,3-trimethoxy-10-methylsulfanyl-9-oxo-5,6,7,9-tetrahydro-benzo[a]heptalen-7-yl-carbamonyl]-pyridine-2-yl}-methylester

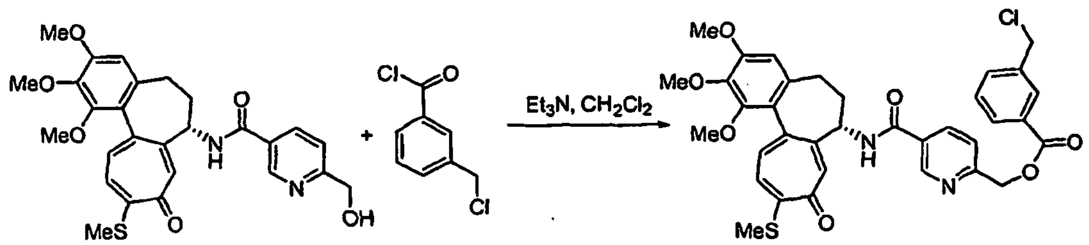


A compound prepared in step 1 of the Example 1 (100 mg, 0.19 mmol) was dissolved in 3 mℓ of dichloromethane. 3-(Chloromethyl) benzoylchloride (0.030 ml, 0.21 mmol) and triethylamine (0.082 ml, 0.59 mmol) were slowly added therein, and the mixture was reacted at room temperature for 10 minutes. Water was added to quench the reaction, and aqueous layer was extracted with dichloromethane. Combined organic layer was dried over anhydrous sodium sulfate, filtered and concentrated under reduced pressure. The residue was purified by column chromatography (ethyl acetate), to give 99 mg (yield: 79%, yellow solid) of the target compound.

$^1$H NMR (400MHz, $CDCl_3$): δ2.14-2.16(m, 1H), 2.30-2.39(m, 1H), 2.43-2.45(m, 1H), 2.48(s, 3H), 2.68-2.96(m, 1H), 3.67(s, 3H), 3.90(s, 3H), 3.91(s, 3H), 4.83-4.93(m, 1H), 4.88(s, 2H), 5.51(s, 2H), 6.77(s, 1H), 7.27(s, 1H), 7.52(t, J=7.7Hz, 1H), 7.65-7.70(m, 2H), 8.05(d, J=7.7Hz, 1H), 8.14(s, 1H), 8.28(d, J=10.6Hz, 1H), 9.02(s, 1H)

<Step 2> Preparation of 3-nitrooxy-benzoic acid-{5-[(7S)-1,2,3-trimethoxy-10-methylsulfanyl-9-oxo-5,6,7,9-tetrahydro-benzo[a]heptalen-7-yl-carbamoyl]-pyridine-2-yl}-methylester

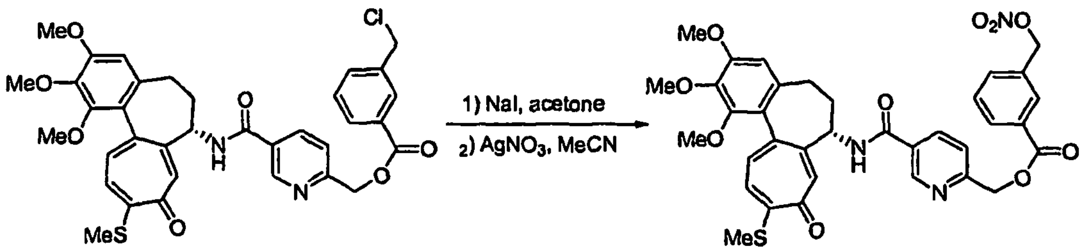


A compound prepared in the step 1 (90 mg, 0.13 mmol) and sodium iodide (31 mg, 0.20 mmol) were dissolved in 3 ml of acetone, and the mixture was reacted at room temperature for 1 day. Water was added to quench the reaction, and aqueous layer was extracted with ethyl acetate. Solvent was concentrated under reduced pressure. The reaction concentrate and silver nitrate (30mg, 0.045 mmol) were dissolved in 5 ml of acetonitrile, and the mixture was reacted at room temperature for 1 hour. Water was added to quench the reaction, and aqueous layer was extracted with ethyl acetate. Solvent was concentrated under reduced pressure. The residue was purified by short column chromatography (ethyl acetate) and PLC, to give 26 mg (yield: 29%, yellow solid) of the target compound.

$^1$H NMR (400MHz, $CDCl_3$): δ2.09-2.17(m, 1H), 2.35-2.41(m, 2H), 2.44(s, 3H), 2.55-2.58(m, 1H), 3.74(s, 3H), 3.91(s, 3H), 3.96(s, 3H), 4.92-4.95(m, 1H), 5.45(s, 2H), 5.47(s, 2H), 6.56(s, 1H), 7.13(d, J=10.2Hz, 1H), 7.35(t, J=9.1Hz, 2H), 7.50(t, J=8.5Hz, 1H), 7.54(s, 1H), 7.62(d, J=7.3Hz, 1H), 8.14(d, J=8.3Hz, 2H), 8.24(dd, J=2.2, 6.2Hz, 1H, ArH), 8.36(d, J=6.9Hz, 1H), 9.09(s, 1H)

Example 26~34

Compounds of Example 26 - Example 34 were synthesized in analogy to the procedure as described in Example 25, and intermediates were prepared by the method described as follows.

<Intermediate 16> Preparation of 2-hydroxy-*N*-[(7S)-1,2,3-trimethoxy-10-methylsulfanyl-9-oxo-5,6,7,9-tetrahydro-benzo[a]heptalen-7-yl]-benzamide

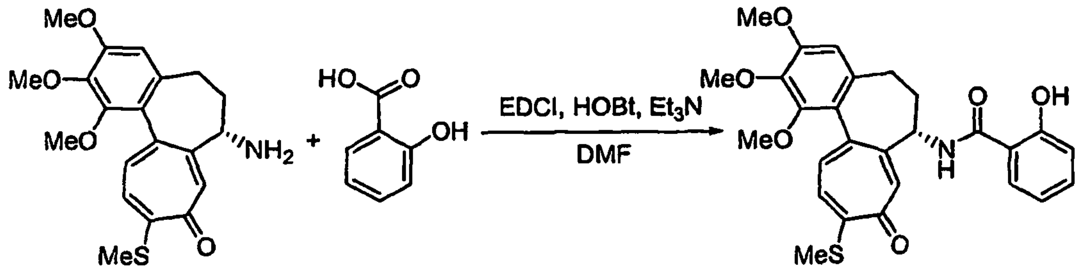


2-Hydroxy-benzoic acid (203 mg, 1.47 mmol), EDCI (385 mg, 2.01 mmol) and HOBt (271 mg, 2.01 mmol) were dissolved in dimethylformamide (10 ml). Triethylamine (0.37 ml, 2.67 mmol) was added therein, and the mixture was stirred at room temperature for 1 day. 7-Amino-1,2,3-trimethoxy-10-methylsulfonyl-6,7-dihydro-5H-benzo[a]-heptalen-9-one (500 mg, 1.34 mmol) was added therein, and the mixture was stirred at room temperature for 1 day. Water was added to quench the reaction, and aqueous layer was extracted with diethyl ether. Combined organic layer was dried over anhydrous sodium sulfate, filtered and concentrated under reduced pressure. The residue was purified by column chromatography (hexane:ethyl acetate = 1:5) and recrystallized in methanol, to give 516 mg (yield: 78%, yellow solid) of the target compound.

$^1H$ NMR (400MHz, $CDCl_3$): δ2.14-2.17(m, 1H), 2.31-2.37(m, 1H), 2.40-2.44(s, 1H), 2.45(s, 3H), 2.56-2.60(m, 1H), 3.74(s, 3H), 3.91(s, 3H), 3.97(s, 3H), 4.85-4.92(m, 1H), 6.56(s, 1H), 6.64(d, J=7.7Hz, 1H), 6.77(d, J=8.4Hz, 1H), 7.15(d, J=10.6Hz, 1H), 7.22(d, J=7.3Hz, 1H), 7.38(d, J=10.6Hz, 1H), 7.57(s, 1H), 7.71(d, J=8.0Hz, 1H)

<Intermediate 17> Preparation of 3-hydroxy-*N*-[(7S)-1,2,3-trimethoxy-10-methylsulfanyl-9-oxo-5,6,7,9-tetrahydro-benzo[a]heptalen-7-yl]-benzamide

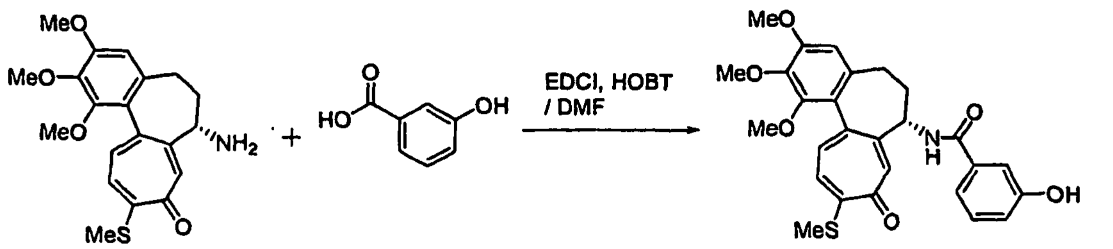


According to the similar procedure in the preparation method of intermediate 16, by using 3-hydroxybenzoicacid (497 mg, 1.33 mmol), 558 mg (yield: 85%, yellow solid) of the target compound was obtained.

$^1H$ NMR (400MHz, DMSO-$d_6$): δ2.07-2.15(m, 1H), 2.18-2.35(m, 6H), 3.55(s, 3H), 3.80(s, 3H), 3.84(s, 3H), 4.53-4.56(m, 1H), 6.81(s, 1H), 6.92(d, *J*=6.8Hz, 1H), 7.10(s, 1H), 7.15-7.31(m, 5H), 8.98(d, *J*=7.6Hz, 1H, - NH), 9.70(s, 1H, -OH)

Example 26: 4-nitrooxybutyric acid-5-[(7S)-1,2,3-trimethoxy-10-methylsulfanyl-9-oxo-5,6,7,9-tetrahydro-benzo[a]heptalen-7-yl-carbamoyl]-pyridine-2-yl-methylester

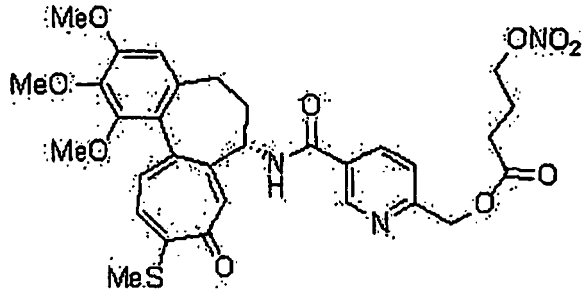

$^1H$ NMR (400MHz, $CDCl_3$): δ2.06-2.16(m, 1H), 2.08-2.11(m, 2H), 2.31-2.49(m, 1H), 2.45(s, 3H), 2.57(t, J=7.1Hz, 2H), 3.75(s, 3H), 3.91(s, 3H), 3.96(s, 3H), 4.52 (t, J=6.4Hz, 2H), 4.91-4.94(m, 1H), 5.21(s, 2H), 6.56(s, 1H), 7.13(d, J=10.9Hz, 1H), 7.21-7.25(m, 1H), 7.38 (d, J=10.6Hz, 1H), 7.52(s, 1H), 8.22 (d, J=8.0Hz, 1H), 9.05(s, 1H)

Example 27: 3-nitrooxymethyl-benzoicacid-6-[(7S)-1,2,3-trimethoxy-10-methylsulfanyl-9-oxo-5,6,7,9-tetrahydro-benzo[a]heptalen-7-yl-carbamoyl]-pyridine-2-yl-methylester

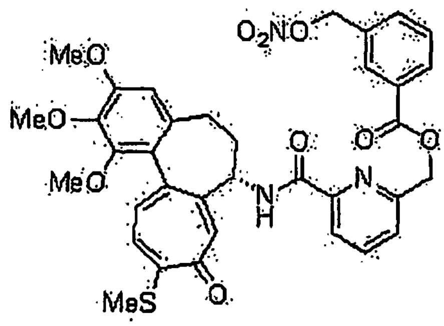

$^1H$ NMR (400MHz, $CDCl_3$): δ1.84-1.92(m, 1H), 2.33-2.60(m, 1H), 2.43(s, 3H), 2.46-2.54(m, 1H) 2.55-2.60(m, 1H), 3.73(s, 3H), 3.92(s, 3H), 3.96(S, 3H), 4.74-4.81(m, 1H), 5.51(s, 2H) 5.57(d, *J*=4.0Hz, 1H), 6.56(s, 1H), 7.04(d, *J*=10.4Hz, 1H), 7.25(d, *J*=10.4Hz, 1H), 7.54-7.60(m, 2H), 7.65(d, *J*=7.6Hz, 1H), 7.87(t, *J*=8.0Hz, 1H), 7.99(d, *J*=7.6Hz, 1H), 8.21(s, 2H), 8.22(s, 1H), 8.41(d, *J*=7.2Hz, 1H)

Example 28: 4-nitrooxybutyric acid-6-[(7S)-1,2,3-trimethoxy-10-methylsulfanyl-9-oxo-5,6,7,9-tetrahydro-benzo[a]heptalen-7-yl-carbamoyl]-pyridine-2-yl-methylester

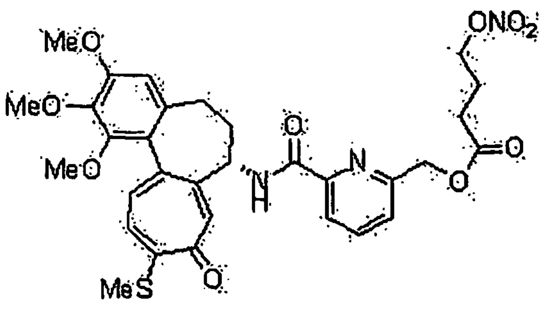

$^1H$ NMR (400MHz, $CDCl_3$): δ2.02-2.09(m, 1H), 2.11-2.18(m, 2H), 2.21-2.40(m, 2H), 2.43(s, 3H), 2.47-2.56(m, 1H), 2.63(t, *J*=7.2Hz, 2H), 3.73(s, 3H), 3.92(s, 3H), 3.96(S, 3H), 4.57(t, *J*=6.4Hz, 2H), 4.77-4.83(m, 1H), 5.30(d, *J*=4.8Hz, 2H), 6.58(s, 1H), 7.05(d, *J*=10.4Hz, 1H), 7.25(S, 1H), 7.32(d, *J*=10.4Hz, 1H), 7.52(d, *J*=8.0Hz, 1H), 7.84(t, *J*=8.0Hz, 1H), 7.98(d, *J*=8.0Hz, 1H), 8.45(d, *J*=6.8Hz, 1H)

Example 29: 3-nitrooxymethyl-benzoic acid-2-[(7S)-1,2,3-trimethoxy-10-methylsulfanyl-9-oxo-5,6,7,9-tetrahydro-benzo[a]heptalen-7-yl-carbamoyl]-phenylester

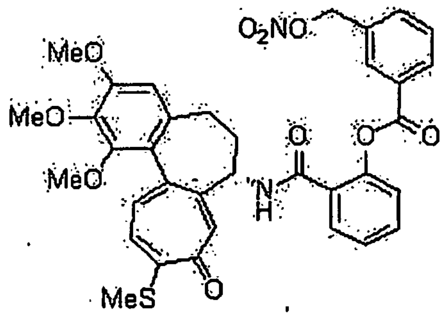

$^1H$ NMR (400MHz, $CDCl_3$): δ1.48-1.55(m, 1H), 1.83-1.89(m, 1H), 2.22-2.39(m, 2H), 2.42(s, 3H), 3.62(s, 3H), 3.87(s, 3H), 3.92(s, 3H), 4.63-4.70(m, 1H), 5.54(s, 2H), 6.47(s, 1H), 6.89(d, J=7.4Hz, 1H), 7.03(d, J=10.2Hz, 1H), 7.17 (s, 1H), 7.23(d, J=10.1Hz, 1H), 7.35(t, J=7.3Hz, 1H), 7.54(t, J=7.4Hz, 1H), 7.58(t, J=7.6Hz, 1H), 7.72(d, J=8.0Hz, 1H), 7.83(d, J=7.7Hz, 1H), 8.26(m, 2H)

Example 30: 4-nitrooxybutyric acid-2-[(7S)-1,2,3-trimethoxy-10-methylsulfanyl-9-oxo-5,6,7,9-tetrahydro-benzo[a]heptalen-7-yl-carbamoyl]-phenylester

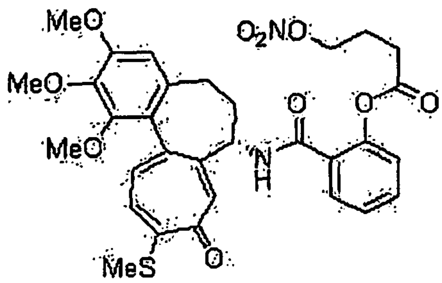

$^1H$ NMR (400MHz, $CDCl_3$): δ1.60-1.90(m, 1H), 2.10-2.14(m, 2H), 2.26-2.39(m, 1H), 2.44(s, 3H), 2.46-2.51(m, 1H), 2.56-2.61(m, 1H), 2.80(t, J=7.1Hz, 2H), 3.70(s, 3H), 3.92(s, 3H), 3.96(s, 3H), 4.55(t, J=6.2Hz, 2H), 4.74-4.81(m, 1H), 6.57(s, 1H), 6.82(d, J=6.9Hz, 1H), 7.08(d, J=10.6Hz, 1H), 7.13(d, J=9.1Hz, 1H), 7.23(s, 1H), 7.28-7.36(m, 1H), 7.47(t, J=9.9Hz, 1H), 7.68(d, J=9.5Hz, 1H)

Example 31: 3-nitrooxymethyl-benzoic acid-3-[(7S)-1,2,3-trimethoxy-10-methylsulfanyl-9-oxo-5,6,7,9-tetrahydro-benzo[a]heptalen-7-yl-carbamoyl]-phenylester

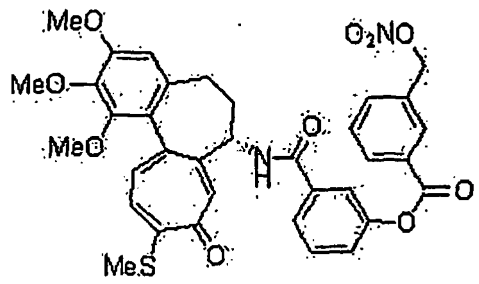

$^1H$ NMR (400MHz, $CDCl_3$): δ2.05-2.15(m, 1H), 2.23-2.59(m, 6H), 3.75(s, 3H), 3.91(s, 3H), 3.97(s, 3H), 4.90-5.00 (m, 1H), 5.49(s, 2H), 6.56(s, 1H), 7.03(d, *J*=10.4Hz, 1H), 7.27(s, 2H), 7.34(d, *J*=8.8Hz, 2H) 7.53-7.65(m, 2H), 7.76(s, 1H), 7.77(d, *J*=6.4Hz, 1H), 8.13(m, 3H)

Example 32: 4-nitrooxybutyric acid-3-[(7S)-1,2,3-trimethoxy-10-methylsulfanyl-9-oxo-5,6,7,9-tetrahydro-benzo[a]heptalen-7-yl-carbamoyl]-phenylester

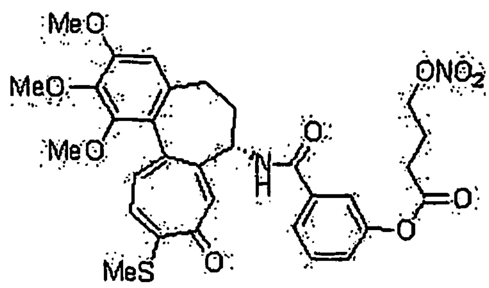

$^1H$ NMR (400MHz, $CDCl_3$): δ2.14-2.18(m, 3H), 2.25-2.60(m, 6H), 2.65(t, *J*=6.8Hz, 2H), 3.74(s, 3H), 3.91(s, 3H), 3.96(s, 3H), 4.56(t, *J*=6.4Hz, 2H), 4.87-4.94(m, 1H), 6.56(s, 1H), 7.08-7.12(m, 2H), 7.26-7.35(m, 2H), 7.48(s, 1H), 7.59(s, 1H), 7.71(d, *J*=8.0Hz, 1H), 7.75 (d, *J*=7.6Hz, 1H, -NH)

Example 33: 3-nitrooxymethyl-benzoic acid-3-[(7S)-1,2,3-trimethoxy-10-methyl-sulfanyl-9-oxo-5,6,7,9-tetrahydro-benzo[a]heptalen-7-yl-carbamoyl]-benzylester

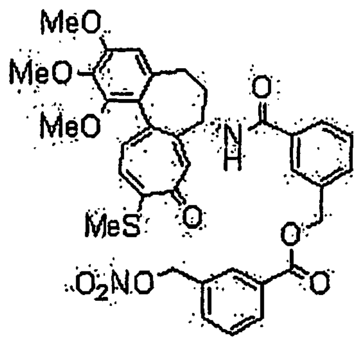

$^1H$ NMR (400MHz, $CDCl_3$): δ2.05-2.18(m, 1H), 2.31-2.47(m, 2H), 2.40(s, 3H), 2.55-2.59(m, 1H), 3.75(s, 3H),

3.91(s, 3H), 3.97(s, 3H), 4.90-4.96(m, 1H), 5.24(s, 2H), 5.44(s, 2H), 6.56(s, 1H), 7.09(d, *J*=10.6Hz, 1H), 7.27(dd, *J*=7.6, 7.6Hz, 1H), 7.36(d, *J*=10.6Hz, 1H), 7.44(d, *J*=7.6Hz, 1H), 7.45(dd, *J*=8.0, 7.6Hz, 1H), 7.58(d, *J*=8.0Hz, 1H), 7.59(s, 1H), 7.79(d, *J*=7.6Hz, 1H), 7.91(s, 1H), 8.05(d, *J*=7.6Hz, 1H), 8.06(s, 1H), 8.18(d, *J*=7.2Hz, 1H)

Example 34: 4-nitrooxybutyric acid-3-[(7S)-1,2,3-trimethoxy-10-methylsulfanyl-9-oxo-5,6,7,9-tetrahydro-benzo[a]heptalen-7-yl-carbamoyl]-benzylester

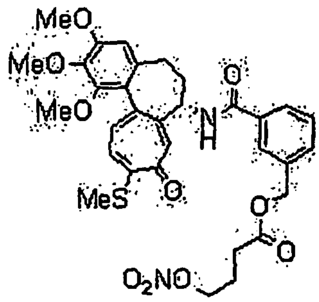

$^1$H NMR (400MHz, $CDCl_3$): δ2.00-2.14(m, 3H), 2.31-2.51(m, 4H), 2.44(s, 3H), 2.56-2.60(m, 1H), 3.75(s, 3H), 3.92(s, 3H), 3.97(s, 3H), 4.49(t, *J*=6.2Hz, 2H), 4.89-4.95(m, 1H), 4.99(d, *J*=12.4Hz, 1H), 5.04(d, *J*=12.4Hz, 1H), 6.56(s, 1H), 7.11(d, *J*=10.6Hz, 1H), 7.26(dd, *J*=7.6, 7.6Hz, 1H), 7.35-7.37(m, 2H), 7.54(s, 1H), 7.76(d, *J*=8.0Hz, 1H), 7.81(s, 1H), 8.00(d, *J*=7.6Hz, 1H)

Reference Example 35: Preparation of 2-nitrosothio-N-[(7S)-1,2,3-trimethoxy-10-methylsulfanyl-9-oxo-5,6,7,9-tetrahydro-benzo[a]heptalen-7-yl]-benzamide

<Step 1> Preparation of 2-mercapto-*N*-[(7S)-1,2,3-trimethoxy-10-methylsulfanyl-9-oxo-5,6,7,9-tetrahydro-benzo[a]heptalen-7-yl]-benzamide

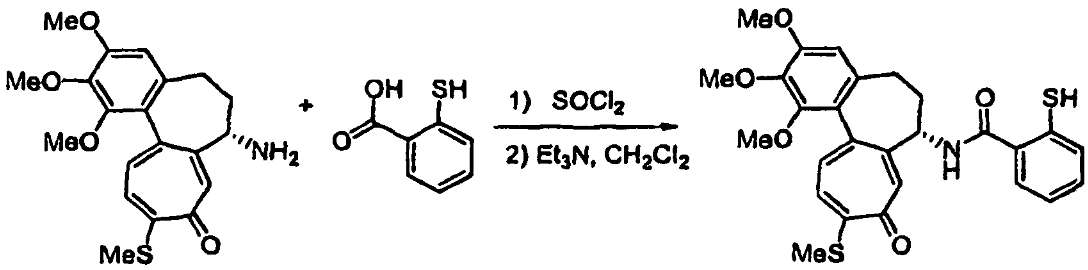


Excess thionylchloride was added in thiosalycilic acid (115 mg, 0.75 mmol), and the mixture was stirred with heating for 1 day. The reaction mixture was concentrated under reduced pressure to remove thionylchloride, and to give a chloride compound. 7-amino-1,2,3-trimethoxy-10-methylsulfonyl-6,7-dihydro-5*H*-benzo[a]-heptalen-9-one (243 mg, 0.62 mmol) was dissolved in purified dichloromethane. Triethylamine (0.26 ml, 1.86 mmol) was slowly added therein. Thiosalycilic chloride dissolved in dichloromethane was also added therein at 0°C, and the mixture was stirred for 30 minutes. Water was added to quench the reaction, and aqueous layer was extracted with chloroform. Combined organic layer was dried over anhydrous sodium sulfate, filtered and concentrated under reduced pressure. The residue was purified by column chromatography (chloroform:methanol = 12:1), to give 210 mg (yield: 66%, white solid) of the target compound.

$^1$H NMR (400MHz, $CDCl_3$). δ2.06-2.17(m, 1H), 2.33-2.49(m, 2H), 2.42(s, 3H), 2.54-2.59(m, 1H), 3.72(s, 3H), 3.92(s, 3H), 3.96(s, 3H), 4.86-4.93(m, 1H), 6.56(s, 1H), 7.06(d, J=10.6Hz, 1H), 7.14(t, J=7.5Hz, 1H), 7.24(d, J=7.7Hz, 1H), 7.31(d, J=10.6Hz, 1H), 7.51(s, 1H), 7.58(d, J=7.3Hz, 1H), 7.63(t, J=8.6Hz, 2H)

<Step 2> Preparation of 2-nitrosothio-N-[(7S)-1,2,3-trimethoxy-10-methylsulfanyl-9-oxo-5,6,7,9-tetrahydro-benzo[a]heptalen-7-yl]-benzamide

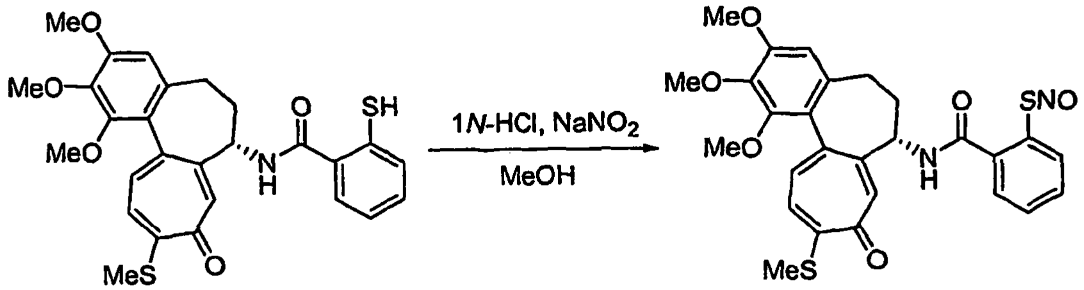


Compound prepared in step 1 (40 mg, 0.078 mmol) was dissolved in methanol. 1 N HCl aqueous solution (3 ml) was added therein. Sodium nitrite ($NaNO_2$, 6.5 mg, 0.094 mmol) dissolved in water (1.5 ml) was also added therein, and the mixture was stirred at room temperature for 1 hour. Sodium hydrogen carbonate was added to quench the reaction, and aqueous layer was extracted with chloroform. Combined organic layer was dried over anhydrous sodium sulfate, filtered and concentrated under reduced pressure. The residue was purified by column chromatography (chloroform:methanol = 12:1) and recrystallized in methanol, to give 34.1 mg (yield: 81%, yellow solid) of the target compound.

$^1H$ NMR (400MHz, $CDCl_3$): δ2.07-2.14(m, 1H), 2.31-2.37(m, 1H), 2.42(s, 3H), 2.45-2.48(m, 1H), 2.54-2.59(m, 1H), 3.72(s, 3H), 3.91(s, 3H), 3.96(s, 3H), 4.88-4.94(m, 1H), 6.56(s, 1H), 7.06(d, J=10.6Hz, 1H), 7.10(t, J=7.5Hz, 1H), 7.21(t, J=6.9Hz, 1H), 7.31(d, J=10.6Hz, 1H), 7.54(s, 1H), 7.64(t, J=8.6Hz, 2H), 7.72(d, J=7.3Hz, 1H)

Reference Example 36: Preparation of 3-nitrosooxymethyl-*N*-[(7S)-1,2,3-trimethoxy-10-methylsulfanyl-9-oxo-5,6,7,9-tetrahydro-benzo[a]heptalen-7-yl]-benzamide

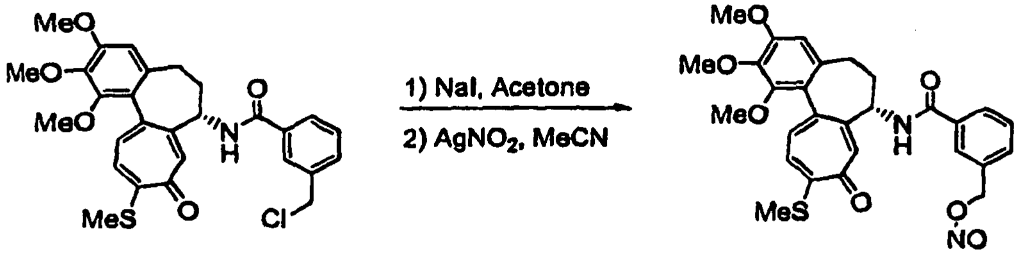


3-Chloromethyl-*N*-[(7S)-1,2,3-trimethoxy-10-methylsulfanyl-9-oxo-5,6,7,9-tetrahydrobenzo[a]heptalen -7-yl]-benzamide (119.8 mg, 0.228 mmol) and sodium iodide (136,5 mg, 0.911 mmol) were dissolved in acetone (15 ml), and the mixture was stirred at 55°C for 1 day. The reaction mixture was extracted with chloroform and washed with saturated sodium chloride aqueous solution. Combined organic layer was dried over anhydrous sodium sulfate, filtered and concentrated under reduced pressure. The residue and silver nitrite (125.5 mg, 0.812 mmol) were dissolved in acetonitrile (5 ml), and the mixture was stirred at room temperature for 1 day. The reaction mixture was extracted with chloroform. Combined organic layer was dried over anhydrous sodium sulfate, filtered and concentrated under reduced pressure. The residue was purified by column chromatography (ethyl acetate:chloroform = 4:1), to give 35.4 mg (yield: 32.4%, yellow solid) of the target compound.

$^1H$ NMR (400 MHz, $CDCl_3$): δ2.35-2.55(m, 3H), 2.48(s, 3H), 2.62-2.66(m, 1H), 3.76(s, 3H), 3.84(d, *J*=12.8Hz, 1H), 3.92(s, 3H), 3.98(s, 3H), 4.26(d, *J*=12.8Hz, 1H), 4.91-4.97(m, 1H), 6.58(s, 1H), 6.96(dd, *J*=7.7, 7.7Hz, 1H), 7.22(d, *J*=10.6Hz, 1H), 7.26-7.30(m, 2H), 7.44-7.47(m, 2H), 7.86(s, 1H), 9.30(d, *J*=6.8Hz, 1H)

Example 37: 3-fluoro-5-nitrosooxymethyl-*N*-[(7S)-1,2,3-trimethoxy-10-methylsulfanyl-9-oxo-5,6,7,9-tetrahydro-benzo[a]heptalen-7-yl]-benzamide

A target compound was synthesized in analogy to the procedure as described in the Example 36.

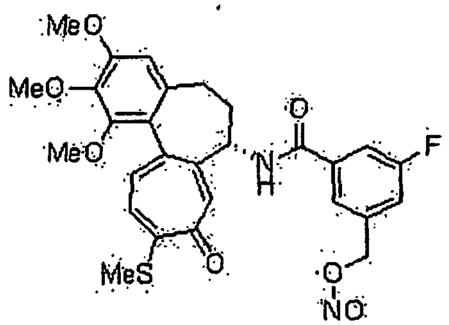

$^1$H NMR (400 MHz, $CDCl_3$): δ2. 04~2.20(m, 1H), 2.47(s, 3H), 2.52~2.62(m, 2H), 2.67~.2.72(m, 1H), 3.81(s, 3H), 3.91(s, 3H), 3.97(s, 3H), 4.71(s, 2H), 4.93~4.96(m, 1H), 6.42(d, J=6.2, 1H), 6.58(s, 1H), 7.14~7.21(m, 2H), 7.30 (d, J=8.4, 1H), 7.42(s, 1H), 7.53(d, J=10.6, 1H)

Reference Example 39: Preparation of 3-nitrosothiomethyl-*N*-[(7S)-1,2,3-trimethoxy-10-methylsulfanyl-9-oxo-5,6,7,9-tetrahydro-benzo[a]heptalen-7-yl]-benzamide

<Step 1> Preparation of methanesulfonic acid-3-[(7S)-12,3-trimethoxy-10-methylsulfanyl-9-oxo-5,6,7,9-tetrahydro-benzo[a]heptalen-7-yl-carbamoyl]-benzyl ester

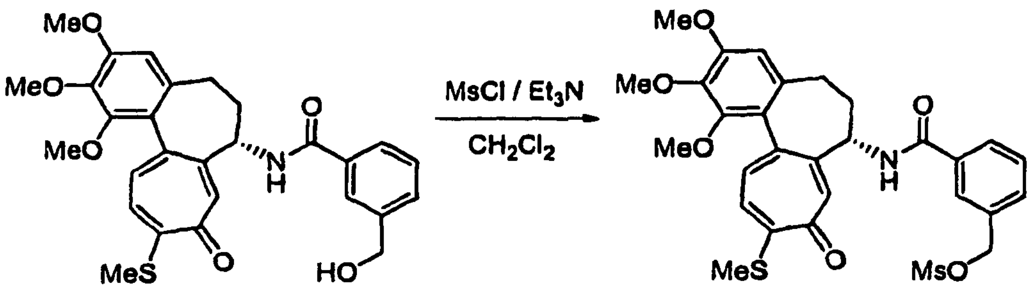


3-Hydroxymethyl-*N*-[(7S)-1,2,3-trimethoxy-10-methylsulfanyl-9-oxo-5,6,7,9-tetrahydro-benzo[a]heptalen-7-yl]-benzamide (252.5 mg, 0.497 mmol) was dissolved in dichloromethane (10 ml), and the temperature was lowered into 0°C. Methanesulfonylchloride (42.4 μℓ, 0.547 mmol) and triethylamine (0.104 ml, 0.745 mmol) were added therein, and the mixture was stirred at room temperature for 2 hours. The reaction mixture was extracted with chloroform. Combined organic layer was dried over anhydrous sodium sulfate, filtered and concentrated under reduced pressure. The residue was purified by column chromatography (ethyl acetate:chloroform = 3:2), to give 182.3 mg (yield: 62.6%, yellow solid) of the target compound.

$^1$H NMR (400 MHz, $CDCl_3$): δ2.14-2.21(m, 1H), 2.31-2.50(m, 2H), 2.46(s, 3H), 2.57-2.62(m, 1H), 2.85(s, 3H), 3.76(s, 3H), 3.92(s, 3H), 3.97(s, 3H), 4.87-4.94(m, 1H), 5.06(d, *J*=12.7Hz, 1H), 5.10(d, *J*=12.7Hz, 1H), 6.57(s, 1H), 7.14 (d, *J*=10.6Hz, 1H), 7.28(dd, *J*=7.7, 7.7Hz, 1H), 7.38-7.42(m, 2H), 7.56(s, 1H), 7.74(d, *J*=8.0Hz, 1H), 8.24(d, *J*=7.7Hz, 1H)

<Step 2> Preparation of thioacetic acid-S-3-[(7S)-1,2,3-trimethoxy-10-methylsulfanyl-9-oxo-5,6,7,9-tetrahydro-benzo[a]heptalen-7-yl-carbamoyl]-benzyl ester

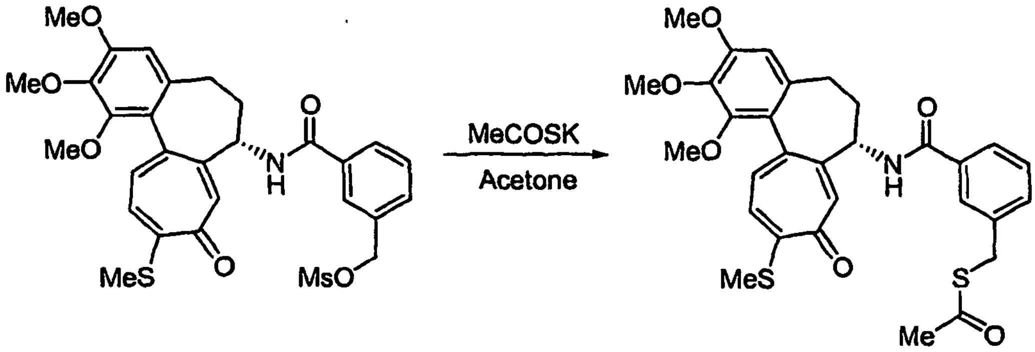


A compound prepared in the step 1 (182.3 mg, 0.311 mol) was dissolved in acetone (6 ml), and the temperature was lowered into 0°C. Potassium thioacetate (53.2 mg, 0.467 mmol) was slowly added therein at 0°C, and the mixture was stirred for 1 hour. The reaction mixture was extracted with chloroform. Combined organic layer was dried over anhydrous sodium sulfate, filtered and concentrated under reduced pressure, to give 173.6 mg (yield: 98.6%, yellow solid) of the target compound.

$^{1}$H NMR (400MHz, $CDCl_3$) : δ2.05-2.14 (m, 1H) , 2.30(s, 2H), 2.32-2.49(m, 2H), 2.44(s, 3H), 2.52-2.61(m, 1H), 3.74(s, 3H), 3.92(s, 3H), 3.97(s, 3H), 4.01(d, *J*=13.9Hz, 1H), 4.05(d, *J*=13.9Hz, 1H), 4.87-4.93(m, 1H), 6.56(s, 1H), 7.10 (d, *J*=10.3Hz, 1H), 7.21(dd, *J*=7.7, 7.7Hz, 1H), 7.32-7.36(m, 2H), 7.49(s, 1H), 7.64-7.71(m, 3H)

<Step 3> Preparation of 3-mercaptomethyl-*N*-[(7S)-1,2,3-trimethoxy-10-methylsulfanyl-9-oxo-5,6,7,9-tetrahydro-benzo[a]heptalen-7-yl]-benzamide

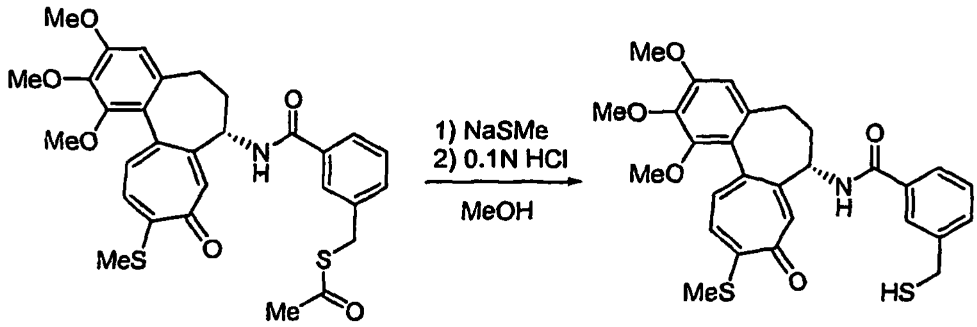


A compound prepared in the step 2 (165.2 mg, 0.292 mol) was dissolved in methanol (6 ml), and the temperature was lowered into 0°C. Sodium thiomethoxide (21.5 mg, 0.307 mmol) was slowly added therein at 0°C, and the mixture was stirred at room temperature for 30 minutes. Reaction was quenched by adding 0.1N HCl aqueous solution. The reaction mixture was extracted with chloroform and washed with saturated sodium chloride aqueous solution. Combined organic layer was dried over anhydrous sodium sulfate, filtered and concentrated under reduced pressure. The residue was purified by column chromatography (ethyl acetate:chloroform = 1:1), to give 182.3 mg (yield: 62.6%, yellow solid) of the target compound.

$^{1}$H NMR (400MHz, $CDCl_3$) : δ1.70(t, *J*=7.3Hz, 1H), 2.12-2.19(m, 1H), 2.31-2.50(m, 2H), 2.18(s, 3H), 2.57-2.61 (m, 1H), 3.58 (d, *J*=7.3Hz, 1H), 3.74(s, 3H), 3.92(s, 3H), 3.97(s, 3H), 4.89-4.95(m, 1H), 6.57(s, 1H), 7.12(d, *J*=10.3Hz, 1H), 7.19(dd, *J*=7.7, 7.7Hz, 1H), 7.34(d, *J*=7.7Hz, 1H), 7.37(d, *J*=10.3Hz, 1H), 7.55(s, 1H), 7.59(d, *J*=7.7Hz, 1H), 7.70 (s, 1H), 7.93(d, *J*=7.3Hz, 1H)

<Step 4> Preparation of 3-nitrosothiomethyl-*N*-[(7S)-1,2,3-trimethoxy-10-methylsulfanyl-9-oxo-5,6,7,9-tetrahydro-benzo[a]heptalen-7-yl]-benzamide

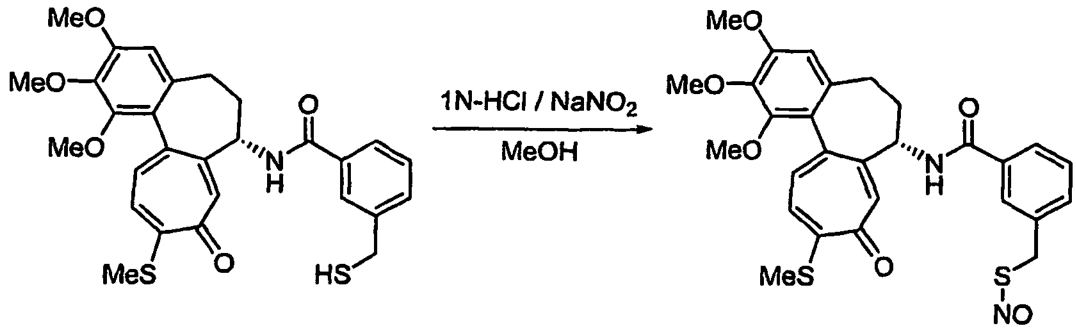


A compound prepared in the step 3 (101.2 mg, 0.193 mol) was dissolved in methanol (3 ml) and dimethylformamide (3ml), and the temperature was lowered into 0°C. 0.1N HCl aqueous solution (3 ml) and sodium nitrite (16.0 mg) were slowly added therein at 0°C, and the mixture was stirred at room temperature for 2 hours. The reaction mixture was extracted with chloroform and washed with saturated sodium carbonate solution. Combined organic layer was dried over anhydrous sodium sulfate, filtered and concentrated under reduced pressure. The residue was purified by column chromatography (ethyl acetate:chloroform:hexane = 3:2:1.5), to give 19.5 mg (yield: 18.3%, yellow solid) of the target compound.

$^1$H NMR (400MHz, $CDCl_3$) : δ2.08-2.16 (m, 1H), 2.31-2.51(m, 2H), 2.45(s, 3H), 2.57-2.61(m, 1H), 3.57(s, 3H), 3.80(s, 2H), 3.92(s, 3H), 3.97(s, 3H), 4.89-4.96(m, 1H), 6.57(s, 1H), 7.10(d, *J*=10.3Hz, 1H), 7.25(dd, *J*=7.7, 7.7Hz, 1H), 7.36(d, *J*=10.3Hz, 1H), 7.38(d, *J*=7.7Hz, 1H), 7.53(s, 1H), 7.68(d, *J*=7.7Hz, 1H), 7.77(s, 1H), 7.79(d, *J*=7.0Hz, 1H)

<u>Example 39: 3-fluoro-5-nitrosothiomethyl-*N*-[(7S)-1,2,3-trimethoxy-10-methylsulfanyl-9-oxo-5,6,7,9-tetrahydro-benzo [a]heptalen-7-yl]-benzamide</u>

A target compound of Example 39 was synthesized in analogy to the procedure as described in the Example 38.

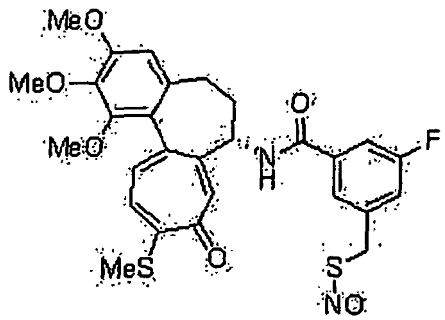

$^1$H NMR (400MHz, $CDCl_3$) : δ2.30-2.48 (m, 3H), 2.44 (s, 3H), 2.56~2.59(m, 1H), 3.57(q, J=13.9, 16.5, 2H), 3.73(s, 3H), 3.91(s, 3H), 3.97(s, 3H), 4.97-5.03(m, 1H), 6.57(s, 1H), 7.02(d, J=8.4, 1H), 7.15(d, J=10.6, 1H), 7.39(d, J=10.2, 1H), 7.43(s, 1H), 7.64(d, J=9.1, 1H), 7.73(s, 1H), 8.96(d, J=7.3, 1H)

<u>Example 40: Preparation of 3-fluoro-5-nitrooxymethyl-N-[(7S)-1,2,3,10-tetramethoxy-9-oxo-5,6,7,9-tetrahydro-benzo [a]heptalen-7-yl]-benzamide</u>

<u><Step 1> Preparation of 3-fluoro-5-hydroxymethyl-N-[(7S)-1,2,3,10-tetramethoxy-9-oxo-5,6,7,9-tetrahydro-benzo[a] heptalen-7-yl]-benzamide</u>

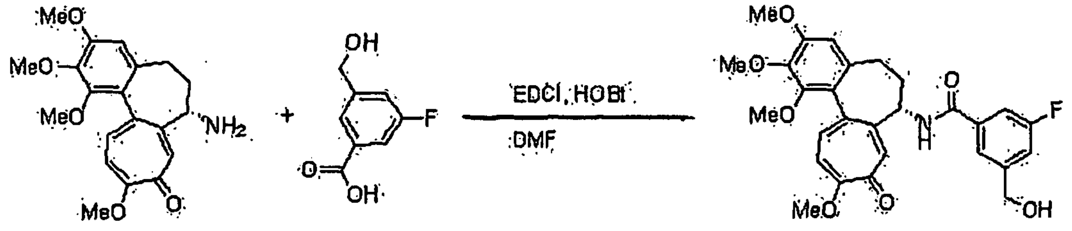


Deacetylcolchicine (150 mg, 0.42 mmol), 3-fluoro-5-hydroxymethyl-benzoic acid (85 mg, 0.50 mmol) and HOBt (67 mg, 0.50 mmol) were dissolved in dimethylformamide solution (2 ml), and the temperature was lowered into 0°C. EDCI (95 mg, 0.50 mmol) was slowly added therein at 0°C, and the mixture was stirred at room temperature. Water was added to quench the reaction, and aqueous layer was extracted with ethyl acetate. Combined organic layer was dried over anhydrous sodium sulfate, filtered and concentrated under reduced pressure. The residue was purified by column chromatography (chloroform:ethyl acetate = 2:1), to give 110 mg (yield: 52%, yellow solid) of the target compound.

$^1H$ NMR (400MHz, $CDCl_3$) : δ2.40-2.58 (m, 3H), 2.62-2.69(m, 1H), 3.64-3.78 (m, 1H), 3.74(s, 3H), 3.92(s, 3H), 3.98(s, 3H), 4.07(S, 3H), 4.23-4.28 (m, 1H), 4.81-4.88 (m, 1H), 6.58(s, 1H), 6.87(d, *J*=9.2Hz, 1H), 6.97(d, *J*=9.2Hz, 1H), 7.03(d, *J*=10.4Hz, 1H), 7.38(s, 1H), 7.50(d, *J*=10.4Hz, 1H), 7.96(s, 1H), 9.64(d, *J*=6.0Hz, 1H)

<Step 2> Preparation of 3-fluoro-5-nitrooxymethyl-N-[(7S)-1,2,3,10-tetramethoxy-9-oxo-5,6,7,9-tetrahydro-benzo[a]heptalen-7-yl]-benzamide

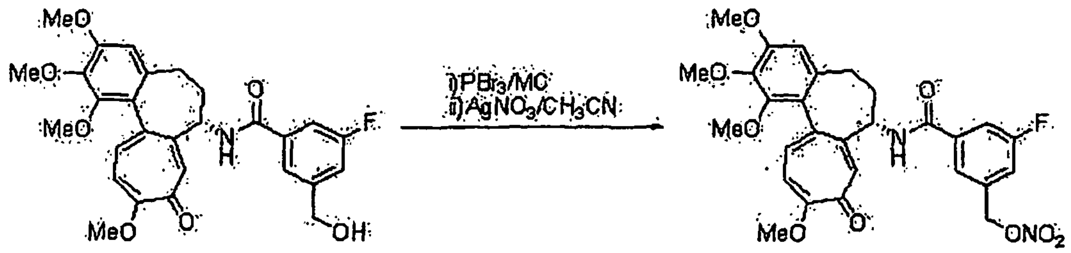


According to the similar procedure in the step 2 and 3 of Example 5, by using a compound prepared in the step 1 (90 mg, 0.18 mmol), 30 mg (yield: 30%, yellow solid) of the target compound was obtained.

$^1H$ NMR (400MHz, $CDCl_3$) : δ2.38-2.49(m, 3H), 2.58-2.59(m, 1H), 3.75(s, 3H), 3.92(s, 3H), 3.98(s, 3H), 4.05 (S, 3H), 4.89-4.93(m, 1H), 4.94(d, *J*=12.8Hz, 1H), 5.10(d, *J*=12.8Hz, 1H), 6.58(s, 1H), 6.96(d, *J*=8.4Hz, 1H), 7.01(d, *J*=10.4Hz, 1H), 7.17(d, *J*=8.4Hz, 1H), 7.45(s, 1H), 7.48(d, *J*=10.4Hz, 1H), 7.89(s, 1H), 9.25(d, *J*=6.4Hz, 1H)

Reference Example 41: Preparation of 3-nitrooxymethyl-N-methyl-N-[(7S)-1,2,3-trimethoxy-10-methylsulfanyl-9-oxo-5,6,7,9-tetrahydro-benzo[a]heptalen-7-yl]-benzamide

<Step 1> Preparation of 3-chloromethyl-N-methyl-N-[(7S)-1,2,3-trimethoxy-10-methylsulfanyl-1-9-oxo-5,6,7,9-tetrahydro-benzo[alheptalen-7-yl]-benzamide

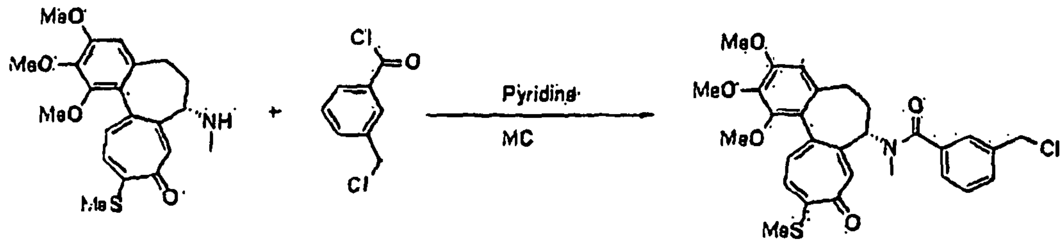


Thiodemecolcine (50 mg, 0.129 mmol) and pyridine (0.012 ml, 0.154 mmol) were dissolved in dichloromethane, and the temperature was lowered into 0°C. 3-(Chloromethyl)benzoyl chloride (0.022 ml, 0.154 mmol) was slowly added therein at 0°C, and the mixture was stirred at room temperature. Water was added to quench the reaction, and aqueous layer was extracted with ethyl acetate. Combined organic layer was dried over anhydrous sodium sulfate, filtered and concentrated under reduced pressure. The residue was purified by column chromatography (chloroform:ethyl acetate = 2:1), to give 45 mg (yield: 67%, yellow solid) of the target compound.

$^1$H NMR (400MHz, $CDCl_3$): δ2.24-2.38(m, 2H), 2.44(s, 3H),2.45-2.58(m, 1H), 2.61-2.74(m, 1H), 3.25(s, 3H), 3.72(s, 3H), 3.91(s, 3H), 3.92(s, 3H), 4.54(s, 2H), 5.05(br, 1H), 6.57(s, 1H), 7.06(d, *J*=10.0Hz, 1H), 7.09(s, 1H), 7.22-7.41 (m, 5H)

<Step 2> Preparation of 3-nitrooxymethyl-N-methyl-N-[(7S)-1,2,3-trimethoxy-10-methylsulfanyl-9-oxo-5,6,7,9-tetrahydro-benzo[a]heptalen-7-yl]-benzamide

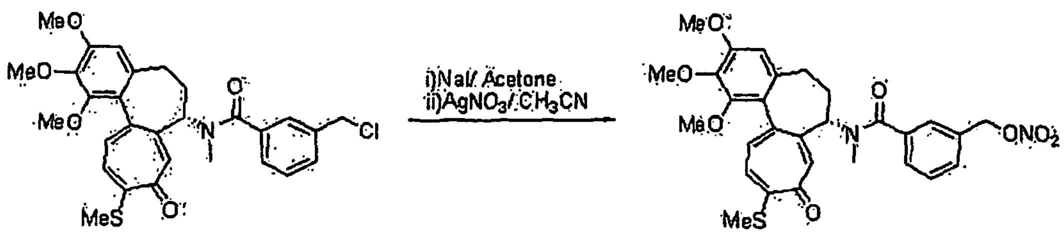


According to the similar procedure in the step 2 of Example 25, by using a compound prepared in the step 1 (45 mg, 0.085 mmol), 40 mg (yield: 83%, yellow solid) of the target compound was obtained.

$^1$H NMR (400MHz, $CDCl_3$) : δ2.24-2.38(m, 2H), 2.44(s, 3H),2.45-2.58(m, 1H), 2.61-2.74(m, 1H), 3.24(s, 3H), 3.71(s, 3H), 3.91(s, 3H), 3.92(s, 3H), 4.77(br, 1H), 5.58(s, 2H), 6.85(s, 2H), 7.21(d, *J*=10.0Hz, 1H), 7.29(d, *J*=10.4Hz, 1H), 7.40-7.53(m, 4H)

Example 42: Preparation of 3-fluoro-N-methyl-5-nitrooxymethyl-N-[(7S)-1,2,3-trimethoxy-10-methylsulfanyl-9-oxo-5,6,7,9-tetrahydro-benzo[a]heptalen-7-yl]-benzamide

<Step 1> Preparation of 3-fluoro-5-hydroxymethyl-N-methyl-N-[(7S)-1,2,3-trimethoxy-10-methylsulfanyl-9-oxo-5,6,7,9-tetrahydro-benzo[a]heptalen-7-yl]-benzamide

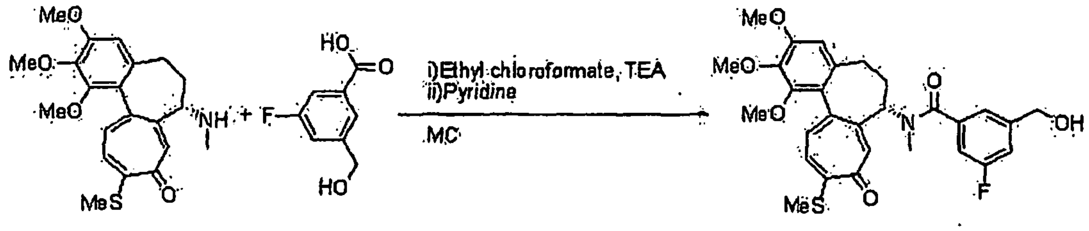


3-Fluoro-5-hydroxymethyl-benzoic acid (52 mg, 0.309 mmol) was dissolved in dichloromethane (3 ml) under a nitrogen atmosphere. Ethylchloroformate (0.022 ml, 0.231 mmol) and TEA (0.042 ml, 0.309 mmol) were slowly added therein at 0°C, and the mixture was stirred at 0°C for 30 minutes. Pyridine (0.012 ml, 0.154 mmol) and thiodemecolcine (60 mg, 0.154 mmol) were also added therein, and the mixture was stirred at room temperature for 3 hours. Water was added to quench the reaction, and aqueous layer was extracted with ethyl acetate. Combined organic layer was dried over anhydrous sodium sulfate, filtered and concentrated under reduced pressure. The residue was purified by column chromatography (chloroform:ethyl acetate = 2:1), to give 30 mg (yield: 21%, yellow solid) of the target compound.

$^1$H NMR (400MHz, $CDCl_3$) : δ1.76(br, 1H), 2.24-2.38(m, 2H), 2.44(s, 3H),2.45-2.58(m, 1H), 2.61-2.74(m, 1H), 3.23(s, 3H), 3.72(s, 3H), 3.93(s, 3H), 3.97(s, 3H), 4.65(s, 2H), 5.02(br, 1H), 6.57(s, 1H), 6.92(s, 1H), 6.99-7.24(m, 4H), 7.34(s, 1H)

<Step 2> Preparation of 3-fluoro-N-methyl-5-nitrooxymethyl-N-[(7S)-1,2,3-trimethoxy-10-methylsulfanyl-9-oxo-5,6,7,9-tetrahydro-benzo[a]heptalen-7-yl]-benzamide

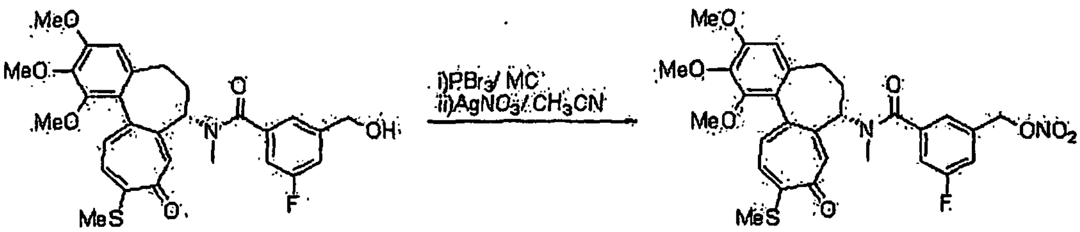


According to the similar procedure in the step 2 and 3 of Example 5, by using a compound prepared in the step 1 (55 mg, 0.101 mmol), 20 mg (yield: 37%, yellow solid) of the target compound was obtained.

$^1$H NMR (400MHz, $CDCl_3$) : δ2.22-2.41(m, 2H), 2.45(s, 3H), 2.45-2.58(m, 1H), 2.61-2.74(m, 1H), 3.24(s, 3H), 3.75(s, 3H), 3.91(s, 3H), 3.95(s, 3H), 5.02(br, 1H), 5.39(s, 2H), 6.57(s, 1H), 7.05-7.24(m, 5H), 7.34(s, 1H)

Example 43: Preparation of 2-(3-fluoro-5-nitrooxymethyl-phenyl)-N-[(7S)-1,2,3-trimethoxy-10-methylsulfanyl-9-oxo-5,6,7,9-tetrahydro-benzo[a]heptalen-7-yl]-acetamide

<Step 1> Preparation of (3-fluoro-5-hydroxymethylphenyl)-acetic acid

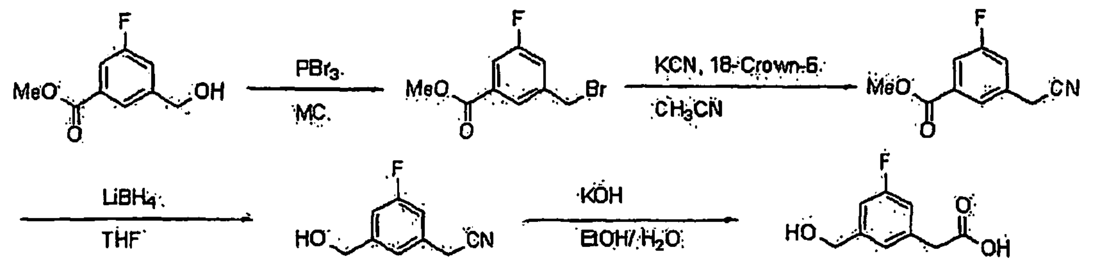


A compound prepared in the step 1 of intermediate 6 (2.5 g, 13.57 mmol) was dissolved in dichloromethane (30 ml). $PBr_3$ (1.15 ml, 12.21 mmol) was slowly added therein, and the mixture was stirred at room temperature for 3 hours to give 3-bromomethyl-5-fluorobenzoic acid methyl ester (1.6 g, yield: 50%, white solid). 3-Bromomethyl-5-fluorobenzoic acid methyl ester (1.5 g, 6.1 mmol), KCN (1.6 g, 24.4 mmol) and 18-crown-6 (820 mg, 3.1 mmol) were dissolved in acetonitrile (10 ml). The reaction mixture was stirred at room temperature for 18 hours to give 3-cyanomethyl-5-fluorobenzoic acid methyl ester (900 mg, yield: 77%, white solid). 3-Cyanomethyl-5-fluoro-benzoic acid methyl ester (900 mg, 4.65 mmol) was dissolved in tetrahydrofuran (10ml). 2M Lithiumborohydride tetrahydrofuran (2.3 ml, 4.6 mmol) was slowly added therein, and the mixture was refluxed, to give (3-fluoro-5-hydroxymethyl-phenyl)-acetonitrile (430 mg, yield: 56%, white solid). (3-Fluoro-5-hydroxymethylphenyl)-acetonitrile (400 mg, 2.42 mmol) and potassiumhydroxide (1.34 g, 23.8 mmol) were dissolved in ethanol (10 ml) and water (5 ml). The reaction mixture was refluxed for 24 hours to give 356 mg (yield: 80%, white solid) of the target compound.

1H NMR(400MHz, $CD_3OD$) ; δ3.62(s, 2H), 4.59(s, 2H), 6.94(d, *J*=9.6Hz, 1H), 7.04(d, *J*=9.6Hz, 1H), 7.08(s, 1H)

<Step 2> Preparation of 2-(3-fluoro-5-hydroxymethylphenyl)-N-[(7S)-1,2,3-trimethoxy-10-methylsulfanyl-9-oxo-5,6,7,9-tetrahydro-benzo[a]heptalen-7-yl]-acetamide

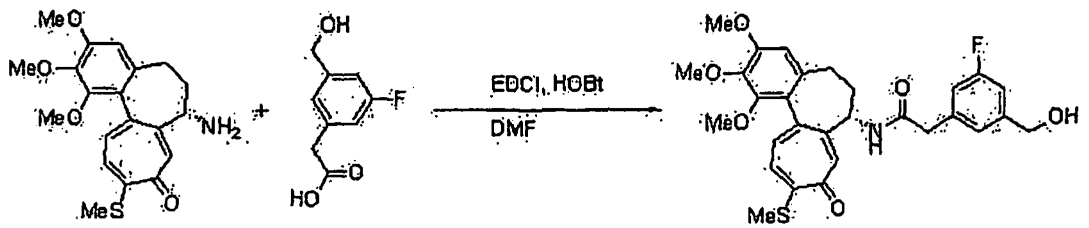


According to the similar procedure in the step 1 of Example 40, by using a compound prepared in the step 1 (30 mg, 0.16 mmol), 58 mg (yield: 70%, yellow solid) of the target compound was obtained.

$^1$H NMR (400MHz, $CDCl_3$) : δ1.92-2.01(m, 1H), 2.19-2.40(m, 2H), 2.41(s, 3H), 2.46-2.52(m, 1H), 3.46(d, *J*=14.0,Hz, 1H), 3.52(d, *J*=14.0,Hz, 1H), 3.63(s, 3H), 3.89(s, 3H), 3.91(s, 3H), 4.64-4.74(m, 1H), 4.75(s, 2H), 6.53(s, 1H), 6.86-6.94(m, 3H), 7.07(d, *J*=10.8Hz, 1H), 7.13(s, 1H), 7.30(d, *J*=10.8Hz, 1H), 7.39(s, 1H)

<Step 3> Preparation of methanesulfonic acid 3-fluoro-5-[(7S)-(1,2,3-trimethoxy-10-methylsulfanyl-9-oxo-5,6,7,9-tetrahydro-benzo[a]heptalen-7-ylcarbamoyl)-methyl]-benzyl ester

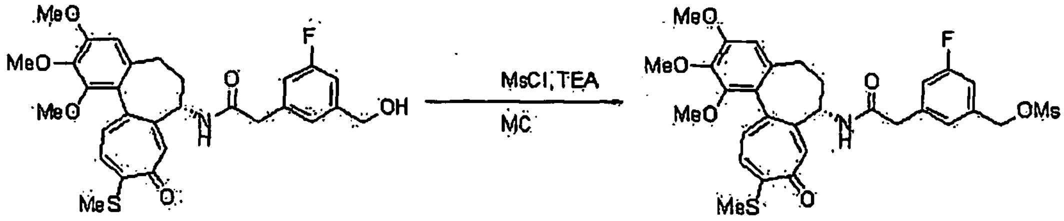


According to the similar procedure in the step 1 of Example 38, by using a compound prepared in the step 2 (58 mg, 0.107 mmol), 60 mg (yield: 90%, yellow solid) of the target compound was obtained.

<Step 4> Preparation of 2-(3-fluoro-5-nitrooxymethyl-phenyl)-N-[(7S)-1,2,3-trimethoxy-10-methylsulfanyl-9-oxo-5,6,7,9-tetrahydro-benzo[a]heptalen-7-yl]-acetamide

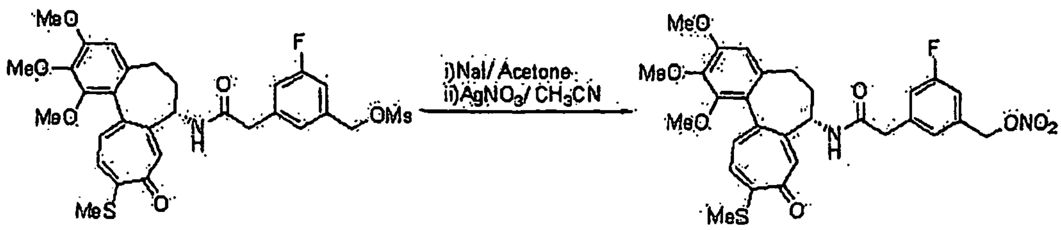


According to the similar procedure in the step 2 of Example 25, by using a compound prepared in the step 3 (60 mg, 0.097 mmol), 30 mg (yield: 83%, yellow solid) of the target compound was obtained.

$^1$H NMR (400MHz, $CDCl_3$) : δ1.92-1.98(m, 1H), 2.22-2.42(m, 2H), 2.46(s, 3H), 2.49-2.52(m, 1H), 3.50(d, *J*=14.0,Hz, 1H), 3.65(s, 3H), 3.66(d, *J*=14.0,Hz, 1H), 3.89(s, 3H), 3.93(s, 3H), 4.64-4.74(m, 1H), 5.37(s, 2H), 6.52(s, 1H), 6.97(d, *J*=8.8Hz, 1H), 7.11-7.17(m, 3H), 7.35(d, *J*=10.8Hz, 1H), 7.50(s, 1H), 8.04(d, *J*=7.2Hz, 1H)

Example 44 : 2-(2-fluoro-5-nitrooxymethyl-phenyl)-N-[(7S)-1,2,3-trimethoxy-10-methylsulfanyl-9-oxo-5,6,7,9-tetrahydro-benzo[a]heptalen-7-yl]-acetamide

A compound of Example 44 was synthesized in analogy to the procedure as described in Example 43, and an intermediate was prepared by the method described as follows.

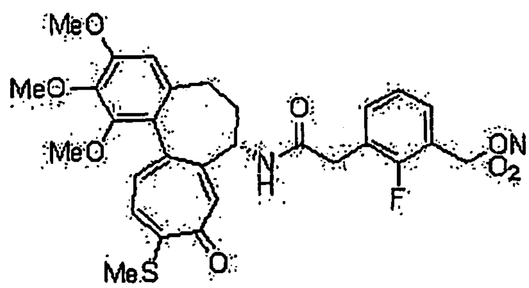

$^1$H NMR (400MHz, $CDCl_3$) : δ1.89-1.93(m, 1H), 2.26-2.29(m, 1H), 2.34-2.40(m, 1H), 2.43(s, 3H), 2.49-2.54(m, 1H), 3.61(s, 3H), 3.62(d, *J*=15.2Hz, 1H), 3.72(d, *J*=15.2Hz, 1H), 3.89(s, 3H), 3.92(s, 3H), 4.70-4.75(m, 1H), 5.49(s, 2H), 6.53(s, 1H), 7.06-7.11(m, 2H), 7.25-7.31(m, 2H), 7.35(t, *J*=7.6Hz, 1H), 7.44(s, 1H), 7.57(d, *J*=7.2Hz, 1H)

<Intermediate 18> Preparation of (2-fluoro-5-hydroxymethyl-phenyl)-acetic acid

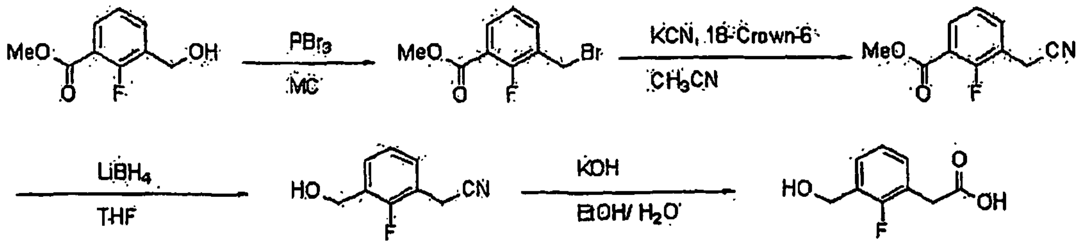


According to the similar procedure in the step 1 of Example 43, by using a compound prepared in the intermediate 5 (2.2 g, 11.94 mmol), 400 mg (yield: 90%, white solid) of the target compound was obtained.

Experimental Example 1: Cytotoxicity test to cancer cell lines

Cytotoxicity to A549 (Korea Research Institute of Chemical Technology), SK-OV-3 (Korea Research Institute of Chemical Technology), SK-MEL-2 (Korea Research Institute of Chemical Technology), HCT-15 (Korea Research Institute of Chemical Technology) and MCF7 (Korean Cell Line Bank, Seoul National University School of medicine) cells was measured by Sulforhodamin-B (SRB) method (1989, National Cancer Institute (NCI)) which was developed for the measurement of *in vitro* anticancer activity of a drug. Cells were separated using 0.25% trypsin-EDTA solution, leading to the preparation of a cell suspension by 5$\times$103 ~ 2$\times 10^4$ cells/well. Then, the suspension was distributed into a 96 well plate by 100 $\mu\ell$/well, which was cultured in a 37°C, 5% $CO_2$ incubator for 24 hours. Compounds prepared in examples of the present invention were used as a sample. Precisely, the compound was dissolved in dimethylsulfoxide and diluted with RPMI 1640 medium before being used as a sample. The final concentration of the sample used varied ranging 1 uM ~ 0.00001 uM. The medium was removed from the 96 well plate and then diluted sample solution was added by 100 $\mu\ell$/well, followed by further culture in a 37°C, 5% $CO_2$ incubator for 48 hours. Tz (time zero) plate was collected from the point of adding the sample. Upon completing the culture, the medium was removed from each well along with Tz plate, then 10% trichloroacetic acid (TCA) was added by 100 $\mu\ell$/well. The plate was left at 4°C for 1 hour to let cells be fixed on the floor of the plate. After cells were fixed completely, the plate was washed with water 5-6 times to remove the remaining trichloroacetic acid solution completely and moisture was completely dried at room temperature. 0.4% Sulforhodamine-B was dissolved in 1% acetic acid solution to prepare a staining solution. Cells were stained for 30 minutes with a dye added by 100 $\mu\ell$ to each well of the completely dried plate. Then, the plate was washed with 1% acetic acid solution 5-6 times to remove sulforhodamine-B remained uncombined with cells. Then, the plate was dried at room temperature. 10 mM Tris solution was added by 100 $\mu\ell$/well thereto to dissolve the dye, and optical density (OD) was measured at 520 nm with a micro plate reader.

$ED_{50}$ (concentration that inhibits cancer cell growth 50%, 50% effective dose, nM/ml) of the sample to cancer cell was calculated as follows. OD value at the point of beginning the culture with the sample was determined as Tz (time zero) value. OD value of a well to be cultured without the sample was determined as a control value (C). OD value of a well pretreated with the sample was determined as experimental value (T). After calculating Tz, C and T, cytotoxicity of the sample was measured by the below <Mathematical Formula 1>.

<Mathematical Formula 1>

$$T \leq Tz,\ (T-Tz)/(C-Tz) \times 100$$

$$T > Tz,\ (T-Tz)/Tz \times 100$$

$ED_{50}$, the concentration that can inhibit cancer cell growth by 50%, was calculated by using a regression analysis of lotus program based on the degree of cytotoxicity obtained by the <Mathematical Formula 1>.

Each $ED_{50}$ of paclitaxel, doxorubicin and colchicine, which were used as controls, was also calculated by the same way as described in the above.

Results were presented in Table 1.

<Table 1>

| Cytotoxicity to cancer cell lines (NT: Not tested) | | | | | |
|---|---|---|---|---|---|
| Example | Cytotoxicity [$ED_{50}$ : nM] | | | | |
| | A549 | SK-OV-3 | SK-MEL-2 | HCT15 | MCF-7 |
| Paclitaxel | 0.3 | 1.2 | 0.1 | 0.1 | 0.9 |
| Doxorubicin | 13.0 | 47.0 | 16.0 | 25.0 | 50.0 |
| Colchicine | 21.0 | 18.0 | 6.0 | 9.0 | NT |
| Example 1 | 1.8 | 1.0 | 0.4 | 0.6 | NT |
| Example 2 | 15.8 | 9.3 | 5.4 | 2.3 | 2.9 |
| Reference Example 3 | 2.4 | 4.0 | 1.0 | 4.8 | 2.3 |
| Reference Example 4 | 0.01 | 0.01 | 0.01 | 0.03 | 0.01 |
| Example 5 | 1.0 | 1.0 | 3.1 | 4.4 | NT |
| Example 6 | 0.70 | 0.37 | 0.08 | 0.27 | 0.16 |
| Reference Example 7 | >50 | >50 | >50 | >50 | >50 |
| Example 8 | 0.02 | 0.04 | 0.01 | 0.03 | 0.01 |
| Example 9 | 0.49 | 0.34 | 0.22 | 0.64 | 0.13 |
| Example 10 | 0.28 | 0.23 | 0.12 | 0.39 | 0.09 |
| Example 11 | >50 | >50 | >50 | >50 | 39.0 |
| Example 12 | 0.05 | 0.27 | 0.11 | 0.05 | 0.03 |
| Example 13 | 0.13 | 0.18 | 0.04 | 0.11 | 0.02 |
| Example 14 | 28.2 | 30.6 | 32.8 | 9.9 | 11.2 |
| Example 15 | 30.2 | >50 | 22.5 | 23.4 | 18.8 |
| Example 16 | 1.6 | 1.0 | 1.0 | 1.0 | NT |
| Example 17 | 0.21 | 0.19 | 0.17 | 0.19 | 0.08 |
| Example 18 | 8.1 | 3.8 | 1.0 | 5.3 | NT |
| Reference Example 19 | 23.5 | 34.8 | 31.0 | 10.1 | 8.2 |
| Example 20 | 3.93 | 1.83 | 2.47 | 1.03 | 1.15 |
| Example 21 | 0.97 | 0.59 | 0.29 | 0.13 | 0.10 |
| Example 22 | 1.11 | 1.41 | 0.87 | 0.95 | 0.21 |
| Reference Example 23 | 5.4 | 5.6 | 3.5 | 3.4 | 3.3 |
| Example 25 | 8.8 | 5.4 | 14.0 | 15.0 | NT |
| Example 26 | 21.0 | >50.0 | 47.0 | >50.0 | NT |
| Example 27 | 5.0 | 16.0 | 7.0 | 2.0 | NT |
| Example 28 | 6.0 | 15.0 | 4.0 | 3.0 | NT |
| Example 29 | 1.1 | 3.0 | 0.9 | 0.8 | NT |
| Example 30 | 3.6 | 8.2 | 3.6 | 5.2 | NT |
| Example 31 | 1.10 | 3.0 | 0.9 | 0.8 | NT |
| Example 32 | 6.2 | 1.0 | 0.05 | 0.08 | NT |
| Example 33 | 3.8 | 6.6 | 2.9 | 1.0 | NT |

(continued)

| Cytotoxicity to cancer cell lines (NT: Not tested) | | | | | |
|---|---|---|---|---|---|
| Example | Cytotoxicity [$ED_{50}$ : nM] | | | | |
| | A549 | SK-OV-3 | SK-MEL-2 | HCT15 | MCF-7 |
| Example 34 | 0.06 | 0.01 | 0.01 | 0.01 | 0.01 |
| Reference Example 35 | >50 | >50 | 41.9 | 20.9 | 24.7 |
| Reference Example 36 | 2.9 | 1.1 | 0.1 | 0.7 | NT |
| Example 37 | >50 | 38.8 | 9.4 | 7.5 | 10.3 |
| Reference Example 38 | 2.4 | 4.1 | 3.3 | 3.0 | NT |
| Example 39 | 40.7 | 28.2 | >50 | 9.9 | 5.0 |
| Example 40 | 43.9 | 45.6 | 60.7 | 24.3 | 10.5 |
| Reference Example 41 | >50 | >50 | >50 | 36.4 | 47.3 |
| Example 42 | >50 | >50 | >50 | >50 | >50 |

As shown in Table 1, tricyclic derivatives according to the present invention showed very strong cytotoxicity to cancer cell lines.

Experimental Example 2: Inhibiting effect of tricyclic derivatives of the present invention on tumor growth

In order to investigate inhibiting effect of tricyclic derivatives of the invention on tumor growth, following experiments were performed.

Samples for the experiment were stored in a refrigerator all the time. Different dosages of compounds were administered to a test animal; dosage of a compound prepared in the Example 8 was 10 mg/kg, dosage of a compound prepared in the Example 12 was 1, 3, 10 mg/kg respectively and dosage of a positive control was 2.5 mg/kg (Taxol) and 2 mg/kg (Adriamycin). The compound of the Example 12 was dissolved in 4% tween 80, and the positive control Taxol was dissolved in a mixed solvent of 5% ethanol + 25% cremophor + 75% PBS. The prepared sample is subject to be precipitated, so that tip sonication was performed right before the administration to disperse it well.

7 week-old female S.P.F. BALB/c nude mice, provided by Charles River Co., Japan, were used as test animals. The test animals were adapted in a Hepa-filter room over a week before the test. The temperature was 21 ± 2°C , humidity was 55 ± 5% and 12-hour light and dark cycle was automatically repeated in that lab. Solid feed (CheilJedang) was sterilized by radioactive rays and drinking water was also sterilized by autoclave. Feed and water were taken by the animal freely. Cancer cell line used in this experiment was NCI-H460 (human lung tumor cell line) provided by Korea Research Institute of Bioscience and Biotechnology.

The tumor cell line, stored in liquid nitrogen, was thawed and cultured in a 37°C, 5% $CO_2$ incubator for a required time. Upon completing the culture, all the cells were recovered and cell concentration of the culture fluid was adjusted using PBS to $3\times10^7$ cells/ml. The adjusted cell culture solution was injected hypodermically into armpit between right shoulder girdle and chest wall by 0.3 ml per mouse. From the next day of grafting, NCI-H460 xenografted nude mice were administered intraperitoneal everyday with the sample solution by 0.2 ml/20 g of weight, once a day.

After the grafting of tumor cells, the volume of a tumor in each individual was measured in three dimensions by using a vernier caliper, which was represented in the below <Mathematical Formula 2>.

<Mathematical Formula 2>

$$\text{Tumor volume} = (\text{length}\times\text{width}\times\text{height})/2$$

Body weight changes of animal were measured three times a week. Each xenografted nude mouse was sacrificed to separate a tumor, which was then weighed.

All the test results of experimental groups were compared with those of control groups by t-Test to see if there is any significant difference between the two groups.

Changes of the volume and the weight of a tumor were shown in Table 2 and in FIG. 1, 3, and 5, and changes

of the body weight of a mouse were shown in Table 3, in FIG. 2 and in FIG. 4.

<Table 2>

| Experimental Group | Dosage (mg/kg) | Tumor volume ($mm^3$) | | | | | | Tumor Weight (mg) |
|---|---|---|---|---|---|---|---|---|
| | | Day 0 | Day 4 | Day 7 | Day 9 | Day 11 | Day 14 | Day 14 |
| V.C-1 | 0 | 0.0 ± 0.0 | 28.3 ± 8.1 | 53.5 ± 13.7 | 98.3 ± 25.5 | 167.5 ± 43.8 | 295.2 ± 62.2 | 1171.2 ± 383.5 |
| V.C-2 | 0 | 0.0 ± 0.0 | 26.7 ± 3.8 | 49.6 ± 12.3 | 80.9 ± 26.7 | 138.2 ± 54.9 | 289.0 ± 53.1 | 1087.9 ±300.5 |
| Example 8 | 10 | 0.0± 0.0 | 18.0 ± 3.7* | 25.2 ± 12.8** | 29.6 ± 13.8*** | 35.2 ± 14.0*** | 44.5 ± 15.0** | 143.4 ± 57.8*** |
| Example 12 | 1 | 0.0 ± 0.0 | 20.7 ± 3.1 | 49.2 ± 12.2 | 78.1 ± 13.0 | 146.5 ± 8.8 | 299.3 ± 41.5 | 950.9 ± 174.8 |
| | 3 | 0.0 ± 0.0 | 16.9 ± 5.1* | 31.2 ± 11.7* | 49.7± | 96.1 ± 15.8** 26.1** | 206.8± 48.0* | 714.5 ± 103.8* |
| | 10 | 0.0 ± 0.0 | 16.1 ± 6.7* | 24.6 ± 9.8** | 35.2 ± 12.9*** | 69.4 ± 26.9*** | 103.0 ± 0 390.9 ± | 0 |
| Taxol | 2.5 | 0.0 ± 0.0 | 17.5 ± 4.6*** | 32.7 ± 3.8** | 47.2 ± 0 | - | - | - |
| Adriamyc in | 2 | 0.0 ± 0.0 | 21.8 ± 8.3 | 33.7 ± 8.7* | 47.6 ± 16.2** | 81.6 ± 24.5** | 138.1 ± 39.9*** | 534.1 ± 87.9 |
| ※ Significance test (t-Test) : *(p<0.05), **(p<0.01), ***(p<0.001),<br>V.C-1 : 4% tween 80,<br>V.C-2 : 5% ethanol + 25% cremophor + 75% PBS | | | | | | | | |

<Table 3>

| Experimental Group | Dosage (mg/kg) | Animal Number (n) | Body weight Change (%) | | | | | |
|---|---|---|---|---|---|---|---|---|
| | | | Day 1 | Day 3 | Day 7 | Day 9 | Day 11 | Day 14 |
| V.C-1 | 0 | 6 | 100.0 ± 0.0 | 105.4 ± 2.5 | 108.7 ± 2.4 | 107.2 ± 3.7 | 107.8 ± 4.6 | 107.8 ± 3.2 |
| V.C-2 | 0 | 6 | 100.0 ± 0.0 | 106.9 ± 2.4 | 110.5 ± 1.5 | 108.5 ± 2.6 | 109.0 ± 52.6 | 108.9 ± 3.6 |
| Example 8 | 10 | 5 | 100.0 ± 0.0 | 103.5 ± 2.2 | 99.8 ± 4.3** | 92.3 ± 4.1*** | 69.3 ± 3.7*** | 89.4 ± 5.3*** |
| Example 12 | 1 | 6 | 100.0 ± 0.0 | 105.0 ± 1.4 | 104.2 ±5.3 | 99.6 ± 4.4** | 97.5 ± 3.6** | 98.3± 3.2*** |
| | 3 | 6 | 100.0 ± 0.0 | 102.8 ± 2.0 | 101.8 ± 4.2** | 99.3 ± 3.3* | 99.1 ± 2.8** | 94.5 ± 3.9*** |
| | 10 | 6 (Day 14-2) | 100.0 ± 0.0 | 103.9 ± 0.9 | 92.2 ± 3.5*** | 89.4 ± 2.0** | 92.3 ± 2.9*** | 93.4 ± 0 |

(continued)

| Experimental Group | Dosage (mg/kg) | Animal Number (n) | Body weight Change (%) | | | | | |
|---|---|---|---|---|---|---|---|---|
| | | | Day 1 | Day 3 | Day 7 | Day 9 | Day 11 | Day 14 |
| Taxol | 2.5 | 6 (Day 9-2, Day 11, Day 14-1) | 100.0 ± 0.0 | 106.0 ± 1.5 | 99.2± 4.0*** | 97.1±0 | - | - |
| Adriamyc in | 2 | 6 | 100.0 ± 0.0 | 105.0 ± 4.1 | 103.9 ± 5.5 | 100.5 ± 4.6* | 96.4 ± 3.5*** | 85.1 ± 1.8*** |
| ※ Significance test (t-Test) : * (p<0.05) , ** (p<0.01), ***(p<0.001),<br>V.C-1 : 4% tween80,<br>V.C-2 : 5% ethanol + 25% cremophor + 75% PBS | | | | | | | | |

As shown in Table 2, and FIG. 1, 3 and 5, the size and the weight of a tumor of NCI-H460 xenografted BALB/c nude mouse were remarkably decreased when it was administered with tricyclic derivatives of the present invention (prepared in Example 8 and Example 12), comparing to when being administered with a solvent only (V.C-1, V.C-2) or with a positive control (taxol, adriamycin). In particular, in the case of the compound of the Example 12, the volume and the weight of a tumor were much decreased with dosage of 10 mg/kg than with the dosage of 1 mg/kg. And in the case of the compound of the Example 8, inhibition rate of the tumor volume showed 85% when it was administered with 10 mg/kg. Therefore, decreasing rate of the volume and the weight of a tumor can appreciate in proportion to dosage of tricyclic derivatives of the present invention.

As shown in Table 3 and in FIG. 2 and 4, the weight of NCI-H460 xenografted BALB/c nude mouse was decreased about 10% when it was administered with tricyclic of the present invention (prepared in Example 8 and Example 12), comparing to when being administered with a solvent only (V.C-1, V.C-2) or with a positive control (taxol, adriamycin).

Therefore, tricyclic derivatives of the present invention make the volume and the weight of a tumor smaller and lighter dose-dependently, and also show excellent anticancer effect. So, tricyclic derivatives of the invention can be effectively used as an anticancer agent and as a anti-proliferation agent.

Experimental Example 3: Inhibitory affect of tricyclic derivatives of the present invention on capillary-like tube formation in HUVEC cells (Capillary-like tube formation assay)

Matrigel used in this experiment was a product of BioCoat. Matrigel was thawed in a refrigerator for 24 hours before use. Thawing matrigel, 96 well plate and yellow tip were put on ice. Then, matrigel was distributed into each well of the plate by 40 μℓ. The polymerization of the plate was performed in a 37°C incubator for 30 minutes. Each well of the plate was inoculated with 180 μℓ of HUVEC cell solution ($2\times10^4$ cells/mℓ) along with 20 μℓ of the compound of the Example 12 in serum-free media (0.3, 1, 3, 10 and 30 μg /mℓ) , followed by further culture for 24 hours.

Tube formation was observed under a microscope to investigate inhibition activity of angiogenesis.

Fumagilin and doxorubicin were used as a positive control.

The results were shown in FIG. 6.

As shown in FIG. 6, tricyclic derivatives of the present invention (prepared in the Example 12) had angiogenesis inhibition activity with dosage over 0.3 μg/mℓ, which was as good effect as that of fumagilin, a positive control, with the dosage of 10 μg/mℓ. Further, tricyclic derivatives of the invention (prepared in the Example 12) totally inhibited angiogenesis with the dosage over 10 μg/mℓ.

Thus, tricyclic derivatives of the present invention can be effectively used as an angiogenesis inhibitor.

Experimental Example 4: Acute toxicity test

5-week-old ICR mice having the weight of 25-35 g (SPF, SLC Co. Japan) were used as test animals. A pair of female and male of the mice was given for the test of each compound. Group T2, T3, T4 and T5 were arranged (10 animals per group) for the test of acute toxicity of the compound prepared in the Example 12. The compound of the Example 12 was dissolved in a solvent [5% DMSO; 20% tween 80; 75% PBS(-)], which was injected into the abdominal

cavity of the mouse (dosage was shown in Table 4), followed by observation for 7 days. Control group (TI) was administered with only a solvent without the compound of the Example 12. And the results were shown in Table 4.

For comparison, colchicine was injected in the test animals by the same method as described in the above. The group treated with colchicine was composed of 6 mice. The test results were shown in Table 5.

Taxol produced by Bristol Myers Sqibb Co. was also used for comparison, and the injection and the test of acute toxicity were performed by the same method as described in the above. The results were shown in Table 6.

<Table 4>

| Acute toxicity test with a compound prepared in Example 12 | | | | | | | | | | |
|---|---|---|---|---|---|---|---|---|---|---|
| Group | Animal Number | Dosage (mg/kg) | Test Day (Death) | | | | | | | |
| | | | 1 | 2 | 3 | 4 | 5 | 6 | 7 | Total |
| T1 | 10 | Solvent | - | - | - | - | - | - | - | 0/10 |
| T2 | 10 | 30 | - | - | - | - | - | - | - | 0/10 |
| T3 | 10 | 40 | - | - | - | - | - | - | - | 0/10 |
| T4 | 10 | 50 | - | - | - | - | - | - | - | 0/10 |
| T5 | 10 | 60 | - | - | - | - | - | - | - | 0/10 |

<Table 5>

| Acute toxicity test with colchicine | | | | | | | | | | |
|---|---|---|---|---|---|---|---|---|---|---|
| Group | Animal Number | Dosage (mg/kg) | Test Day (Death) | | | | | | | |
| | | | 1 | 2 | 3 | 4 | 5 | 6 | 7 | Total |
| T1 | 6 | Solvent | - | - | - | - | - | - | - | 0/6 |
| T2 | 6 | 0.5 | - | - | - | - | - | - | - | 0/6 |
| T3 | 6 | 1.0 | - | - | - | - | - | - | - | 0/6 |
| T4 | 6 | 2.0 | - | - | 1 | - | - | 2 | - | 3/6 |
| T5 | 5 | 5.0 | - | 3 | - | 1 | 1 | - | - | 5/5 |

<Table 6>

| Acute toxicity test with Taxol | | | | | | | | | | |
|---|---|---|---|---|---|---|---|---|---|---|
| Group | Animal Number | Dosage (mg/kg) | Test Day (Death) | | | | | | | |
| | | | 1 | 2 | 3 | 4 | 5 | 6 | 7 | Total |
| T1 | 5 | 4.0 | - | - | - | - | - | - | - | 0/5 |
| T2 | 5 | 6.0 | - | 1 | - | - | - | - | - | 1/5 |
| T3 | 5 | 9.0 | - | - | - | - | 2 | - | - | 2/5 |
| T4 | 5 | 13.0 | 2 | 2 | - | - | - | - | - | 4/5 |
| T5 | 5 | 20.0 | 5 | - | - | - | - | - | - | 5/5 |

As shown in Table 4, 5 and 6, toxicity of natural colchicine to a mouse was confirmed to be $LD_{50}$ = 2mg/kg, which was very similar to earlier reported value of $LD_{50}$ = 1.6mg/kg [Medicinal Research Reviews, Vol.8, No.1, 77-94 (1988)]. Toxicity of taxol injection, paclitaxel, was $LD_{50}$ = 9 - 13 mg/kg (i.v. administration). But toxicity of the compound of the Example 12 of the present invention was $LD_{50}$ = 60mg/kg, which was 30 times as week toxicity as that of colchicine and also weaker than that of taxol injection. Thus, the compound of the present invention was proved to have less toxicity to normal cells than colchicine or Taxol injection.

INDUSTRIAL APPLICABILITY

Tricyclic derivatives of the present invention have very strong cytotoxicity to cancer cell lines but less toxicity to animals themselves than colchicine or Taxol injection has. Tricyclic derivatives of the invention further decrease the volume and the weight of a tumor and inhibit angiogenesis in HUVEC cells excellently. Therefore, the derivatives can be effectively used as an anticancer agent, anti-proliferation agent and an angiogenesis inhibitor as well. In addition, tricyclic derivatives of the present invention can be obtained with ease and be formulated easily for oral administration or for injection owing to its water-solubility.

## Claims

**1.** A tricyclic derivative represented by the following <Formula 1> or pharmaceutically acceptable salts thereof.

<Formula 1>

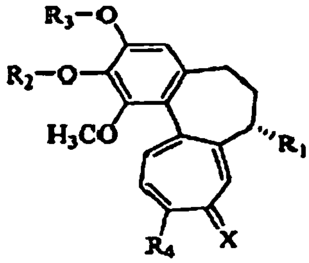

wherein,

(1) $R_1$ is $-T_1-B_1$;
in which $T_1$ is -X-, $-X_1-C(X_2)-$, $-N(R_5)-$, $-N(R_5)C(X_2)-$, - $N(R_5)S(O)n_1-$, $-N(R_5)C(O)-X_1-$ or $-N(R_5)C(X_1)NH-$, in that $X_1$ and $X_2$ are each O or S, $R_5$ is each H or $C_1$~$C_5$ alkyl group, $n_1$ is an integer of 1~2; and $B_1$ is selected from a group consisting of

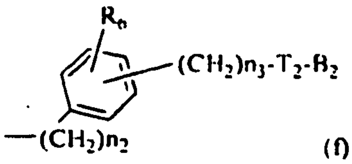

(f)

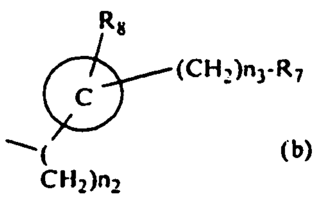

(b)

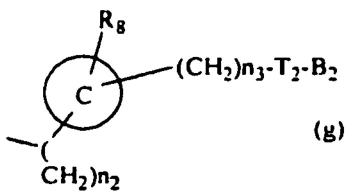

(g)

$-Z_1-T_2-B_2$ (h)

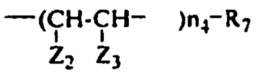 (d) 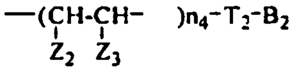 (i)

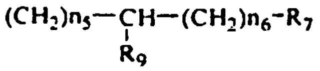 (e) 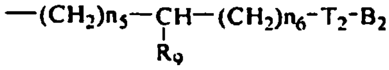 (j)

wherein, $R_6$ and $R_8$ are each H, halogen, hydroxy, $C_1$~$C_3$ alkoxy, amino, nitro, cyano or $C_1$~$C_3$ lower alkyl group; $R_7$ and $R_9$ are each independently halogen, hydroxy, mercapto, -ONO, -$ONO_2$ or SNO, in which $R_7$ and $R_9$ are same or different;

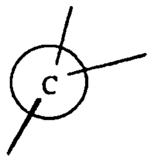

is $C_5$~$C_6$ membered saturated or unsaturated heterocyclic ring containing 1~2 of hetero atom, in which the hetero atom is selected from a group consisting of O, S and N, preferably,

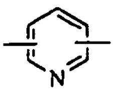 C1 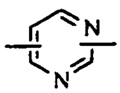 C2 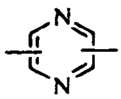 C3 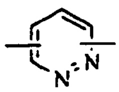 C4 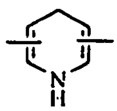 C5 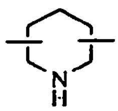 C6

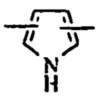 C7 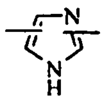 C8 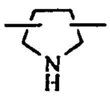 C9 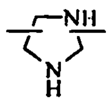 C10 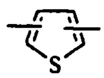 C11 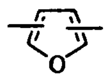 C12 ,

more preferably, C1 (pyridyl group) substituted at position 2 and 6 or position 2 and 5, C7 (pyrrolyl group) substituted at position 2 and 5 or position 2 and 4, C11 (thiophenyl group) or C12 (furanyl group); $Z_1$ is $C_1$~$C_{10}$ straight-chain or branched-chain alkyl group, preferably $C_2$~$C_5$ straight-chain or branched-chain alkyl group or cycloalkyl group having substituent; $Z_2$ and $Z_3$ are each independently H or methyl group, in which $Z_3$ is H when $Z_2$ is methyl group, $Z_2$ is H when $Z_3$ is methyl group; $T_2$ is -$X_1$- or -$X_1$-C($X_2$)-, in that $X_1$ and $X_2$ are each independently O or S; $B_2$ is selected from a group consisting of said

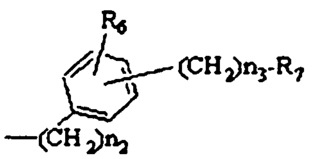 ,

(b), $-Z_1-R_7$, (d) or (e) ; $n_2$ is an integer of 0~3, $n_3$ is an integer of 0~5, $n_4$ is an integer of 1~5, $n_5$ and $n_6$ are each independently an integer of 1~6;

(2) $R_2$ and $R_3$ are each independently H, $-PO_3H_2$, phosphonate, sulfate, $C_3$~$C_7$ cycloalkyl, $C_2$~$C_7$ alkenyl, $C_2$~$C_7$ alkynyl, $C_1$~$C_7$ alkanoyl, $C_1$~$C_7$ straight-chain or branched-chain alkyl or sugar, in which sugar is a monosaccharide such as glucuronyl, glucosyl or galactosyl;

(3) $R_4$ is $OCH_3$, $SCH_3$ or $NR_{10}R_{11}$, in which $R_{10}$ and $R_{11}$ are each independently H or $C_{1\sim5}$ alkyl;

(4) X is O or S.

**2.** A tricyclic derivative represented by the following <Formula 1> or pharmaceutically acceptable salts thereof.

<Formula 1>

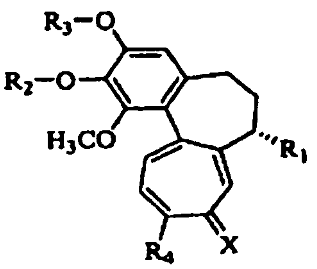

wherein,

(1) $R_1$ is $-T_1-B_1$;

in which $T_1$ is $-X_1-$, $-X_1-C(X_2)-$, $-N(R_5)-$, $-N(R_5)C(X_2)-$, $-N(R_5)S(O)n_1-$, $-N(R_5)C(O)-X_1-$ or $-N(R_5)C(X_1)NH-$, in that $X_1$ and $X_2$ are each O or S, $R_5$ is each H or $C_1$~$C_5$ alkyl group, $n_1$ is an integer of 1~2; and $B_1$ is selected from a group consisting of

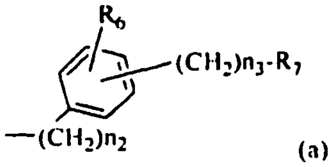
(a)

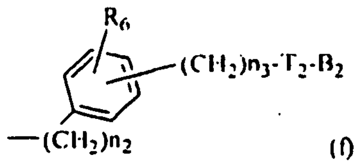
(f)

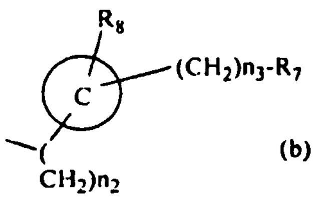
(b)

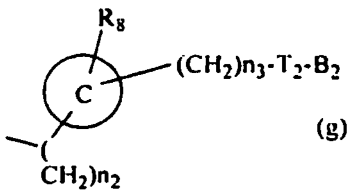
(g)

$-Z_1-T_2-B_2$ (h)

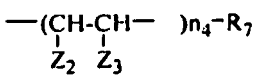
(d)

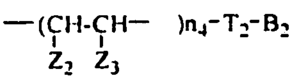
(i)

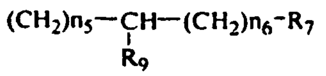 (e) 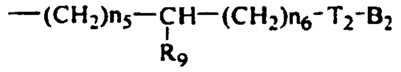 (j)

wherein, $R_6$ is halogen, hydroxy, $C_1$~$C_3$ alkoxy, amino, nitro, cyano or $C_1$~$C_3$ lower alkyl group; $R_8$ is H, halogen, hydroxy, $C_1$~$C_3$ alkoxy, amino, nitro, cyano or $C_1$~$C_3$ lower alkyl group; $R_7$ and $R_9$ are each independently halogen, hydroxy, mercapto, -ONO, -$ONO_2$ or SNO, in which $R_7$ and $R_9$ are same or different;

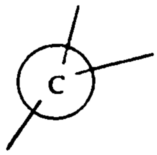

is $C_5$~$C_6$ membered saturated or unsaturated heterocyclic ring containing 1~2 of hetero atom, in which the hetero atom is selected from a group consisting of O, S and N, preferably,

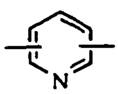 C1 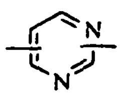 C2 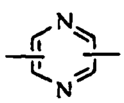 C3 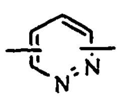 C4 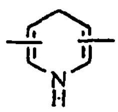 C5 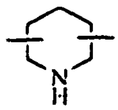 C6

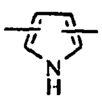 C7 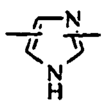 C8 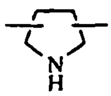 C9 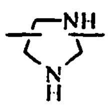 C10 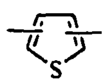 C11 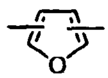 C12 ,

more preferably, C1 (pyridyl group) substituted at position 2 and 6 or position 2 and 5, C7 (pyrrolyl group) substituted at position 2 and 5 or position 2 and 4, C11 (thiophenyl group) or C12 (furanyl group); $Z_1$ is $C_1$~$C_{10}$ straight-chain or branched-chain alkyl group, preferably $C_2$~$C_5$ straight-chain or branched-chain alkyl group or cycloalkyl group having substituent; $Z_2$ and $Z_3$ are each independently H or methyl group, in which $Z_3$ is H when $Z_2$ is methyl group, $Z_2$ is H when $Z_3$ is methyl group; $T_2$ is -$X_1$- or -$X_1$-C($X_2$)-, in that $X_1$ and $X_2$ are each independently O or S; $B_2$ is selected from a group consisting of said (a), (b), -$Z_1$-$R_7$, (d) or (e); $n_2$ is an integer of 0~3, $n_3$ is an integer of 0~5, $n_4$ is an integer of 1~5, $n_5$ and $n_6$ are each independently an integer of 1~6;

(2) $R_2$ and $R_3$ are each independently H, -$PO_3H_2$, phosphonate, sulfate, $C_3$~$C_7$ cycloalkyl, $C_2$~$C_7$ alkenyl, $C_2$~$C_7$ alkynyl, $C_1$~$C_7$ alkanoyl, $C_1$~$C_7$ straight-chain or branched-chain alkyl or sugar, in which sugar is a monosaccharide such as glucuronyl, glucosyl or galactosyl;

(3) $R_4$ is $OCH_3$, $SCH_3$ or $NR_{10}R_{11}$, in which $R_{10}$ and $R_{11}$ are each independently H or $C_{1\text{-}5}$ alkyl;

(4) X is O or S.

3. The tricyclic derivative or pharmaceutically acceptable salts thereof as set forth in claim 1, wherein the compound of <Formula 1> is **characterized** as follows:

(1) $R_1$ is -$T_1$-$B_1$;

in which $T_1$ is -N($R_5$)C($X_2$)-, -N($R_5$)C(O)-$X_1$- or -N($R_5$)C($X_1$)NH-, in that $X_1$ and $X_2$ are each O, $R_5$ is each H or $C_1$~$C_5$ alkyl group; and $B_1$ is selected from a group consisting of

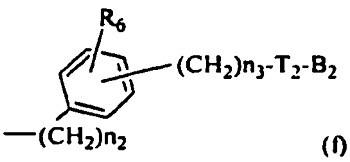

(f)

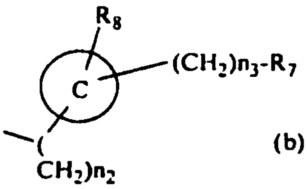

(b)

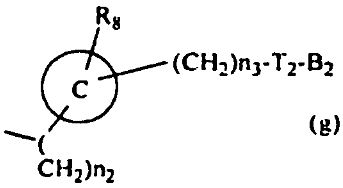

(g)

$-Z_1-T_2-B_2$ (h)

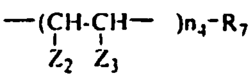

(d)

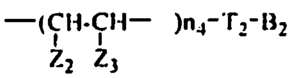

(i)

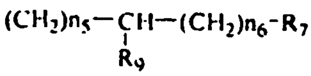

(e)

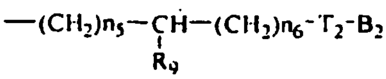

(j)

wherein, $R_6$ and $R_8$ are each H, halogen, hydroxyl, $C_1$~$C_3$ alkoxy, amino, nitro, cyano or $C_1$~$C_3$ lower alkyl group; $R_7$ and $R_9$ are each independently halogen, hydroxyl, mercapto(thiol), -ONO, $-ONO_2$ or SNO, in which $R_7$ and $R_9$ are same or different;

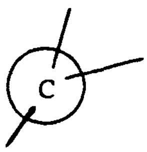

is $C_5$~$C_6$ membered saturated or unsaturated heterocyclic ring containing 1~2 of hetero atom, in which the hetero atom is selected from a group consisting of O S and N, preferably,

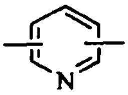

C1

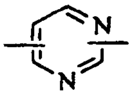

C2

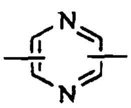

C3

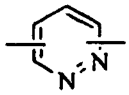

C4

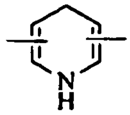

C5

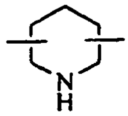

C6

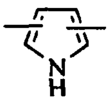 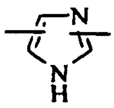 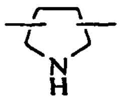 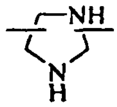 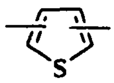 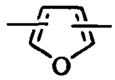

C7 C8 C9 C10 C11 C12 ,

more preferably, C1 (pyridyl group) substituted at position 2 and 6 or position 2 and 5, C7 (pyrrolyl group) substituted at position 2 and 5 or position 2 and 4, C11 (thiophenyl group) or C12 (furanyl group), a bond of substituents may be at symmetrical or asymmetrical position; $Z_1$ is $C_1$~$C_{10}$ straight-chain or branched-chain alkyl group, preferably $C_2$~$C_5$ straight-chain or branched-chain alkyl group or cycloalkyl group having substituent; $Z_2$ and $Z_3$ are each independently H or methyl group, in which $Z_3$ is H when $Z_2$ is methyl group, $Z_2$ is H when $Z_3$ is methyl group; $T_2$ is $-X_1-$ or $-X_1-C(X_2)-$, in that $X_1$ and $X_2$ are each 0 or S; $B_2$ is selected from a group consisting of said

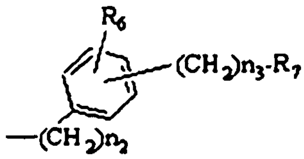

,

(b), $-Z_1-R_7$, (d) or (e); $n_2$ is an integer of 0~3, $n_3$ is an integer of 0~5, $n_4$ is an integer of 1~3, $n_5$ and $n_6$ are each independently an integer of 1~3;

(2) $R_2$ and $R_3$ are each independently $C_3$~$C_7$ cycloalkyl or $C_1$~$C_7$ alkyl;

(3) $R_4$ is $SCH_3$ or $OCH_3$;

(4) X is O or S.

**4.** The tricyclic derivative or pharmaceutically acceptable salts thereof as set forth in claim 2, wherein the compound of <Formula 1> is **characterized** as follows:

(1) $R_1$ is $-T_1-B_1$;
in which $T_1$ is $-N(R_5)C(X_2)-$, $-N(R_5)C(O)-X_1-$ or $-N(R_5)C(X_1)NH-$, in that $X_1$ and $X_2$ are each O, $R_5$ is each H or $C_1$~$C_5$ alkyl group; and $B_1$ is selected from a group consisting of

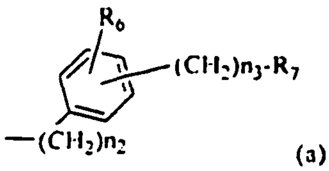
(a)

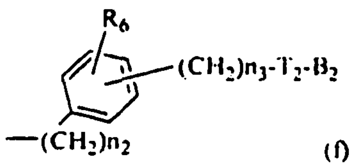
(f)

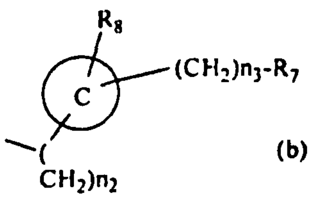
(b)

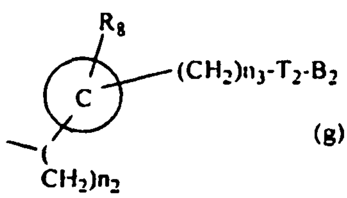
(g)

$-Z_1-T_2-B_2$ (h)

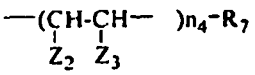 (d)

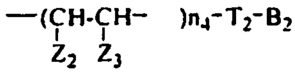 (i)

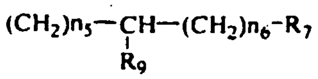 (e)

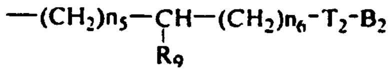 (j)

wherein, $R_6$ is halogen, hydroxy, $C_1$~$C_3$ alkoxy, amino, nitro, cyano or $C_1$~$C_3$ lower alkyl group; $R_8$ is H, halogen, hydroxy, $C_1$~$C_3$ alkoxy, amino, nitro, cyano or $C_1$~$C_3$ lower alkyl group; $R_7$ and $R_9$ are each independently halogen, hydroxy, mercapto, -ONO, -$ONO_2$ or SNO, in which $R_7$ and $R_9$ are same or different;

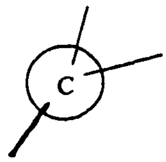

is $C_5$~$C_6$ membered saturated or unsaturated heterocyclic ring containing 1~2 of hetero atom, in which the hetero atom is selected from a group consisting of O, S and N, preferably,

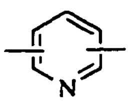 C1 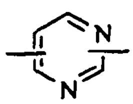 C2 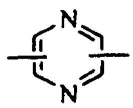 C3 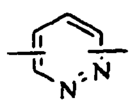 C4 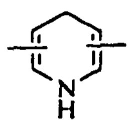 C5 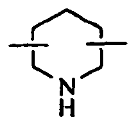 C6

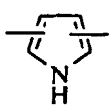 C7 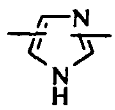 C8 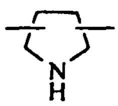 C9 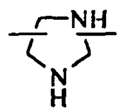 C10 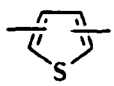 C11 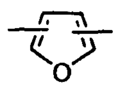 C12 ,

more preferably, C1 (pyridyl group) substituted at position 2 and 6 or position 2 and 5, C7 (pyrrolyl group) substituted at position 2 and 5 or position 2 and 4, C11 (thiophenyl group) or C12 (furanyl group), a bond of substituents may be at symmetrical or asymmetrical position; $Z_1$ is $C_1$~$C_{10}$ straight-chain or branched-chain alkyl group, preferably $C_2$~$C_5$ straight-chain or branched-chain alkyl group or cycloalkyl group having substituent; $Z_2$ and $Z_3$ are each independently H or methyl group, in which $Z_3$ is H when $Z_2$ is methyl group, $Z_2$ is H when $Z_3$ is methyl group; $T_2$ is -$X_1$- or -$X_1$-C($X_2$)-, in that $X_1$ and $X_2$ are each O or S; $B_2$ is selected from a group consisting of said (a), (b), -$Z_1$-$R_7$, (d) or (e); $n_2$ is an integer of 0~3, $n_3$ is an integer of 0~5, $n_4$ is an integer of 1~5, $n_5$ and $n_6$ are each independently an integer of 1~6;

(2) $R_2$ and $R_3$ are each independently $C_3$~$C_7$ cycloalkyl or $C_1$~$C_7$ alkyl;

(3) $R_4$ is $SCH_3$ or $OCH_3$;

(4) X is O or S.

**5.** The tricyclic derivative or pharmaceutically acceptable salts thereof as set forth in any one of claims 1 to 4, wherein the tricyclic derivative comprises:

1) 6-nitrooxymethyl-N-[(75)-1,2,3-trimethoxy-10-methylsulfanyl-9-oxo-5,6,7,9-tetrahydro-benzo[a]heptalen -7-yl]-, nicotineamide;
2) 5-nitrooxymethyl-furan-2-carboxylic acid-[(7S)-1,2,3-trimethoxy-10-methylsulfanyl-9-oxo-5,6,7,9-tetrahydro-benzo[a]heptalen-7-yl]-amide;
5) 6-nitrooxymethyl-pyridine-2-carboxylic acid-[(7S)-1,2,3-trimethoxy-10-methylsulfanyl-9-oxo-5,6,7,9-tetrahydro-benzo[a]heptalen-7-yl]-amide;
6) 5-nitrooxymethyl-thiophene-2-carboxylic acid-[(7S)-1,2,3-trimethoxy-10-methylsulfanyl-9-oxo-5,6,7,9-tetrahydro-benzo[a]heptalen-7-yl]-amide;
8) N-[(7S)-3-ethoxy-1,2-dimethoxy-10-methylsulfanyl-9-oxo-5,6,7,9-tetrahydro-benzo[a]heptalen-7-yl]-2-fluoro-3-nitrooxymethyl-benzamide;
9) 2-fluoro-N-[(7S)-3-isopropoxy-1,2-dimethoxy-10-methylsulfanyl-9-oxo-5,6,7,9-tetrahydro-benzo[a]heptalen-7-yl]-3-nitrooxymethyl-benzamide;
10) 2-fluoro-3-nitrooxymethyl-N-[(7S)-1,2,3-trimethoxy-10-methylsulfanyl-9-oxo-5,6,7,9-tetrahydro-benzo[a]heptalen-7-yl]-benzamide;
11) N-[(7S)-3-cyclopentyloxy-1,2-dimethoxy-10-methylsulfanyl-9-oxo-5,6,7,9-tetrahydro-benzo[a]heptalen-7-yl]-2-fluoro-3-nitrooxymethyl-benzamide;
12) 3-fluoro-5-nitrooxymethyl-N-[(7S)-1,2,3-trimethoxy-10-methylsulfanyl-9-oxo-5,6,7,9-tetrahydro-benzo[a]heptalen-7-yl]-benzamide;
13) N-[(7S)-3-ethoxy-1,2-dimethoxy-10-methylsulfanyl-9-oxo-5,6,7,9-tetrahydro-benzo[a]heptalen-7-yl]-3-fluoro-5-nitrooxymethyl-benzamide;
14) 3-fluoro-N-[(7S)-3-isopropoxy-1,2-dimethoxy-10-methylsulfanyl-9-oxo-5,6,7,9-tetrahydro-benzo[a]heptalen-7-yl]-5-nitrooxymethyl-benzamide;
15) N-[(7S)-3-cyclopentyloxy-1,2-dimethoxy-10-methylsulfanyl-9-oxo-5,6,7,9-tetrahydro-benzo[a]heptalen-7-yl]-3-fluoro-5-nitrooxymethyl-benzamide;
16) 4-fluoro-3-nitrooxymethyl-N-[(7S)-1,2,3-trimethoxy-10-methylsulfanyl-9-oxo-5,6,7,9-tetrahydro-benzo[a]heptalen-7-yl]-benzamide;
17) 2-fluoro-5-nitrooxymethyl-N-[(7S)-1,2,3-trimethoxy-10-methylsulfanyl-9-oxo-5,6,7,9-tetrahydro-benzo[a]heptalen-7-yl]-benzamide;
18) 3-hydroxy-5-nitrooxymethyl-N-[(7S)-1,2,3-trimethoxy-10-methylsulfanyl-9-oxo-5,6,7,9-tetrahydro-benzo[a]heptalen-7-yl]-benzamide;
20) 2-hydroxy-4-nitrooxymethyl-N-[(7S)-1,2,3-trimethoxy-10-methylsulfanyl-9-oxo-5,6,7,9-tetrahydro-benzo[a]heptalen-7-yl]-benzamide;
21) 4-nitrooxymethyl-thiophene-2-carboxylic acid [(7S)-1,2,3-trimethoxy-10-methylsulfanyl-9-oxo-5,6,7,9-tetrahydro-benzo[a]heptalen-7-yl]-amide;
22) 3-nitrooxymethyl-thiophene-2-carboxylic acid [(7S)-1,2,3-trimethoxy-10-methylsulfanyl-9-oxo-5,6,7,9-tetrahydro-benzo[a]heptalen-7-yl]-amide;
25) 3-nitrooxybenzoic acid-5-[(7S)-1,2,3-trimethoxy-10-methylsulfanyl-9-oxo-5,6,7,9-tetrahydro-benzo[a]heptalen-7-yl-carbamoyl]-pyridine-2-yl-methylester;
26) 4-nitrooxybutyric acid-5-[(7S)-1,2,3-trimethoxy-10-methylsulfanyl-9-oxo-5,6,7,9-tetrahydro-benzo[a]heptalen-7-yl-carbamoyl]-pyridine-2-yl-methylester;
27) 3-nitrooxymethyl-benzoic acid-6-[(7S)-1,2,3-trimethoxy-10-methylsulfanyl-9-oxo-5,6,7,9-tetrahydro-benzo[a]heptalen-7-yl-carbarnoyl]-pyridine-2-yl-methylester;
28) 4-nitrooxybutyric acid-6-[(7S)-1,2,3-trimethoxy-10-methylsulfanyl-9-oxo-5,6,7,9-tetrahydro-benzo[a]heptalen-7-yl-carbamoyl]-pyridine-2-yl-methylester;
29) 3-nitrooxymethyl-benzoic acid-2-[(7S)-1,2,3-trimethoxy-10-methylsulfanyl-9-oxo-5,6,7,9-tetrahydro-benzo[a]heptalen-7-yl-carbamoyl]-phenylester;
30) 4-nitrooxybutyric acid-2-[(7S)-1,2,3-trimethoxy-10-methylsulfanyl-9-oxo-5,6,7,9-tetrahydro-benzo[a]heptalen-7-yl-carbamoyl]-phenylester;
31) 3-nitrooxymethyl-benzoic acid-3-[(7S)-1,2,3-trimethoxy-10-methylsulfanyl-9-oxo-5,6,7,9-tetrahydro-benzo[a]heptalen-7-yl-carbamoyl]-phenylester;
32) 4-nitrooxybutyric acid-3-[(7S)-1,2,3-trimethoxy-10-methylsulfanyl-9-oxo-5,6,7,9-tetrahydro-benzo[a]heptalen-7-yl-carbamoyl]-phenylester;
33) 3-nitrooxymethyl-benzoic acid-3-[(7S)-1,2,3-trimethoxy-10-methylsulfanyl-9-oxo-5,6,7,9-tetrahydro-benzo[a]heptalen-7-yl-carbamoyl]-benzylester;
34) 4-nitrooxybutyric acid-3-[(7S)-1,2,3-trimethoxy-10-methylsulfanyl-9-oxo-5,6,7,9-tetrahydro-benzo[a]heptalen-7-yl-carbamoyl]-benzylester;
37) 3-fluoro-5-nitrosooxymethyl-N-[(7S)-1,2,3-trimethoxy-10-methylsulfanyl-9-oxo-5,6,7,9-tetrahydro-benzo[a]heptalen-7-yl]-benzamide;
39) 3-fluoro-5-nitrosothiomethyl-N-[(7S)-1,2,3-trimethoxy-10-methylsulfanyl-9-oxo-5,6,7,9-tetrahydro-benzo[a]heptalen-7-yl]-benzamide;
40) 3-fluoro-5-nitrooxymethyl-N-[(7S)-1,2,3,10-tetramethoxy-9-oxo-5,6,7,9-tetrahydro-benzo[a]heptalen-7-yl]-benzamide;
42) 3-fluoro-N-methyl-5-nitrooxymethyl-N-(7S)-1,2,3-trimethoxy-10-methylsulfanyl-9-oxo-5,6,7,9-tetrahydro-benzo[a]heptalen-7-yl]-benzamide;
43) 2-(3-fluoro-5-nitrooxymethyl-phenyl)-N-[(7S)-1,2,3-trimethoxy-10-methylsulfanyl-9-oxo-5,6,7,9-tetrahydro-benzo[a]heptalen-7-yl]-acetamide: or
44) 2-(2-fluoro-5-nitrooxymethyl-phenyl)-N-[(7S)-1,2,3-trimethoxy-10-methylsulfanyl-9-oxo-5,6,7,9-tetrahydro-benzo[a]heptalen-7-yl]-acetamide.

**6.** A method for preparing tricyclic derivatives as represented in <Scheme 1> comprising the following steps:

(1) Reaction of the compound of formula (III) with the compound formula (IV) or the compound of formula (VI) is performed to give the compound of formula (V) or the compound of formula (VII), or reaction of the resultant compound of formula (VII) with the halogen compound is performed to give the compound of formula (V) (Step 1); and
(2) Nitration or nitrosation of the prepared compound of formula (V) or the compound of formula (VII) is performed to give the compound of formula (IIa) (Step 2).

<Scheme 1>

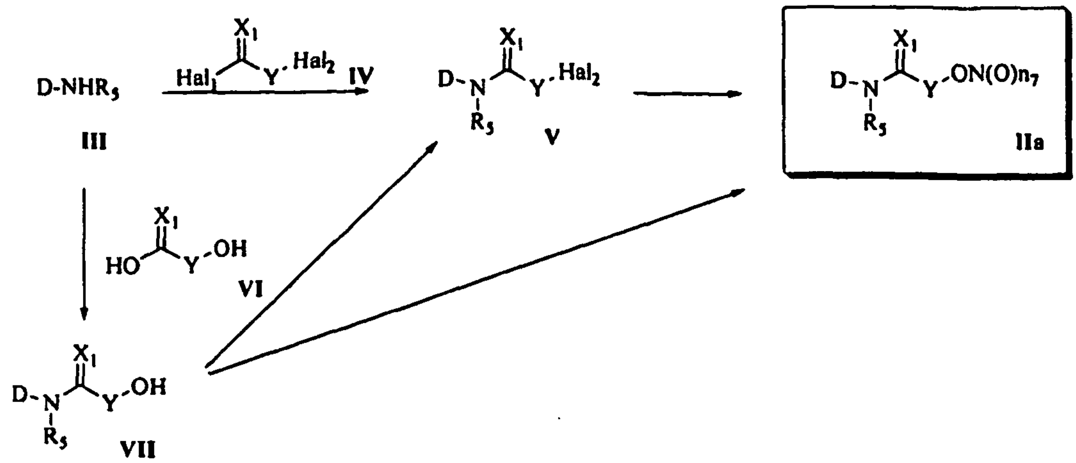

wherein, D is

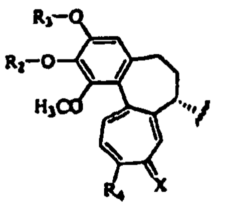

,

and $R_2$, $R_3$, $R_4$ and X are same as defined in any one of claims 1 to 4;

$R_5$ is H or low molecular weight alkyl; $X_1$ is O or S; $Hal_1$ and $Hal_2$ are halogens; $Hal_1$ and $Hal_2$ of general formula (IV) and (V) are each same or different halogens, for example F, Cl, Br or I; Y indicates general formula (a'), and (b'), (c'), (d') and (e') respectively,

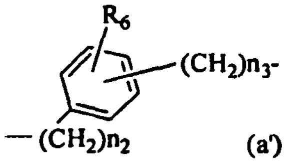
(a')

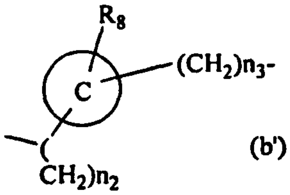
(b')

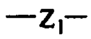
(c')

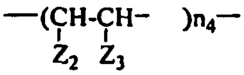
(d')

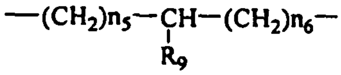
(e')

wherein,

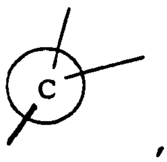

$R_6$, $R_8$, $R_9$, $Z_1$, $Z_2$, $Z_3$, $n_2$, $n_3$, $n_4$, $n_5$ and $n_6$ are same as defined in any one of claims 1 to 4.

7. An anticancer agent or anti-proliferation agent containing tricyclic derivatives of any one of claims 1 to 5 or pharmaceutically acceptable salts thereof as an effective ingredient.

8. An angiogenesis inhibitor containing tricyclic derivatives of any one of claims 1 to 5 or pharmaceutically acceptable salts thereof as an effective ingredient.

9. Use of a compound of the tricyclic derivative of any one of claims 1 to 5 or pharmaceutically acceptable salts thereof for the preparation of a medicament for the treatment of cancer or diseases, which can be treated by an anti-proliferation agent.

10. Use of a compound of the tricyclic derivative of any one of claims 1 to 5 or pharmaceutically acceptable salts thereof for the preparation of a medicament for angiogenesis inhibition.

11. A compound of any one of claims 1 to 5 or a pharmaceutically acceptable salt thereof for the use as anticancer agent or anti-proliferation agent.

12. A compound of any one of claims 1 to 5 or a pharmaceutically acceptable salt thereof for the use as angiogenesis inhibitor.

**Patentansprüche**

1. Tricyclisches Derivat, dargestellt durch die folgende <Formel 1> oder pharmazeutisch verträgliche Salze davon.

<Formel 1>

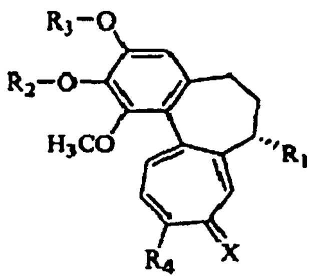

wobei

(1)$R_1$ $T_1$-$8_1$ ist;
in dem $T_1$ -$X_1$-, -$X_1$-C($X_2$)-, -N($R_5$)-, -N($R_5$)C($X_2$)-, -N($R_5$)S(O)$n_1$-, -N($R_5$)C(O)-$X_1$- oder - N($R_5$)C($X_1$)NH- ist, in dem $X_1$ und $X_2$ jeweils O oder S sind, $R_5$ jeweils H oder eine $C_1$-$C_5$ Alkylgruppe ist, $n_1$ eine ganze Zahl von 1-2 ist; und $B_1$ ausgewählt ist aus der Gruppe bestehend aus

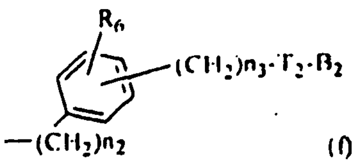

(f)

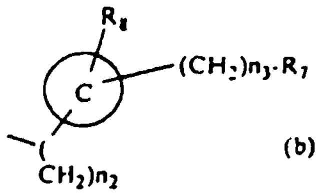

(b)

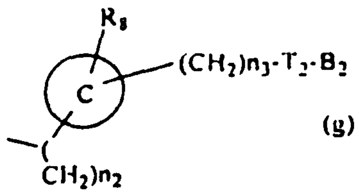

(g)

$-Z_1-T_2-B_2$ (h)

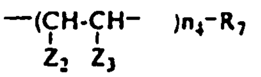

(d)

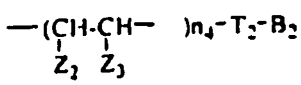

(i)

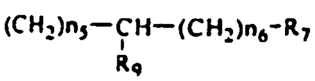

(e)

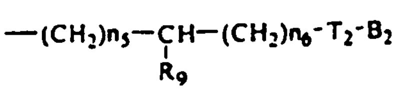

(j)

wobei $R_6$ und $R_8$ jeweils H, Halogen, Hydroxy, $C_1$-$C_3$ Alkoxy, Amino, Nitro, Cyano oder eine $C_1$-$C_3$ Niederalkylgruppe sind; $R_7$ und $R_9$ jeweils unabhängig Halogen, Hydroxy, Mercapto, -ONO, -$ONO_2$ oder SNO sind, wobei $R_7$ und $R_9$ gleich oder unterschiedlich sind;

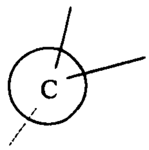

ist ein $C_5$-$C_6$ gliedr iger gesättigter oder ungesättigter heterocyclischer Ring, enthaltend 1-2 Heteroatome, in welchem das Heteroatom ausgewählt ist aus einer Gruppe bestehend aus O, S und N, vorzugsweise

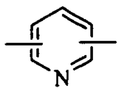 C1 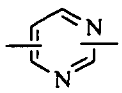 C2 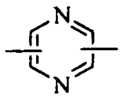 C3 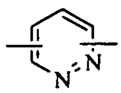 C4 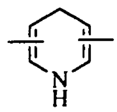 C5 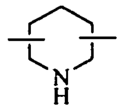 C6

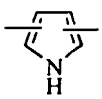 C7 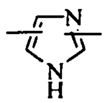 C8 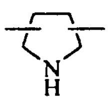 C9 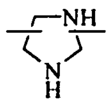 C10 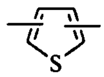 C11 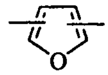 C12 ,

besonders bevorzugt C1 (Pyridylgruppe) substituiert an Position 2 und 6 oder Position 2 und 5, C7 (Pyrrolylgruppe) substituiert an Position 2 und 5 oder Position 2 und 4, C11 (Thiophenylgruppe) oder C12 (Furanylgruppe); $Z_1$ ist eine $C_1$-$C_{10}$ geradkettige oder verzweigtkettige Alkylgruppe, vorzugsweise eine $C_2$-$C_5$ geradkettige oder verzweigtkettige Alkylgruppe oder Cycloalkylgruppe mit Substituent; $Z_2$ und $Z_3$ sind jeweils unabhängig H oder eine Methylgruppe, in welcher $Z_3$ H ist, wenn $Z_2$ eine Methylgruppe ist, $Z_2$ H ist, wenn $Z_3$ eine Methylgruppe ist; $T_2$ ist $-X_1-$ oder $-X_1-C(X_2)-$, in dem $X_1$ und $X_2$ jeweils unabhängig O oder S sind; $B_2$ ist ausgewählt aus einer Gruppe bestehend aus den genannten

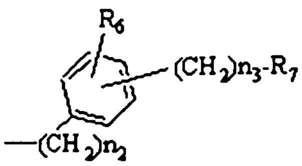

,

(b), $-Z_1-R_7$. (d) oder (e); $n_2$ ist eine ganze Zahl von 0-3, $n_3$ ist eine ganze Zahl von 0-5, $n_4$ ist eine ganze Zahl von 1-5, $n_5$ und $n_6$ sind jeweils unabhängig eine ganze Zahl von 1-6;

(2) $R_2$ und $R_3$ sind jeweils unabhängig H, $-PO_3H_2$, Phosphonat, Sulfat, $C_3$-$C_7$ Cycloalkyl, $C_2$-$C_7$ Alkenyl, $C_2$-$C_7$ Alkinyl, $C_1$-$C_7$ Alkanoyl, $C_1$-$C_7$ geradkettiges oder verzweigtkettiges Alkyl oder Zucker, in welchem der Zucker ein Monosaccharid wie Glucuronyl, Glucosyl oder Galactosyl ist;

(3) $R_4$ ist $OCH_3$, $SCH_3$ oder $NR_{10}R_{11}$, in welchem $R_{10}$ und $R_{11}$ jeweils unabhängig H oder $C_{1-5}$ Alkyl sind;

(4) X ist O oder S.

**2.** Tricyclisches Derivat, dargestellt durch die folgende <Formel 1> oder pharmazeutisch verträgliche Salze davon.

<Formel 1>

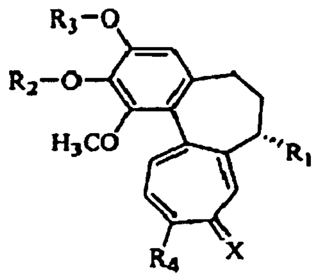

wobei,

(1)$R_1$ $T_1$-$B_1$ ist; in dem $T_1$ -$X_1$-, -$X_1$-C($X_2$)-, -N($R_5$)-, -N($R_5$)C($X_2$)-, -N($R_5$)S(O)$n_1$-, -N($R_5$)C(O)-$X_1$- oder - N($R_5$) C($X_1$)NH- ist, in dem $X_1$ und $X_2$ jeweils O oder S sind, $R_5$ jeweils H oder eine $C_1$-$C_5$ Alkylgruppe ist, $n_1$ eine ganze Zahl von 1-2 ist; und $B_1$ ausgewählt ist aus der Gruppe bestehend aus

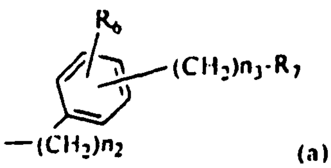

(a)

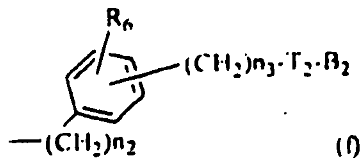

(f)

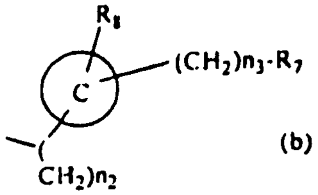

(b)

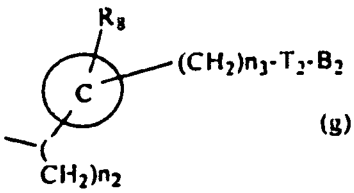

(g)

$-Z_1-T_2-B_2$ (h)

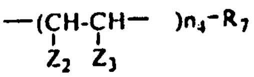

(d)

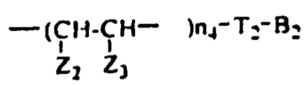

(i)

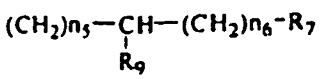

(e)

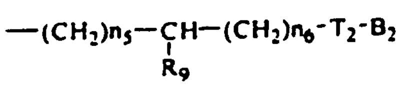

(j)

wobei $R_6$ Halogen, Hydroxy, $C_1$-$C_3$ Alkoxy, Amino, Nitro, Cyano oder eine $C_1$-$C_3$ Niederalkylgruppe ist; $R_8$ H, Halogen, Hydroxy, $C_1$-$C_3$ Alkoxy, Amino, Nitro, Cyano oder eine $C_1$-$C_3$ Niederalkylgruppe ist; $R_7$ und $R_9$ jeweils

unabhängig Halogen, Hydroxy, Mercapto, - ONO, $-ONO_2$ oder SNO sind, wobei $R_7$ und $R_9$ gleich oder unterschiedlich sind;

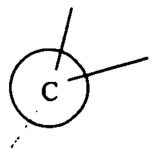

ist ein $C_5$-$C_6$ gliedriger gesättigter oder ungesättigter heterocyclischer Ring, enthaltend 1-2 Heteroatome, in welchem das Heteroatom ausgewählt ist aus einer Gruppe bestehend aus O, S und N, vorzugsweise

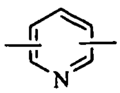 C1 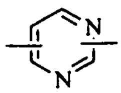 C2 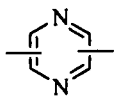 C3 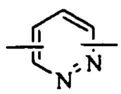 C4 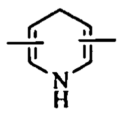 C5 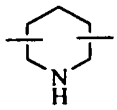 C6

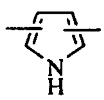 C7 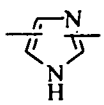 C8 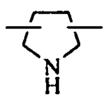 C9 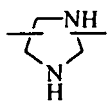 C10 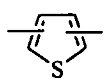 C11 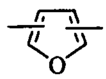 C12 ,

besonders bevorzugt C1 (Pyridylgruppe) substituiert an Position 2 und 6 oder Position 2 und 5, C7 (Pyrrolylgruppe) substituiert an Position 2 und 5 oder Position 2 und 4, C11 (Thiophenylgruppe) oder C12 (Furanylgruppe); $Z_1$ ist eine $C_1$-$C_{10}$ geradkettige oder verzweigtkettige Alkylgruppe, vorzugsweise eine $C_2$-$C_5$ geradkettige oder verzweigtkettige Alkylgruppe oder Cycloalkylgruppe mit Substituent; $Z_2$ und $Z_3$ sind jeweils unabhängig H oder eine Methylgruppe, in welcher $Z_3$ H ist, wenn $Z_2$ eine Methylgruppe ist, $Z_2$ H ist, wenn $Z_3$ eine Methylgruppe ist; $T_2$ ist $-X_1$- oder $-X_1-C(X_2)-$, in dem $X_1$ und $X_2$ jeweils unabhängig O oder S sind; $B_2$ ist ausgewählt aus einer Gruppe bestehend aus den genannten (a), (b), $-Z_1-R_7$, (d) oder (e); $n_2$ ist eine ganze Zahl von 0-3, $n_3$ ist eine ganze Zahl von 0-5, $n_4$ ist eine ganze Zahl von 1-5, $n_5$ und $n_6$ sind jeweils unabhängig eine ganze Zahl von 1-6;
(2) $R_2$ und $R_3$ sind jeweils unabhängig H, $-PO_3H_2$, Phosphonat, Sulfat, $C_3$-$C_7$ Cycloalkyl, $C_2$-$C_7$ Alkenyl, $C_2$-$C_7$ Alkinyl, $C_1$-$C_7$ Alkanoyl, $C_1$-$C_7$ geradkettiges oder verzweigtkettiges Alkyl oder Zucker, in welchem der Zucker ein Monosaccharid wie Glucuronyl, Glucosyl oder Galactosyl ist;
(3) $R_4$ ist $OCH_3$, $SCH_3$ oder $NR_{10}R_{11}$, in welchem $R_{10}$ und $R_{11}$ jeweils unabhängig H oder $C_{1-5}$ Alkyl sind;
(4) X ist O oder S.

**3.** Tricyclisches Derivat oder pharmazeutisch verträgliche Salze davon nach Anspruch 1, wobei die Verbindung der <Formel 1> wie folgt **gekennzeichnet** ist:

(1) $R_1$ ist-$T_1$-$B_1$;
wobei $T_1$ $-N(R_5)C(X_2)-$, $-N(R_5)C(O)-X_1-$ oder $-N(R_5)C(X_1)NH-$ ist, in dem $X_1$ und $X_2$ jeweils O sind, $R_5$ jeweils H oder eine $C_1$-$C_5$ Alkylgruppe ist; und $B_1$ ausgewählt ist aus einer Gruppe bestehend aus

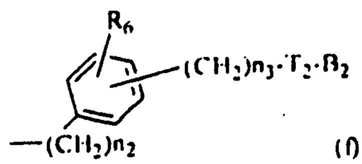
(f)

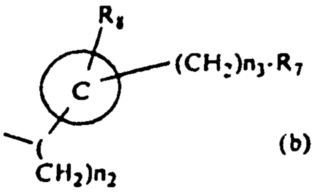

(b)

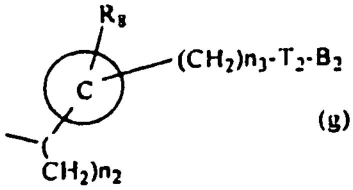

(g)

$-Z_1-T_2-B_2$ (h)

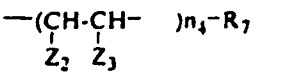

(d)

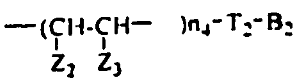

(i)

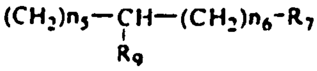

(e)

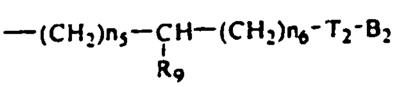

(j)

wobei $R_6$ und $R_8$ jeweils H, Halogen, Hydroxyl, $C_1$-$C_3$ Alkoxy, Amino, Nitro, Cyano oder eine $C_1$-$C_3$ Niederalkylgruppe sind; $R_7$ und $R_9$ jeweils unabhängig Halogen, Hydroxyl, Mercapto (Thiol), -ONO, -$ONO_2$ oder SNO sind, wobei $R_7$ und $R_9$ gleich oder unterschiedlich sind

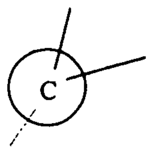

ist ein $C_5$-$C_6$ gliedriger gesättigter oder ungesättigter heterocyclischer Ring, enthaltend 1-2 Heteroatome, in welchem das Heteroatom ausgewählt ist aus einer Gruppe bestehend aus O, S und N, vorzugsweise

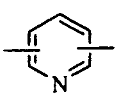
C1
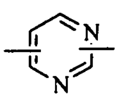
C2
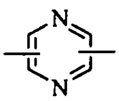
C3
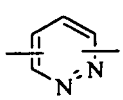
C4
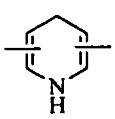
C5
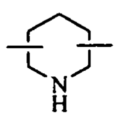
C6

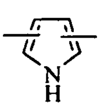
C7
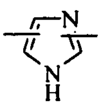
C8
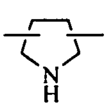
C9
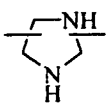
C10
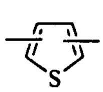
C11
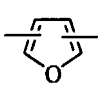
C12
,

besonders bevorzugt C1 (Pyridylgruppe) substituiert an Position 2 und 6 oder Position 2 und 5, C7 (Pyrrolylgruppe) substituiert an Position 2 und 5 oder Position 2 und 4, C11 (Thiophenylgruppe) oder C12 (Furanylgruppe), eine Bindung von Substituenten kann in einer symmetrischen oder asymmetrischen Position sein; $Z_1$ ist

eine $C_1$-$C_{10}$) geradkettige oder verzweigtkettige Alkylgruppe, vorzugsweise eine $C_2$-$C_5$ geradkettige oder verzweigtkettige Alkylgruppe oder Cycloalkylgruppe mit Substituent; $Z_2$ und $Z_3$ sind jeweils unabhängig H oder eine Methylgruppe, in welcher $Z_3$ H ist, wenn $Z_2$ eine Methylgruppe ist, $Z_2$ H ist, wenn $Z_3$ eine Methylgruppe ist; $T_2$ ist -$X_1$- oder -$X_1$-C($X_2$)-, in dem $X_1$ und $X_2$ jeweils O oder S sind; $B_2$ ist ausgewählt aus einer Gruppe bestehend aus den genannten

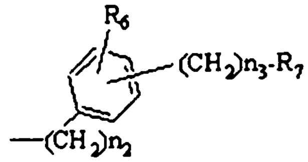,

(b), -$Z_1$-$R_7$, (d) oder (e); $n_2$ ist eine ganze Zahl von 0-3, $n_3$ ist eine ganze Zahl von 0-5, $n_4$ ist eine ganze Zahl von 1-3, $n_5$ und $n_6$ sind jeweils unabhängig eine ganze Zahl von 1-3;

(2) $R_2$ und $R_3$ sind jeweils unabhängig $C_3$-$C_7$ Cycloalkyl oder $C_1$-$C_7$ Alkyl;

(3) $R_4$ ist $SCH_3$ oder $OCH_3$;

(4) X ist O oder S.

**4.** Tricyclisches Derivat oder pharmazeutisch verträgliche Salze davon nach Anspruch 2, wobei die Verbindung der <Formel 1> wie folgt **gekennzeichnet** ist:

(1) $R_1$ ist -$T_1$-$B_1$;
wobei $T_1$ -N($R_5$)C($X_2$)-, -N($R_5$)C(O)-$X_1$- oder -N($R_5$)C($X_1$)NH- ist, in dem $X_1$ und $X_2$ jeweils O sind, $R_5$ jeweils H oder eine $C_1$-$C_5$ Alkylgruppe ist; und $B_1$ ausgewählt ist aus einer Gruppe bestehend aus

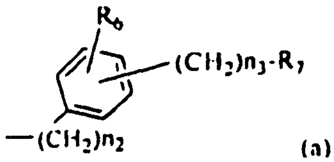 (a)

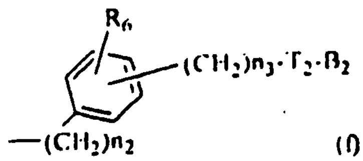 (f)

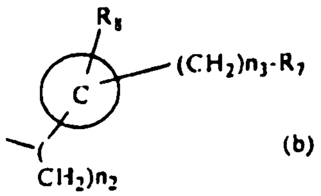 (b)

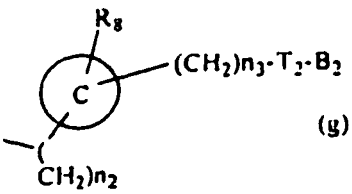 (g)

$—Z_1-T_2-B_2$ (h)

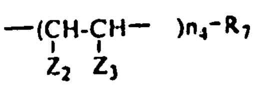 (d)

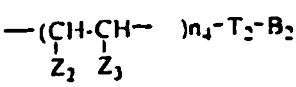 (i)

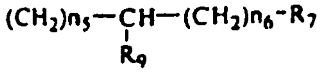 (e) 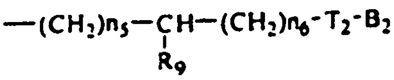 (j)

wobei $R_6$ Halogen, Hydroxy, $C_1$-$C_3$ Alkoxy, Amino, Nitro, Cyano oder eine $C_1$-$C_3$ Niederalkylgruppe ist; $R_8$ H, Halogen, Hydroxy, $C_1$-$C_3$ Alkoxy, Amino, Nitro, Cyano oder eine $C_1$-$C_3$ Niederalkylgruppe ist; $R_7$ und $R_9$ jeweils unabhängig Halogen, Hydroxy, Mercapto, - ONO, -$ONO_2$ oder SNO sind, wobei $R_7$ und $R_9$ gleich oder unterschiedlich sind;

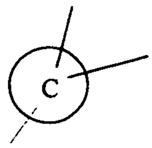

ist ein $C_5$-$C_6$ gliedriger gesättigter oder ungesättigter heterocyclischer Ring, enthaltend 1-2 Heteroatome, in welchem das Heteroatom ausgewählt ist aus einer Gruppe bestehend aus O S und N, vorzugsweise

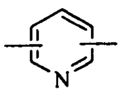 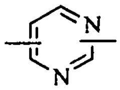 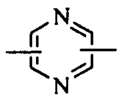 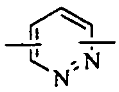 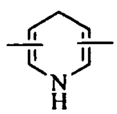 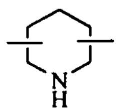

C1 C2 C3 C4 C5 C6

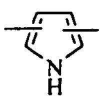 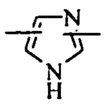 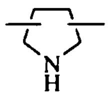 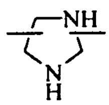 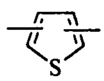 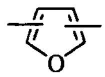

C7 C8 C9 C10 C11 C12 ,

besonders bevorzugt C1 (Pyridylgruppe) substituiert an Position 2 und 6 oder Position 2 und 5, C7 (Pyrrolylgruppe) substituiert an Position 2 und 5 oder Position 2 und 4, C11 (Thiophenylgruppe) oder C12 (Furanylgruppe), eine Bindung von Substituenten kann in einer symmetrischen oder asymmetrischen Position sein; $Z_1$ ist eine $C_1$-$C_{10}$ geradkettige oder verzweigtkettige Alkylgruppe, bevorzugt eine $C_2$-$C_5$ geradkettige oder verzweigtkettige Alkylgruppe oder Cycloalkylgruppe mit Substituent; $Z_2$ und $Z_3$ sind jeweils unabhängig H oder eine Methylgruppe, in welcher $Z_3$ H ist, wenn $Z_2$ eine Methylgruppe ist, $Z_2$ H ist, wenn $Z_3$ eine Methylgruppe ist; $T_2$ ist -$X_1$- oder -$X_1C(X_2)$-, in dem $X_1$ und $X_2$ jeweils unabhängig O oder S sind; $B_2$ ist ausgewählt aus einer Gruppe bestehend aus den genannten (a), (b), -$Z_1$-$R_7$, (d) oder (e); $n_2$ ist eine ganze Zahl von 0-3, $n_3$ ist eine ganze Zahl von 0-5, $n_4$ ist eine ganze Zahl von 1-5, $n_5$ und $n_6$ sind jeweils unabhängig eine ganze Zahl von 1-6;
(2) $R_2$ und $R_3$ sind jeweils unabhängig $C_3$-$C_7$ Cycloalkyl oder $C_1$-$C_7$ Alkyl;
(3) $R_4$ ist $SCH_3$ oder $OCH_3$;
(4) X ist O oder S.

**5.** Tricyclisches Derivat oder pharmazeutisch verträgliche Salze davon nach einem der Ansprüche 1 bis 4, wobei das tricyclische Derivat umfasst:

1) 6-Nitrooxymethyl-N-[(7S)-1,2,3-trimethoxy-1 0-methylsulfanyl-9-oxo-5,6,7,9-tetrahydro-benzo[a]heptalen-7-yl]-nicotinamid;
2) 5-Nitrooxymethyl-furan-2-carbonsäure-[(7S)-1,2,3-trimethoxy-1 0-methylsulfanyl-9-oxo-5,6,7,9-tetrahydro-benzo[a]heptalen-7-yl]-amid;
5) 6-Nitrooxymethyl-pyridin-2-carbonsäure-[(7S)-1,2,3-trimethoxy-1 0-methylsulfanyl-9-oxo-5,6,7,9-tetrahydro-benzo[a]heptalen-7-yl]-amid;
6) 5-Nitrooxymethyl-thiophen-2-carbonsäure-[(7S)-1,2,3-trimethoxy-1 0-methylsulfanyl-9-oxo-5,6,7,9-tetrahydro-benzo[a]heptalen-7-yl]-amid;
8) N-[(7S)-3-Ethoxy-1,2-dimethoxy-10-methylsulfanyl-9-oxo-5,6,7,9-tetrahydro-benzo[a]heptalen-7-yl]-2-fluor-3-nitrooxymethyl-benzamid;
9) 2-Fluor-N-[(7S)-3-isopropoxy-1,2-dimethoxy-1 0-methylsulfanyl-9-oxo-5,6,7,9-tetrahydro-benzo[a]heptalen-7-yl]-3-nitrooxymethyl-benzamid;
10) 2-Fluor-3-nitrooxymethyl-N-[(7S)-1,2,3-trimethoxy-10-methylsulfanyl-9-oxo-5,6,7,9-tetrahydro-benzo[a]heptalen-7-yl]-benzamid;
11) N-[(7S)-3-Cyclopentyloxy-1,2-dimethoxy-10-methylsulfanyl-9-oxo-5,6,7,9-tetrahydro-benzo[a]heptaten-7-yl]-2-fluor-3-nitrooxymethyl-benzamid;
12) 3-Fluor-5-nitrooxymethyl-N-[(7S)-1,2,3-trimethoxy-10-methylsulfanyl-9-oxo-5,6,7,9-tetrahydro-benzo[a]heptalen-7-yl]-benzamid;
13) N-[(7S)-3-Ethoxy-1,2-dimethoxy-1 0-methylsulfanyl-9-oxo-5,6,7,9-tetrahydro-benzo[a]heptalen-7-yl]-3-fluor-5-nitrooxymethyl-benzamid;
14) 3-Fluor-N-[(7S)-3-isopropoxy-1,2-dimethoxy-10-methylsulfanyl-9-oxo-5,6,7,9-tetrahydro-benzo[a]heptalen-7-yl]-5-nitrooxymethyl-benzamid;
15) N-[(7S)-3-Cyclopentyloxy-1,2-dimethoxy-1 0-methylsulfanyl-9-oxo-5,6,7,9-tetrahydro-benzo[a]heptalen-7-yl]-3-fluor-5-nitrooxymethyl-benzamid;
16) 4-Fluor-3-nitrooxymethyl-N-[(7S)-1,2,3-trimethoxy-1 0-methylsulfanyl-9-oxo-5,6,7,9-tetrahydro-benzo[a]heptalen-7-yl]-benzamid;
17) 2-Fluor-5-nitrooxymethyl-N-[(7S)-1,2,3-trimethoxy-10-methylsulfanyl-9-oxo-5,6,7,9-tetrahydro-benzo[a]heptalen-7-yl]-benzamid;
18) 3-Hydroxy-5-nitrooxymethyl-N-[(7S)-1,2,3-trimethoxy-10-methylsulfanyl-9-oxo-5,6,7,9-tetrahydro-benzo[a]heptalen-7-yl]-benzamid;
20) 2-Hydroxy-4-nitrooxymethyl-N-[(7S)-1,2,3-trimethoxy-1 0-methylsulfanyl-9-oxo-5,6,7,9-tetrahydro-benzo[a]heptalen-7-yl]-benzamid;
21) 4-Nitrooxymethyl-thiophen-2-carbonsäure-[(7S)-1,2,3-trimethoxy-1 0-methylsulfanyl-9-oxo-5,6,7,9-tetrahydro-benzo[a]heptalen-7-yl]-amid;
22) 3-Nitrooxymethyl-thiophen-2-carbonsäure-[(7S)-1,2,3-trimethoxy-1 0-methylsulfanyl-9-oxo-5,6,7,9-tetrahydro-benzo[a]heptalen-7-yl]-amid;
25) 3-Nitrooxybenzoesäure-5-[(7S)-1,2,3-trimethoxy-1 0-methylsulfanyl-9-oxo-5,6,7,9-tetrahydro-benzo[a]heptalen-7-yl-carbamoyl]-pyridin-2-yl-methylester;
26) 4-Nitrooxybuttersäure-5-[(7S)-1,2,3-trimethoxy-10-methylsulfanyl-9-oxo-5,6,7,9-tetrahydro-benzo[a]heptalen-7-yl-carbamoyl]-pyridin-2-yl-methylester;
27) 3-Nitrooxymethylbenzoesäure-6-[(7S)-1,2,3-trimethoxy-10-methylsulfanyl-9-oxo-5,6,7,9-tetrahydro-benzo[a]heptalen-7-yl-carbamoyl]-pyridin-2-yl-methylester;
28) 4-Nitrooxybuttersäure-6-[(7S)-1,2,3-trimethoxy-1 0-methylsulfanyl-9-oxo-5,6,7,9-tetrahydro-benzo[a]heptalen-7-yl-carbamoyl]-pyridin-2-yl-methylester;
29) 3-Nitrooxymethyl-benzoesäure-2-[(7S)-1,2,3-trimethoxy-1 0-methylsulfanyl-9-oxo-5,6,7,9-tetrahydro-benzo[a]heptalen-7-yl-carbamoyl]-phenylester;
30) 4-Nitrooxybuttersäure-2-[(7S)-1,2,3-trimethoxy-10-methylsulfanyl-9-oxo-5,6,7,9-tetrahydro-benzo[a]heptalen-7-yl-carbamoyl]-phenylester;
31) 3-Nitrooxymethyl-benzoesäure-3-[(7S)-1,2,3-trimethoxy-1 0-methylsulfanyl-9-oxo-5,6,7,9-tetrahydro-benzo[a]heptalen-7-yl-carbamoyl]-phenylester;
32) 4-Nitrooxybuttersäure-3-[(7S)-1,2,3-trimethoxy-1 0-methylsulfanyl-9-oxo-5,6,7,9-tetrahydro-benzo[a]heptalen-7-yl-carbamoyl]-phenylester;
33) 3-Nitrooxymethyl-benzoesäure-3-[(7S)-1,2,3-trimethoxy-1 0-methylsulfanyl-9-oxo-5,6,7,9-tetrahydro-benzo[a]heptalen-7-yl-carbamoyl]-benzylester;
34) 4-Nitrooxybuttersäure-3-[(7S)-1,2,3-trimethoxy-10-methylsulfanyl-9-oxo-5,6,7,9-tetrahydro-benzo[a]heptalen-7-yl-carbamoyl]-benzylester;
37) 3-Fluor-5-nitrosooxymethyl-N-[(7S)-1,2,3-trimethoxy-1 0-methylsulfanyl-9-oxo-5,6,7,9-tetrahydro-benzo[a]heptalen-7-yl]-benzamid;
39) 3-Fluor-5-nitrosothiomethyl-N-[(7S)-1,2,3-trimethoxy-1 0-methylsulfanyl-9-oxo-5,6,7,9-tetrahydro-benzo[a]heptalen-7-yl]-benzamid;
40) 3-Fluor-5-nitrooxymethyl-N-[(7S)-1,2,3,10-tetramethoxy-9-oxo-5,6,7,9-tetrahydro-benzo[a]heptalen-7-yl]-benzamid;
42) 3-Fluor-N-methyl-5-nitrooxymethyl-N-[(7S)-1,2,3-trimethoxy-10-methylsulfanyl-9-oxo-5,6,7,9-tetrahydro-benzo[a]heptalen-7-yl]-benzamid;

43) 2-(3-Fluor-5-nitrooxymethyl-phenyl)-N-[(7S)-1,2,3-trimethoxy-10-methylsulfanyl-9-oxo-5,6,7,9-tetrahydro-benzo[a]heptalen-7-yl]-acetamid; oder

44) 2-(2-Fluor-5-nitrooxymethyl-phenyl)-N-[(7S)-1,2,3-trimethoxy-10-methylsulfanyl-9-oxo-5,6,7,9-tetrahydro-benzo[a]heptalen-7-yl]-acetamid.

**6.** Verfahren zum Herstellen von tricyclischen Derivaten, wie in <Schema 1> dargestellt, umfassend die folgenden Schritte:

(1) Umsetzung der Verbindung der Formel (III) mit der Verbindung der Formel (IV) oder der Verbindung der Formel (VI) wird durchgeführt, um die Verbindung der Formel (V) oder die Verbindung der Formel (VII) zu geben, oder Umsetzung der resultierenden Verbindung der Formel (VII) mit der Halogenverbindung wird durchgeführt, um die Verbindung der Formel (V) zu geben (Schritt 1); und

(2) Nitrierung oder Nitrosierung der hergestellten Verbindung der Formel (V) oder der Verbindung der Formel (VII) wird durchgeführt, um die Verbindung der Formel (IIa) zu geben (Schritt 2).

## <Schema 1>

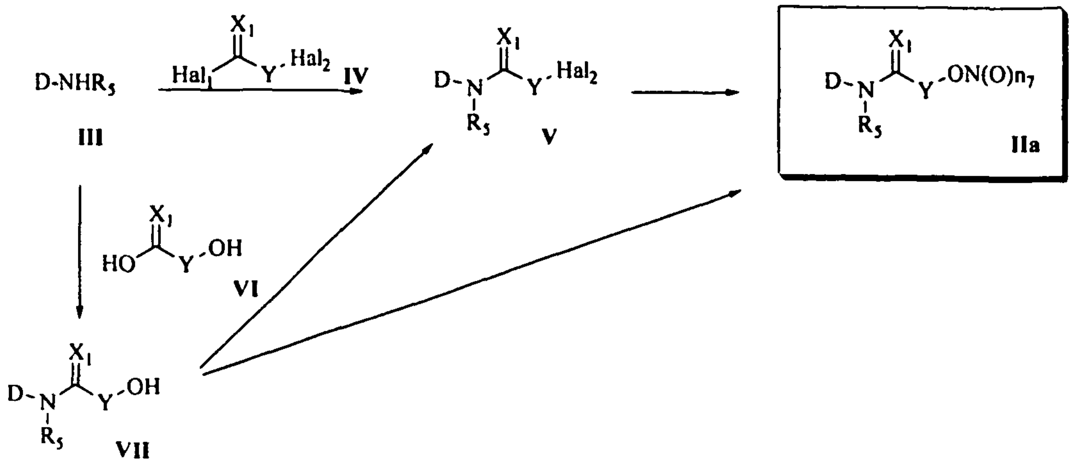

wobei D

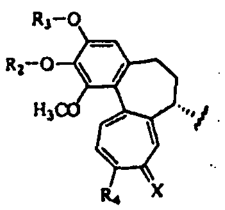

ist, und $R_2$, $R_3$, $R_4$ und X die Gleichen sind wie in einem der Ansprüche 1 bis 4 definiert;
$R_5$ H oder Alkyl mit niedrigem Molekulargewicht ist; $X_1$ O oder S ist; $Hal_1$ und $Hal_2$ Halogene sind; $Hal_1$ und $Hal_2$ der allgemeinen Formeln (IV) and (V) sind jeweils gleiche oder unterschiedliche Halogene, zum Beispiel F, Cl, Br oder I; Y zeigt die allgemeine Formel (a') und (b'), (c'), (d') und (e') an,

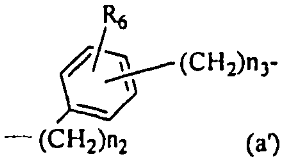 (a')

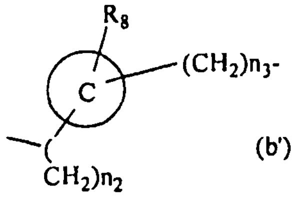 (b')

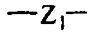 (c')

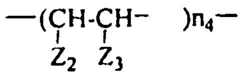 (d')

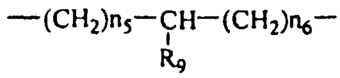 (e')

wobei

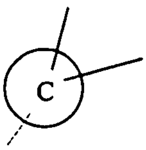,

$R_6$, $R_8$, $R_9$, $Z_1$, $Z_2$, $Z_3$, $n_2$, $n_3$, $n_4$, $n_5$ und $n_6$ die Gleichen sind wie in einem der Ansprüche 1 bis 4 definiert.

**7.** Antikrebsmittel oder Antiproliferationsmittel, enthaltend tricyclische Derivate nach einem der Ansprüche 1 bis 5 oder pharmazeutisch verträgliche Salze davon als ein wirksamer Bestandteil.

**8.** Angiogeneseinhibitor, enthaltend tricyclische Derivate nach einem der Ansprüche 1 bis 5 oder pharmazeutisch verträgliche Salze davon als ein wirksamer Bestandteil.

**9.** Verwendung einer Verbindung des tricyclischen Derivats nach einem der Ansprüche 1 bis 5 oder pharmazeutisch verträglicher Salze davon zur Herstellung eines Medikaments zur Behandlung von Krebs oder Krankheiten, die mit einem Antiproliferationsmittel behandelt werden können.

**10.** Verwendung einer Verbindung des tricyclischen Derivats nach einem der Ansprüche 1 bis 5 oder pharmazeutisch verträglicher Salze davon zur Herstellung eines Medikaments zur Angiogeneseinhibition.

**11.** Verbindung nach einem der Ansprüche 1 bis 5 oder ein pharmazeutisch verträgliches Salz davon zur Verwendung als Antikrebsmittel oder Antiproliferationsmittel.

**12.** Verbindung nach einem der Ansprüche 1 bis 5 oder ein pharmazeutisch verträgliches Salz davon zur Verwendung als Angiogeneseinhibitor.

## Revendications

**1.** Dérivé tricyclique représenté par la Formule 1 suivante, ou ses sels pharmaceutiquement acceptables.

Formule 1

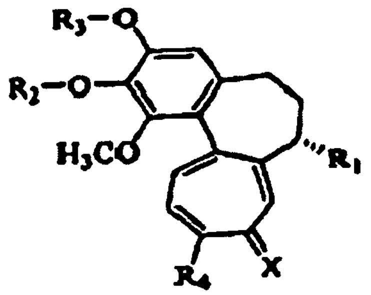

dans laquelle

(1) $R_1$ est $-T_1-B_1$; où $T_1$ est $-X_1-$, $X_1-C(X_2)$ -, $-N(R_5)-$, $-N(R_5)C(X_2)-$, $-N(R_5)S(O)n_1-$, $-N(R_5)C(O)-X_1-$ ou $-N(R_5)C(X_1)NH-$, chacun de $X_1$ et $X_2$ étant O ou S, chaque $R_5$ étant H ou un groupe alkyle en $C_1$ à $C_5$, $n_1$ étant l'entier 1 ou 2 ; et $B_1$ est choisi dans l'ensemble constitué par

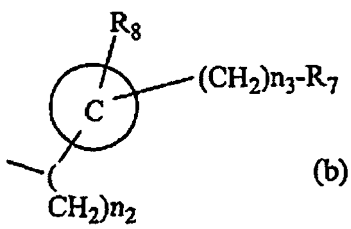

(b)

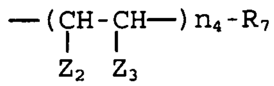

(d)

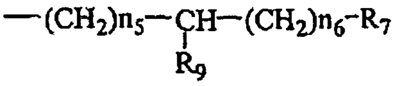

(c)

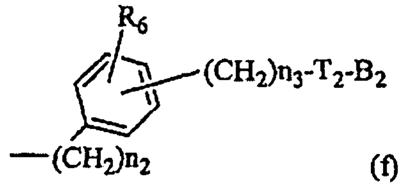

(f)

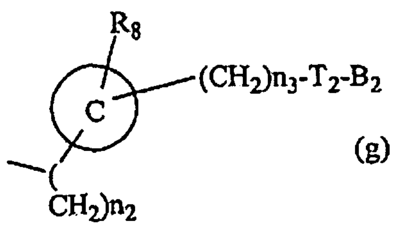

(g)

$-Z_1-T_2-B_2$ (h)

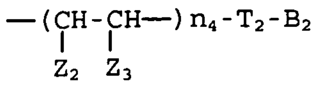

(i)

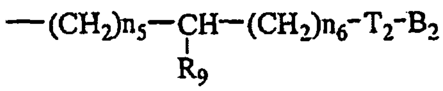

(j)

où chacun de $R_6$ et $R_8$ est H, un halogène ou un groupe hydroxy, alcoxy en $C_1$ à $C_3$, amino, nitro, cyano ou alkyle inférieur en $C_1$ à $C_3$ ; et chacun de $R_7$ et $R_9$ est indépendamment un halogène, hydroxy, mercapto, -ONO, $-ONO_2$ ou SNO, $R_7$ et $R_9$ étant identiques ou différents ;

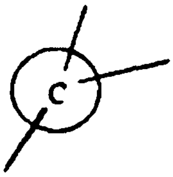

est un hétérocycle saturé ou insaturé à 5 ou 6 chaînons contenant 1 ou 2 hétéroatomes, l'hétéroatome étant choisi dans l'ensemble constitué par O, S et N, de préférence,

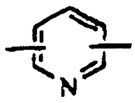 C1 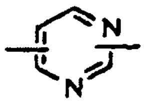 C2 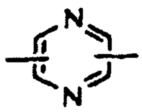 C3 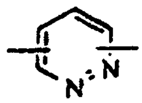 C4 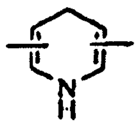 C5 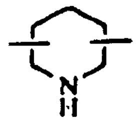 C6

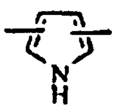 C7 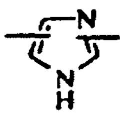 C8 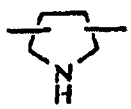 C9 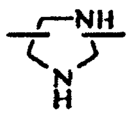 C10 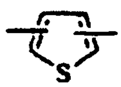 C11 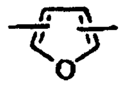 C12 ,

plus préférablement, C1 (groupe pyridyle) substitué en positions 2 et 6 ou en positions 2 et 5, C7 (groupe pyrrolyle) substitué en positions 2 et 5 ou en positions 2 et 4, C11 (groupe thiophényle) ou C12 (groupe furanyle) ; $Z_1$ est un groupe alkyle à chaîne droite ou ramifiée en $C_1$ à $C_{10}$, de préférence un groupe alkyle à chaîne droite ou ramifiée en $C_2$ à $C_5$ ou un groupe cycloalkyle portant un substituant ; chacun de $Z_2$ et $Z_3$ est indépendamment H ou le groupe méthyle, $Z_3$ étant H quand $Z_2$ est le groupe méthyle, et $Z_2$ étant H quand $Z_3$ est le groupe méthyle ; $T_2$ est $-X_1-$ ou $-X_1-C(X_2)-$, chacun de $X_1$ et $X_2$ étant indépendamment O ou S ; $B_2$ est choisi dans l'ensemble constitué par ledit

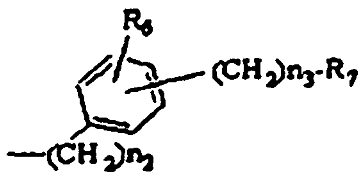 ,

(b), $-Z_1-R_7$, (d) ou (e) ; $n_2$ est un entier de 0 à 3 ; $n_3$ est un entier de 0 à 5, $n_4$ est un entier de 1 à 5, chacun de $n_5$ et $n_6$ est indépendamment un entier de 1 à 6 ;
(2) chacun de $R_2$ et $R_3$ est indépendamment H, $-PO_3H_2$, phosphonate, sulfate, cycloalkyle en $C_3$ à $C_7$ , alcényle en $C_2$ à $C_7$, alcynyle en $C_2$ à $C_7$, alcanoyle en $C_1$ à $C_7$, alkyle à chaîne droite ou ramifiée en $C_1$ à $C_7$ ou un sucre, lequel sucre est un monosaccharide tel que glucuronyle, glucosyle ou galactosyle ;
(3) $R_4$ est $OCH_3$, $SCH_3$ ou $NR_{10}R_{11}$, où chacun de $R_{10}$ et $R_{11}$ est indépendamment H ou un alkyle en $C_1$ à $C_5$ ;
(4) X est O ou S.

**2.** Dérivé tricyclique représenté par la Formule 1 suivante, ou ses sels pharmaceutiquement acceptables.

Formule 1

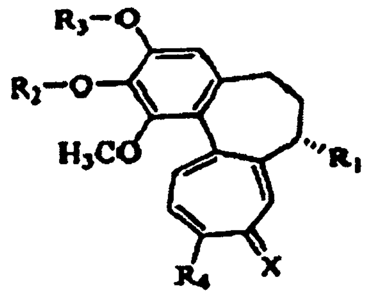

dans laquelle

(1) $R_1$ est $-T_1-B_1$ ;
où $T_1$ est $-X_1-$, $-X_1-C(X_2)-$, $-N(R_5)-$, $-N(R_5)C(X_2)-$, $-N(R_5)S(O)n_1-$, $-N(R_5)C(O)-X_1-$ ou $-N(R_5)C(X_1)NH-$, chacun de $X_1$ et $X_2$ étant O ou S, chaque $R_5$ étant H ou un groupe alkyle en $C_1$ à $C_5$, $n_1$ étant l'entier 1 ou 2 ; et $B_1$ est choisi dans l'ensemble constitué par

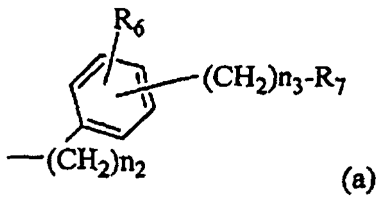
(a)

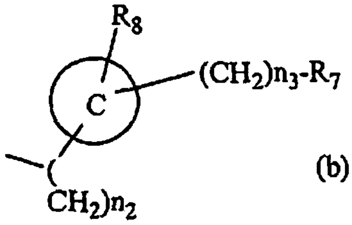
(b)

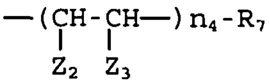
(d)

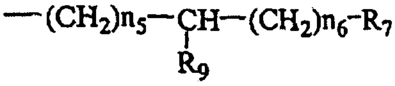
(e)

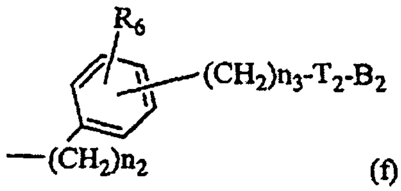
(f)

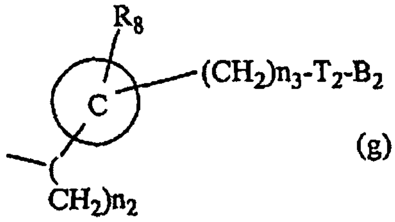
(g)

$-Z_1-T_2-B_2$ (h)

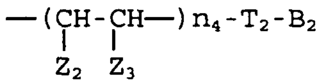
(i)

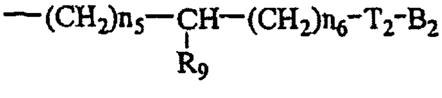
(j)

où $R_6$ est un halogène ou un groupe hydroxy, alcoxy en $C_1$ à $C_3$, amino, nitro, cyano ou alkyle inférieur en $C_1$ à $C_3$ ; $R_8$ est H, un halogène ou un groupe hydroxy, alcoxy en $C_1$ à $C_3$, amino, nitro, cyano ou alkyle inférieur

en $C_1$ à $C_3$ ; chacun de $R_7$ et $R_9$ est indépendamment un halogène, hydroxy, mercapto, -ONO, $-ONO_2$ ou SNO, $R_7$ et $R_9$ étant identiques ou différents ;

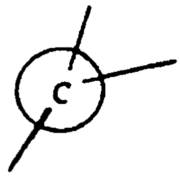

est un hétérocycle saturé ou insaturé à 5 ou 6 chaînons contenant 1 ou 2 hétéroatomes, l'hétéroatome étant choisi dans l'ensemble constitué par O, S et N, de préférence,

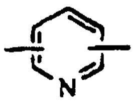 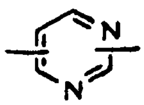 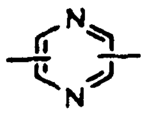 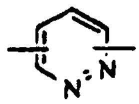 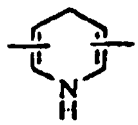 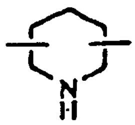

C1 C2 C3 C4 C5 C6

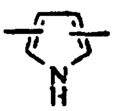 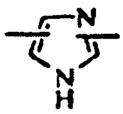 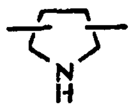 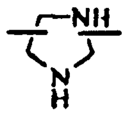 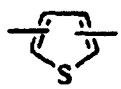 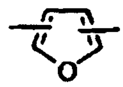

C7 C8 C9 C10 C11 C12 ,

plus préférablement, C1 (groupe pyridyle) substitué en positions 2 et 6 ou en positions 2 et 5, C7 (groupe pyrrolyle) substitué en positions 2 et 5 ou en positions 2 et 4, C11 (groupe thiophényle) ou C12 (groupe furanyle) ; $Z_1$ est un groupe alkyle à chaîne droite ou ramifiée en $C_1$ à $C_{10}$, de préférence un groupe alkyle à chaîne droite ou ramifiée en $C_2$ à $C_5$ ou un groupe cycloalkyle portant un substituant ; chacun de $Z_2$ et $Z_3$ est indépendamment H ou le groupe méthyle, $Z_3$ étant H quand $Z_2$ est le groupe méthyle, et $Z_2$ étant H quand $Z_3$ est le groupe méthyle ; $T_2$ est $-X_1-$ ou $-X_1-C(X_2)$ -, chacun de $X_1$ et $X_2$ étant indépendamment O ou S ; $B_2$ est choisi dans l'ensemble constitué par ledit (a), (b), $-Z_1-R_7$, (d) ou (e) ; $n_2$ est un entier de 0 à 3 ; $n_3$ est un entier de 0 à 5, $n_4$ est un entier de 1 à 5, chacun de $n_5$ et $n_6$ est indépendamment un entier de 1 à 6 ;

(2) chacun de $R_2$ et $R_3$ est indépendamment H, $-PO_3H_2$, phosphonate, sulfate, cycloalkyle en $C_3$ à $C_7$, alcényle en $C_2$ à $C_7$, alcynyle en $C_2$ à $C_7$, alcanoyle en $C_1$ à $C_7$, alkyle à chaîne droite ou ramifiée en $C_1$ à $C_7$ ou un sucre, lequel sucre est un monosaccharide tel que glucuronyle, glucosyle ou galactosyle ;

(3) $R_4$ est $OCH_3$ , $SCH_3$ ou $NR_{10}R_{11}$, où chacun de $R_{10}$ et $R_{11}$ est indépendamment H ou un alkyle en $C_1$ à $C_5$ ;

(4) X est O ou S.

**3.** Dérivé tricyclique ou ses sels pharmaceutiquement acceptables selon la revendication 1, dans lesquels le composé de formule 1 est **caractérisé** comme suit :

(1) $R_1$ est $-T_1-B_1$ ;

où $T_1$ est $-N(R_5)C(X_2)-$, $-N(R_5)C(O)-X_1-$ ou $-N(R_5)C(X_1)NH-$, chacun de $X_1$ et $X_2$ étant 0, chaque $R_5$ étant H ou un groupe alkyle en $C_1$ à $C_5$ ; et $B_1$ est choisi dans l'ensemble constitué par

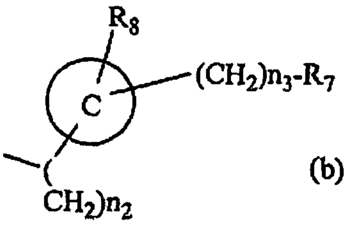

(b)

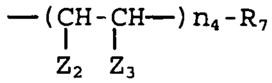

(d)

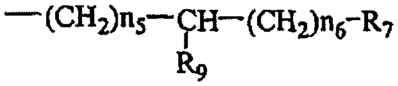

(e)

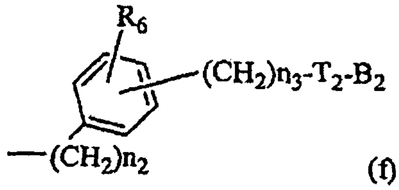

(f)

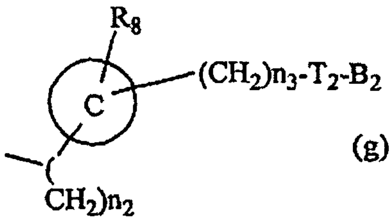

(g)

$-Z_1-T_2-B_2$ (h)

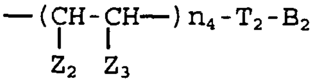

(i)

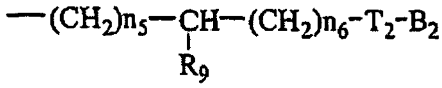

(j)

où chacun de $R_6$ et $R_8$ est H, un halogène ou un groupe hydroxy, alcoxy en $C_1$ à $C_3$, amino, nitro, cyano ou alkyle inférieur en $C_1$ à $C_3$ ; chacun de $R_7$ et $R_9$ est indépendamment un halogène, hydroxy, mercapto (thiol), -ONO, -$ONO_2$ ou SNO, $R_7$ et $R_9$ étant identiques ou différents ;

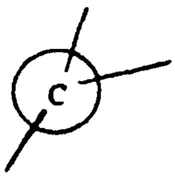

est un hétérocycle saturé ou insaturé à 5 ou 6 chaînons contenant 1 ou 2 hétéroatomes, l'hétéroatome étant choisi dans l'ensemble constitué par O, S et N, de préférence,

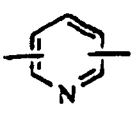

C1

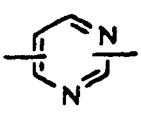

C2

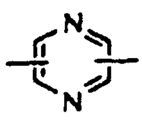

C3

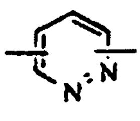

C4

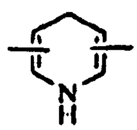

C5

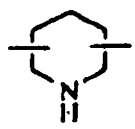

C6

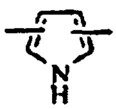 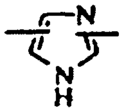 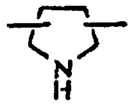 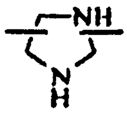 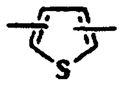 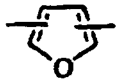

C7 C8 C9 C10 C11 C12 ,

plus préférablement, C1 (groupe pyridyle) substitué en positions 2 et 6 ou en positions 2 et 5, C7 (groupe pyrrolyle) substitué en positions 2 et 5 ou en positions 2 et 4, C11 (groupe thiophényle) ou C12 (groupe furanyle), une liaison de substituants pouvant être présente en position symétrique ou asymétrique ; $Z_1$ est un groupe alkyle à chaîne droite ou ramifiée en $C_1$ à $C_{10}$, de préférence un groupe alkyle à chaîne droite ou ramifiée en $C_2$ à $C_5$ ou un groupe cycloalkyle portant un substituant ; chacun de $Z_2$ et $Z_3$ est indépendamment H ou le groupe méthyle, $Z_3$ étant H quand $Z_2$ est le groupe méthyle, et $Z_2$ étant H quand $Z_3$ est le groupe méthyle ; $T_2$ est $-X_1-$ ou $-X_1-C(X_2)-$, chacun de $X_1$ et $X_2$ étant indépendamment O ou S ; $B_2$ est choisi dans l'ensemble constitué par ledit

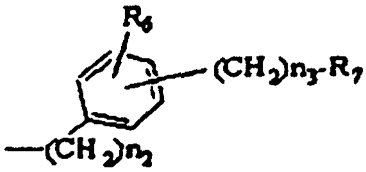 ,

(b), $-Z_1-R_7$, (d) ou (e) ; $n_2$ est un entier de 0 à 3 ; $n_3$ est un entier de 0 à 5, $n_4$ est un entier de 1 à 3, chacun de $n_5$ et $n_6$ est indépendamment un entier de 1 à 3 ;

(2) chacun de $R_2$ et $R_3$ est indépendamment un cycloalkyle en $C_3$ à $C_7$ ou alkyle en $C_1$ à $C_7$ ;

(3) $R_4$ est $SCH_3$ ou $OCH_3$ ;

(4) X est O ou S.

**4.** Dérivé tricyclique ou ses sels pharmaceutiquement acceptables selon la revendication 2, dans lesquels le composé de formule 1 est **caractérisé** comme suit :

(1) $R_1$ est $-T_1-B_1$ ;

où $T_1$ est $-N(R_5)C(X_2)-$, $-N(R_5)C(O)-X_1-$ ou $-N(R_5)C(X_1)NH-$, chacun de $X_1$ et $X_2$ étant O, chaque $R_5$ étant H ou un groupe alkyle en $C_1$ à $C_5$ ; et $B_1$ est choisi dans l'ensemble constitué par

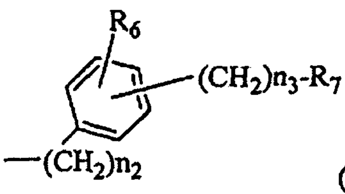 (a)

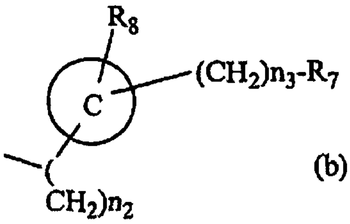 (b)

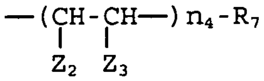 (d)

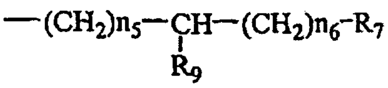 (e)

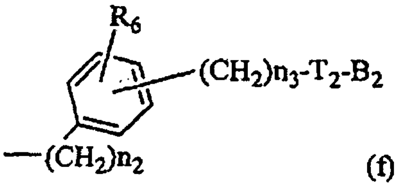

(f)

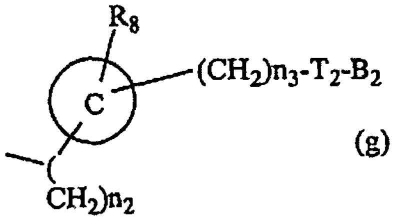

(g)

$-Z_1-T_2-B_2$ (h)

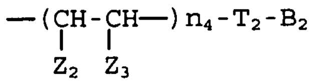

(i)

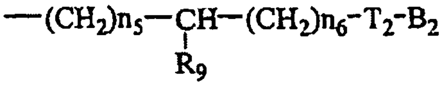

(j)

où $R_6$ est un halogène ou un groupe hydroxy, alcoxy en $C_1$ à $C_3$, amino, nitro, cyano ou alkyle inférieur en $C_1$ à $C_3$ ; $R_8$ est H, un halogène ou un groupe hydroxy, alcoxy en $C_1$ à $C_3$, amino, nitro, cyano ou alkyle inférieur en $C_1$ à $C_3$ ; chacun de $R_7$ et $R_9$ est indépendamment un halogène, hydroxy, mercapto, -ONO, $-ONO_2$ ou SNO, $R_7$ et $R_9$ étant identiques ou différents ;

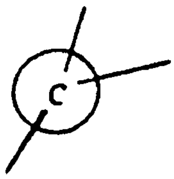

est un hétérocycle saturé ou insaturé à 5 ou 6 chaînons contenant 1 ou 2 hétéroatomes, l'hétéroatome étant choisi dans l'ensemble constitué par O, S et N, de préférence,

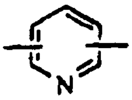 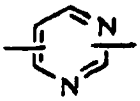 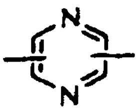 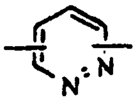 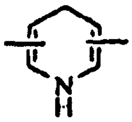 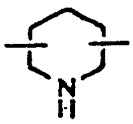

C1 C2 C3 C4 C5 C6

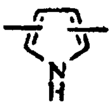 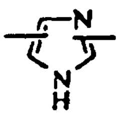 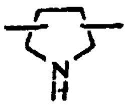 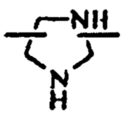 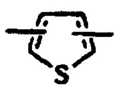 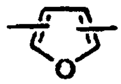

C7 C8 C9 C10 C11 C12 ,

plus préférablement, C1 (groupe pyridyle) substitué en positions 2 et 6 ou en positions 2 et 5, C7 (groupe pyrrolyle) substitué en positions 2 et 5 ou en positions 2 et 4, C11 (groupe thiophényle) ou C12 (groupe furanyle) ; une liaison de substituants pouvant être présente en position symétrique ou asymétrique ; $Z_1$ est un groupe alkyle à chaîne droite ou ramifiée en $C_1$ à $C_{10}$, de préférence un groupe alkyle à chaîne droite ou ramifiée en $C_2$ à $C_5$ ou un groupe cycloalkyle portant un substituant ; chacun de $Z_2$ et $Z_3$ est indépendamment H ou le groupe méthyle, $Z_3$ étant H quand $Z_2$ est le groupe méthyle, et $Z_2$ étant H quand $Z_3$ est le groupe méthyle ; $T_2$ est $-X_1-$ ou $-X_1-C(X_2)-$, chacun de $X_1$ et $X_2$ étant indépendamment O ou S ; $B_2$ est choisi dans l'ensemble

constitué par ledit (a), (b), $-Z_1-R_7$, (d) ou (e) ; $n_2$ est un entier de 0 à 3, $n_3$ est un entier de 0 à 5, $n_4$ est un entier de 1 à 5, chacun de $n_5$ et $n_6$ est indépendamment un entier de 1 à 6 ;
(2) chacun de $R_2$ et $R_3$ est indépendamment un cycloalkyle en $C_3$ à $C_7$ ou alkyle en $C_1$ à $C_7$ ;
(3) $R_4$ est $SCH_3$ ou $OCH_3$ ;
(4) X est O ou S.

**5.** Dérivé tricyclique ou ses sels pharmaceutiquement acceptables selon l'une quelconque des revendications 1 à 4, lequel dérivé tricyclique englobe l'un des suivants :

1) 6-nitroxyméthyl-N-[(7S)-1,2,3-triméthoxy-10-méthyl-sulfanyl-9-oxo-5,6,7,9-tétrahydrobenzo[a]heptalén-7-yl]-nicotinamide ;
2) [(7S)-1,2,3-triméthoxy-10-méthylsulfanyl-9-oxo-5,6,7,9-tétrahydrobenzo[a]heptalén-7-yl]amide d'acide 5-nitroxyméthylfurane-2-carboxylique ;
5) [(7S)-1,2,3-triméthoxy-10-méthylsulfanyl-9-oxo-5,6,7,9-tétrahydrobenzo[a]heptalén-7-yl]amide d'acide 6-nitroxyméthylpyridine-2-carboxylique ;
6) [(7S)-1,2,3-triméthoxy-10-méthylsulfanyl-9-oxo-5,6,7,9-tétrahydrobenzo[a]heptalén-7-yl]amide d'acide 5-nitroxyméthylthiophène-2-carboxylique ;
8) N-[(7S)-3-éthoxy-1,2-diméthoxy-10-méthylsulfanyl-9-oxo-5,6,7,9-tétrahydrobenzo[a]heptalén-7-yl]-2-fluoro-3-nitroxyméthylbenzamide ;
9) 2-fluoro-N-[(7S)-3-isopropoxy-1,2-diméthoxy-10-méthylsulfanyl-9-oxo-5,6,7,9-tétrahydrobenzo[a]heptalén-7-yl]-3-nitroxyméthylbenzamide ;
10) 2-fluoro-3-nitroxyméthyl-N-[(7S)-1,2,3-triméthoxy-10-méthylsulfanyl-9-oxo-5,6,7,9-tétrahydrobenzo[a]heptalén-7-yl]-benzamide ;
11) N-[(7S)-3-cyclopentyloxy-1,2-diméthoxy-10-méthyl-sulfanyl-9-oxo-5,6,7,9-tétrahydrobenzo[a]heptalén-7-yl]-2-fluoro-3-nitroxyméthylbenzamide ;
12) 3-fluoro-5-nitroxyméthyl-N-[(7S)-1,2,3-triméthoxy-10-méthylsulfanyl-9-oxo-5,6,7,9-tétrahydrobenzo[a]heptalén-7-yl]-benzamide ;
13) N-[(7S)-3-éthoxy-1,2-diméthoxy-10-méthylsulfanyl-9-oxo-5,6,7,9-tétrahydrobenzo[a]heptalén-7-yl]-3-fluoro-5-nitroxyméthylbenzamide ;
14) 3-fluoro-N-[(7S)-3-isopropoxy-1,2-diméthoxy-10-méthylsulfanyl-9-oxo-5,6,7,9-tétrahydrobenzo[a]heptalén-7-yl]-5-nitroxyméthylbenzamide ;
15) N-[(7S)-3-cyclopentyloxy-1,2-diméthoxy-10-méthyl-sulfanyl-9-oxo-5,6,7,9-tétrahydrobenzo[a]heptalén-7-yl]-3-fluoro-5-nitroxyméthylbenzamide ;
16) 4-fluoro-3-nitroxyméthyl-N-[(7S)-1,2,3-triméthoxy-10-méthylsulfanyl-9-oxo-5,6,7,9-tétrahydrobenzo[a]heptalén-7-yl]-benzamide ;
17) 2-fluoro-5-nitroxyméthyl-N-[(7S)-1,2,3-triméthoxy-10-méthylsulfanyl-9-oxo-5,6,7,9-tétrahydrobenzo[a]heptalén-7-yl]-benzamide ;
18) 3-hydroxy-5-nitroxyméthyl-N-[(7S)-1,2,3-triméthoxy-10-méthylsulfanyl-9-oxo-5,6,7,9-tétrahydrobenzo[a]-heptalén-7-yl]-benzamide ;
20) 2-hydroxy-4-nitroxyméthyl-N-[(7S)-1,2,3-triméthoxy-10-méthylsulfanyl-9-oxo-5,6,7,9-tétrahydrobenzo[a]-heptalén-7-yl]-benzamide ;
21) [(7S)-1,2,3-triméthoxy-10-méthylsulfanyl-9-oxo-5,6,7,9-tétrahydrobenzo[a]heptalén-7-yl]-amide d'acide 4-nitroxyméthylthiophène-2-carboxylique ;
22) [(7S)-1,2,3-triméthoxy-10-méthylsulfanyl-9-oxo-5,6,7,9-tétrahydrobenzo[a]heptalén-7-yl]-amide d'acide 3-nitroxyméthylthiophène-2-carboxylique ;
25) ester 5-[(7S)-1,2,3-triméthoxy-10-méthylsulfanyl-9-oxo-5,6,7,9-tétrahydrobenzo[a]heptalén-7-ylcarbamoyl]-pyridine-2-ylméthylique d'acide 3-nitroxybenzoïque ;
26) ester 5-[(7S)-1,2,3-triméthoxy-10-méthylsulfanyl-9-oxo-5,6,7,9-tétrahydrobenzo[a]heptalén-7-ylcarbamoyl]-pyridine-2-ylméthylique d'acide 4-nitroxybenzoïque ;
27) ester 6-[(7S)-1,2,3-triméthoxy-10-méthylsulfanyl-9-oxo-5,6,7,9-tétrahydrobenzo[a]heptalén-7-ylcarbamoyl]-pyridine-2-ylméthylique d'acide 3-nitroxyméthylique ;
28) ester 6-[(7S)-1,2,3-triméthoxy-10-méthylsulfanyl-9-oxo-5,6,7,9-tétrahydrobenzo[a]heptalén-7-ylcarbamoyl]-pyridine-2-ylméthylique d'acide 4-nitroxybutyrique ;
29) ester 2-[(7S)-1,2,3-triméthoxy-10-méthylsulfanyl-9-oxo-5,6,7,9-tétrahydrobenzo[a]heptalén-7-ylcarbamoyl]-phénylique d'acide 3-nitroxyméthylbenzoïque ;
30) ester 2-[(7S)-1,2,3-triméthoxy-10-méthylsulfanyl-9-oxo-5,6,7,9-tétrahydrobenzo[a]heptalén-7-ylcarbamoyl]-phénylique d'acide 4-nitroxybutyrique ;
31) ester 3-[(7S)-1,2,3-triméthoxy-10-méthylsulfanyl-9-oxo-5,6,7,9-tétrahydrobenzo[a]heptalén-7-ylcarbamoyl]-phénylique d'acide 3-nitroxyméthylbenzoïque ;

32) ester 3-[(7S)-1,2,3-triméthoxy-10-méthylsulfanyl-9-oxo-5,6,7,9-tétrahydrobenzo[a]heptalén-7-ylcarbamoyl]-phénylique d'acide 4-nitroxybutyrique ;

33) ester 3-[(7S)-1,2,3-triméthoxy-10-méthylsulfanyl-9-oxo-5,6,7,9-tétrahydrobenzo[a]heptalén-7-ylcarbamoyl]-benzylique d'acide 3-nitroxyméthylbenzoïque ;

34) ester 3-[(7S)-1,2,3-triméthoxy-10-méthylsulfanyl-9-oxo-5,6,7,9-tétrahydrobenzo[a]heptalén-7-ylcarbamoyl]-benzylique d'acide 4-nitroxybutyrique ;

37) 3-fluoro-5-nitrosoxyméthyl-N-[(7S)-1,2,3-triméthoxy-10-méthylsulfanyl-9-oxo-5,6,7,9-tétrahydrobenzo[a]-heptalén-7-yl]-benzamide ;

39) 3-fluoro-5-nitrosothiométhyl-N-[(7S)-1,2,3-triméthoxy-10-méthylsulfanyl-9-oxo-5,6,7,9-tétrahydrobenzo-[a]heptalén-7-yl]-benzamide ;

40) 3-fluoro-5-nitroxyméthyl-N-[(7S)-1,2,3,10-tétraméthoxy-9-oxo-5,6,7,9-tétrahydrobenzo[a]heptalén-7-yl]-benzamide ;

42) 3-fluoro-N-méthyl-5-nitroxyméthyl-N-[(7S)-1,2,3-triméthoxy-10-méthylsulfanyl-9-oxo-5,6,7,9-tétrahydrobenzo-[a]heptalén-7-yl]-benzamide ;

43) 2-(3-fluoro-5-nitroxyméthylphényl)-N-[(7S)-1,2,3-triméthoxy-10-méthylsulfanyl-9-oxo-5,6,7,9-tétrahydrobenzo-[a]heptalén-7-yl]-acétamide ; et

44) 2-(2-fluoro-5-nitroxyméthylphényl)-N-[(7S)-1,2,3-triméthoxy-10-méthylsulfanyl-9-oxo-5,6,7,9-tétrahydrobenzo-[a]heptalén-7-yl]-acétamide.

**6.** Procédé pour préparer des dérivés tricycliques, tel que représenté dans le Schéma 1, comprenant les étapes suivantes :

(1) une réaction du composé de formule (III) avec le composé de formule (IV) ou le composé de formule (VI) est mise en oeuvre pour donner le composé de formule (V) ou le composé de formule (VII), ou bien une réaction du composé résultant de formule (VII) avec le composé halogéné est mise en oeuvre pour donner le composé de formule (V) (étape 1) ; et

(2) une nitration ou une nitrosation du composé préparé de formule (V) ou du composé de formule (VII) est mise en oeuvre pour donner le composé de formule (IIa) (étape 2).

Schéma 1

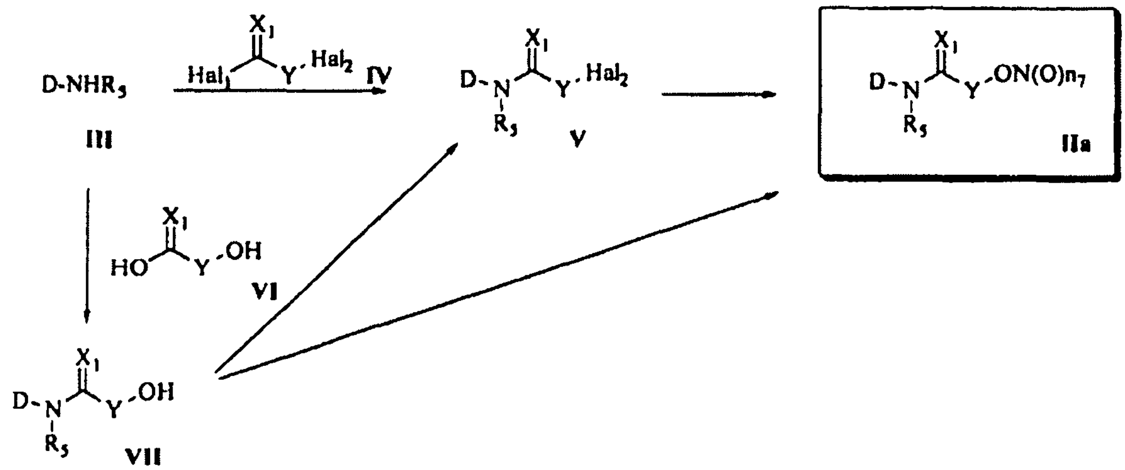

où D est

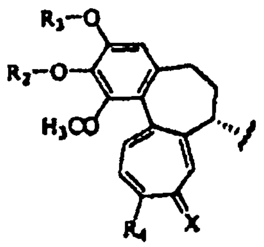,

et $R_2$, $R_3$, $R_4$ et X sont tels que définis dans l'une quelconque des revendications 1 à 4 ;
$R_5$ est H ou un alkyle de faible masse moléculaire ; $X_1$ est O ou S ; $Hal_1$ et $Hal_2$ sont des halogènes ; $Hal_1$ et $Hal_2$ des formules générales (IV) et (V) sont chacun des halogènes identiques ou différents, par exemple F, Cl, Br ou I ; Y indique les formules générales (a'), et (b'), (c'), (d') et (e') respectivement,

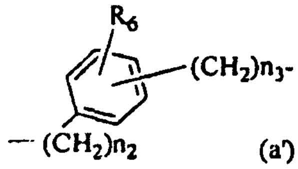
(a')

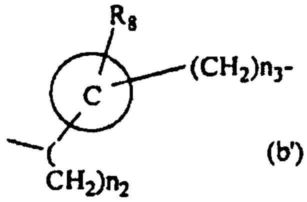
(b')

$-Z_1-$
(c')

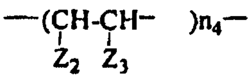
(d')

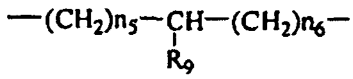
(e')

où

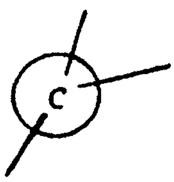,

$R_6$, $R_8$, $R_9$, $Z_1$ $Z_2$, $Z_3$, $n_2$, $n_3$, $n_4$, $n_5$ et $n_6$ sont tels que définis dans l'une quelconque des revendications 1 à 4.

**7.** Agent anticancéreux ou agent antiprolifératif contenant des dérivés tricycliques de l'une quelconque des revendications 1 à 5 ou des sels pharmaceutiquement acceptables de ceux-ci à titre d'ingrédient actif.

**8.** Inhibiteur d'angiogenèse contenant des dérivés tricycliques de l'une quelconque des revendications 1 à 5 ou des sels pharmaceutiquement acceptables de ceux-ci à titre d'ingrédient actif.

**9.** Utilisation d'un composé du dérivé tricyclique de l'une quelconque des revendications 1 à 5 ou de ses sels pharmaceutiquement acceptables pour la préparation d'un médicament destiné au traitement d'un cancer ou de maladies qui peuvent être traitées par un agent antiprolifératif.

**10.** Utilisation d'un composé du dérivé tricyclique de l'une quelconque des revendications 1 à 5 ou de ses sels pharmaceutiquement acceptables pour la préparation d'un médicament destiné à inhiber l'angiogenèse.

**11.** Composé selon l'une quelconque des revendications 1 à 5 ou sel pharmaceutiquement acceptable de celui-ci, pour utilisation en tant qu'agent anticancéreux ou agent antiprolifératif.

**12.** Composé selon l'une quelconque des revendications 1 à 5 ou sel pharmaceutiquement acceptable de celui-ci, pour utilisation en tant qu'inhibiteur de l'angiogenèse.

FIGURE

FIG.1

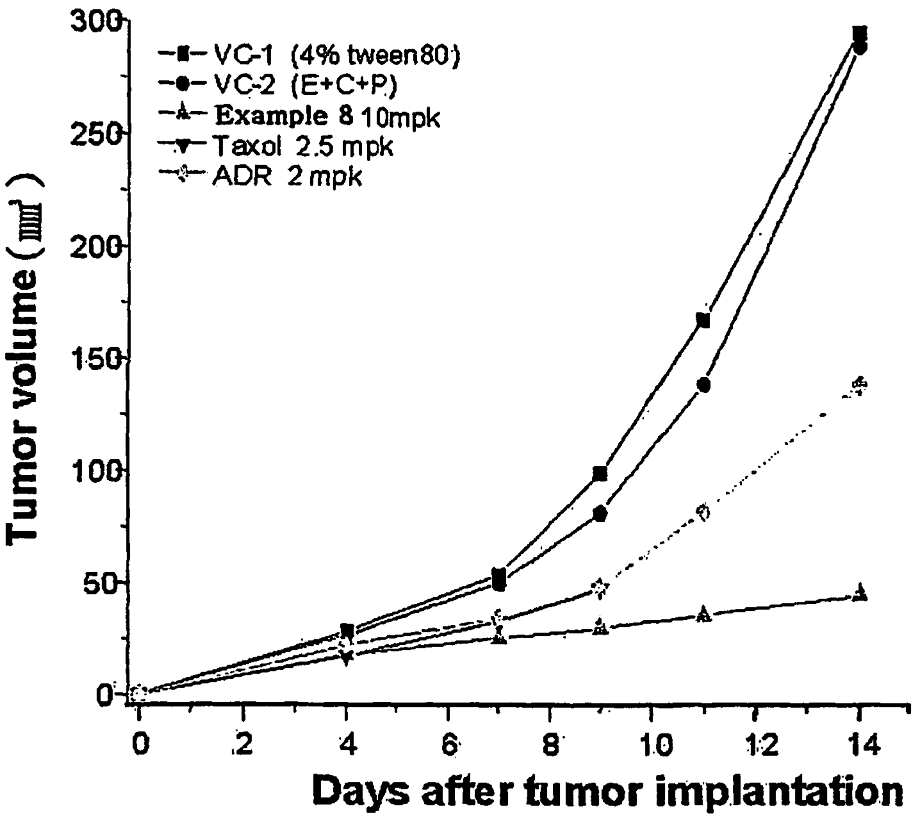
VC-1 (4% tween80)
VC-2 (E+C+P)
Example 8 10mpk
Taxol 2.5 mpk
ADR 2 mpk
Tumor volume (mm³)
0
50
100
150
200
250
300
0
2
4
6
8
10
12
14
Days after tumor implantation

FIG.2

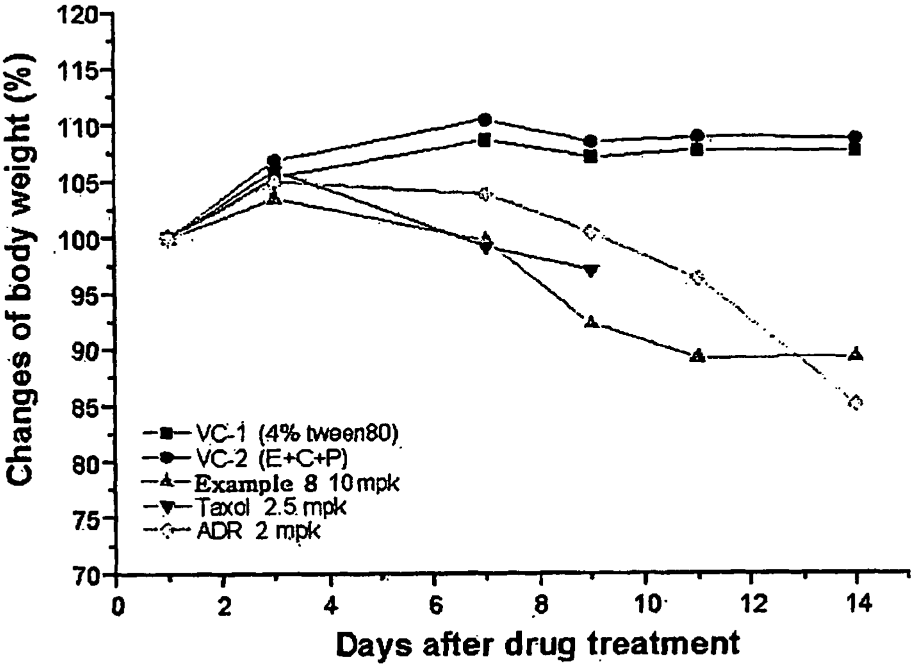
Changes of body weight (%)
120
115
110
105
100
95
90
85
80
75
70
0
2
4
6
8
10
12
14
Days after drug treatment
VC-1 (4% tween80)
VC-2 (E+C+P)
Example 8 10mpk
Taxol 2.5 mpk
ADR 2 mpk

FIG.3

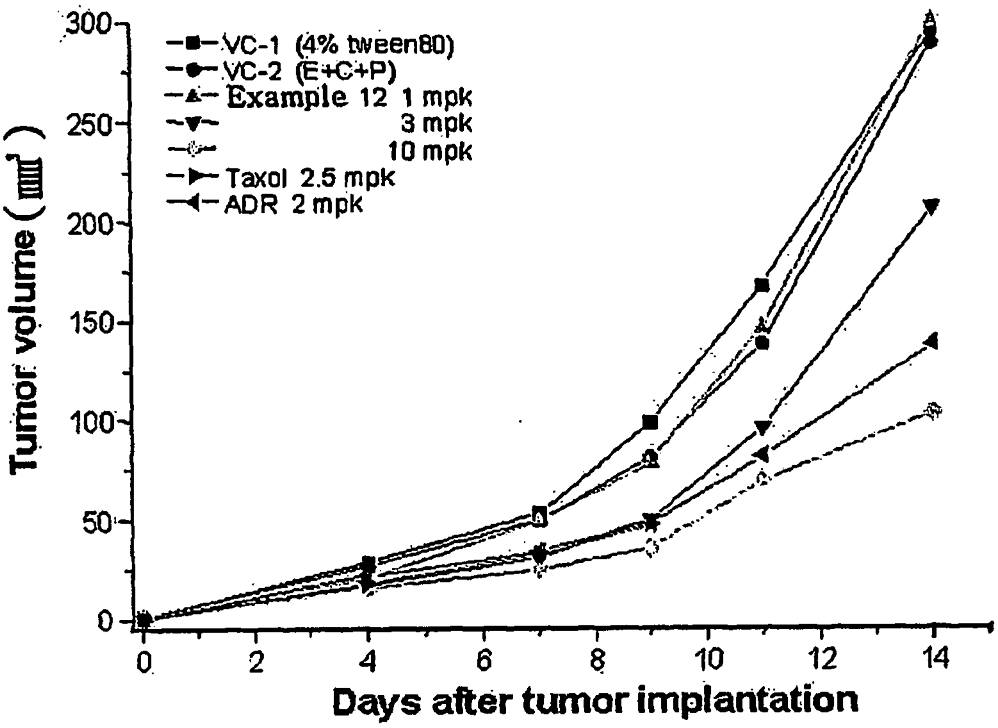
VC-1 (4% tween80)
VC-2 (E+C+P)
Example 12 1 mpk
3 mpk
10 mpk
Taxol 2.5 mpk
ADR 2 mpk
Tumor volume (㎣)
0
50
100
150
200
250
300
0
2
4
6
8
10
12
14
Days after tumor implantation

FIG.4

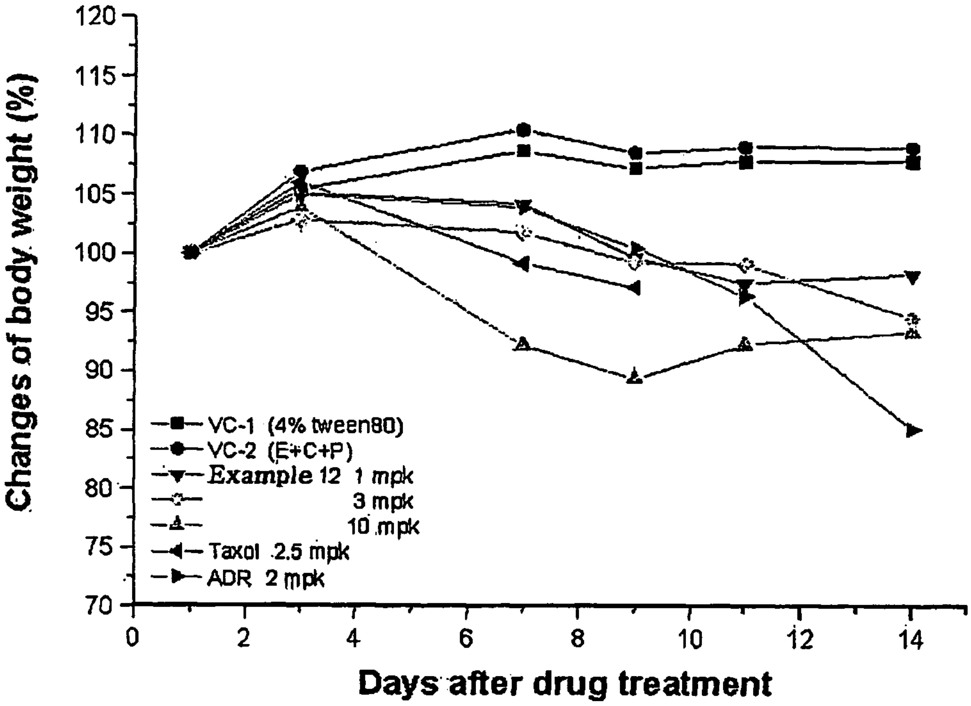
Changes of body weight (%)
120
115
110
105
100
95
90
85
80
75
70
0
2
4
6
8
10
12
14
Days after drug treatment
VC-1 (4% tween80)
VC-2 (E+C+P)
Example 12 1 mpk
3 mpk
10 mpk
Taxol 2.5 mpk
ADR 2 mpk

FIG.5

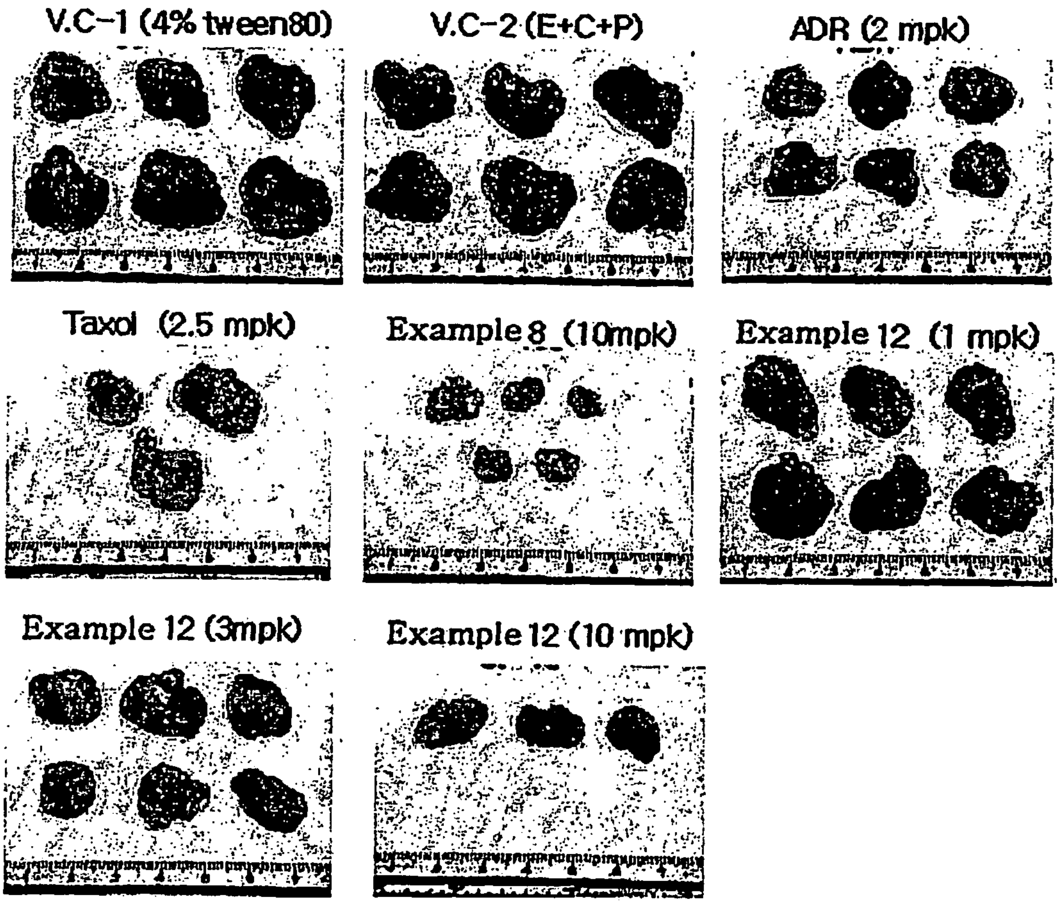
V.C-1 (4% tween80)
V.C-2 (E+C+P)
ADR (2 mpk)
Taxol (2.5 mpk)
Example 8 (10mpk)
Example 12 (1 mpk)
Example 12 (3mpk)
Example 12 (10 mpk)

FIG.6

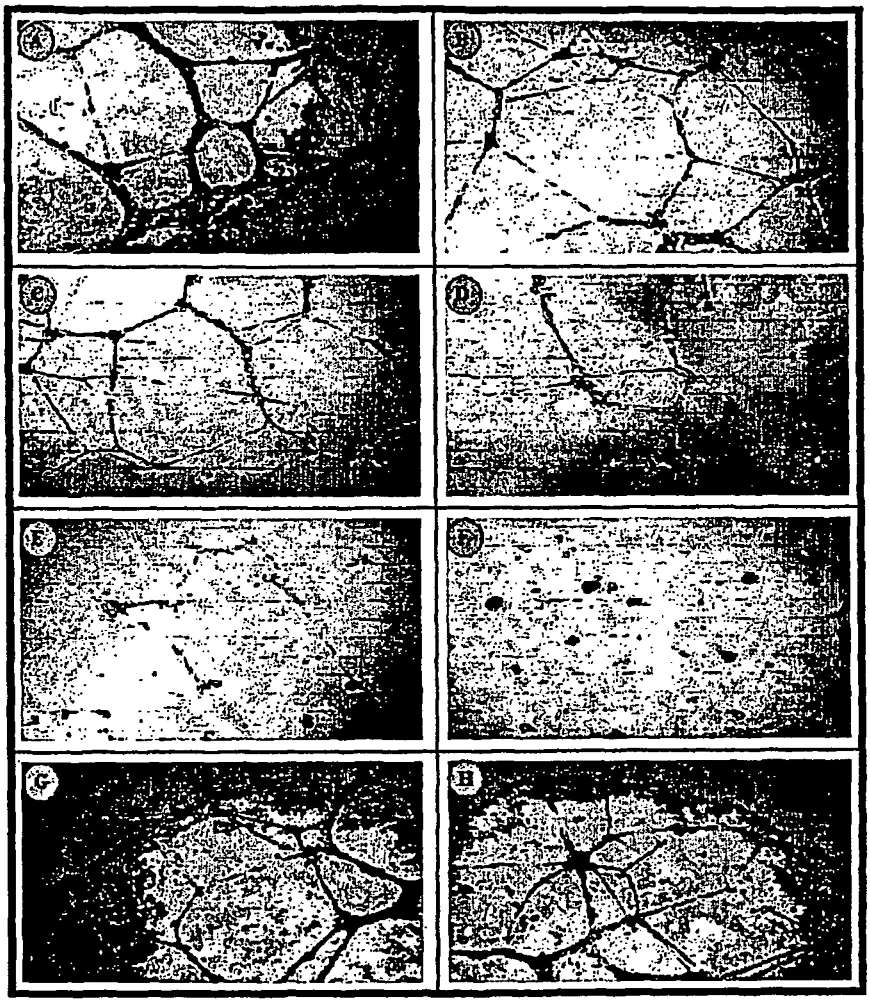


A: Solvent control, B: Example12 (0.3 ug/ml), C: Example 12 (1 ug/ml), D: Example 12 (3 ug/ml), E: Example 12 (10 ug/ml), F: Example 12 (30 ug/ml), G: fumagillin 10 ug/ml, H: doxorubicin 0.3 ug/ml.

Original magnification 100X

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US P5973204 A
- EP 0870761 A1
- WO 02100824 A
- US P4533675 A
- US P3222253 A
- US P006080739 A
- WO 9701570 A
- EP 0493064 A
- WO 9421598 A
- WO 9611184 A


### Non-patent literature cited in the description


- *Internal Medicine,* 2000, vol. 86 (2), 342-345
- *J. Am. Soc. Nephrol.,* 1993, vol. 4 (6), 1294-1299
- *Transplantation Proceedings,* 2002, vol. 32, 2091-2092
- **Sun et al.** *J. Med. Chem,* 1993, vol. 36, 1474-1479
- *The Alkaloids,* 1991, vol. 41, 125-176
- *Pharmacotherapy,* 1991, vol. 11 (3), 196-211
- *Synthetic Communications,* 1997, vol. 27 (2), 293-296
- *Bioorganic & Medicinal Chemistry,* 1997, vol. 5 (12), 2277-2282
- *J. Med. Chem,* 1985, vol. 28, 1204-1208
- *Bioorg. Med. Chem. Lett,* 1996, vol. 6, 3025-3028
- *Helv. Chim. Acta,* 1926, vol. 9, 1068
- *J. Amer. Chem. Soc,* 1982, vol. 104, 2251-2257
- *J. Amer. Chem. Soc.,* 1943, vol. 65, 2308
- *J. Org. Chem,* 1969, vol. 34, 1960-1961
- *Medicinal Research Reviews,* 1988, vol. 8 (1), 77-94